# EUROPEAN PATENT SPECIFICATION

(11) **EP 1 483 334 B1**
(45) Date of publication and mention of the grant of the patent: **04.07.2007**
(21) Application number: 03743838.9
(22) Date of filing: 04.03.2003
(51) Int. Cl.: C09B 44/16

(54) **PROCESS FOR EXCHANGING THE ANIONS OF CATIONIC DYES**
VERFAHREN ZUM AUSTAUSCH DER ANIONEN KATIONISCHER FARBSTOFFE
PROCEDE D'ECHANGE DES ANIONS DE COLORANTS CATIONIQUES

(30) Priority: 11.03.2002 EP 02405179
(43) Date of publication of application: 08.12.2004
(73) Proprietor: Ciba Specialty Chemicals Holding Inc., 4057 Basel (CH)
(72) Inventor: MÖCKLI, Peter, CH-4124 Schönenbuch (CH)
(86) International application number: PCT/EP2003/002199
(87) International publication number: WO 2003/076518

(56) References cited:
- EP-A- 0 097 125
- GB-A- 757 987
- US-A- 4 524 204
- PATENT ABSTRACTS OF JAPAN vol. 2000, no. 16, 8 May 2001 (2001-05-08) & JP 2001 019866 A (ORIENT CHEM IND LTD), 23 January 2001 (2001-01-23)
- KRECHL, J. ET AL.: "p-Substituted Benzamidinium Carboxylates" COLLECT. CZECH. CHEM. COMMUN., vol. 54, 1989, pages 2415-2424, XP001153039

## Description

Cationic dyes are of great importance in various fields of chemistry and medicine.

The counterions of those products, i.e, the anions, normally play more of a secondary part as far as the actual mode of action is concerned. However, they are of the utmost importance to the solubility and often determine whether a product acquires commercial importance or not, it being a question in most cases of imparting maximum water solubility to the product, which offers the opportunity for the formulations of liquid preparations. However, it may also be of great interest to achieve the reverse, i.e. to increase the lipophilicity by the selection of a suitable anion, for example stearate.

However, the alkylating agents used for cationisation in the synthesis of cationic dyes mostly produce anions that impart to the products moderate to poor solubility in water, for example methyl sulfates or, especially, halides. Anions that render products readily soluble, on the other hand, are, for example, acetates, formates, lactates, methanesulfonates, tartrates, etc., for the direct preparation of which in synthesis there are no reagents. They must therefore be introduced subsequently by exchanging the "insoluble" anions. A large number of methods exist for solving that problem, but most can be applied to only very specific products and/or are not toxicologically safe and/or are uneconomic on a large scale.

It has now been found that exchanging the anions of cationic dyes can, surprisingly, be carried out very well by reaction with the alkali metal salts, especially the potassium salts, of the desired acids in low boiling alcohols. The cationic dye goes into solution together with the newly introduced anion, while the original anion precipitates in the form of the alkali metal salt, which is sparingly soluble in the alcohol, and can be removed by clarifying filtration,

Accordingly, the present invention relates to a process for converting sparingly soluble salts of cationic dyes and anions of inorganic acids into more readily soluble salts of organic acids, which process comprises
a) preparing a sparingly water-soluble salt of the cationic dyes with the anion of an inorganic acid,
b) adding thereto, in a monohydric aliphatic alcohol, an alkali metal salt of an organic acid,
c) filtering off the resulting sparingly soluble alkali metal salt of the inorganic acid, and
d) optionally converting the resulting solution into a solid form.

Suitable cationic dyes are, for example, the compounds listed in the Colour Index under the headword "Basic Dyes", the dyes described in K. Venkataraman "The Chemistry of Synthetic Dyes", Vol. 4, Chap. 3-5, Academic Press, New York, 1971, and the numerous dyes containning a cationic group described in the patent literature. Preferred cationic dyes are, for example cationic acridin-, anthrachinon-, azin-, azo-, azomethin-, benzimidazol-, cyanin-, diazo-, dioxazin-, hydrazon-, ketoimin-, methin-, monoazo-, nitro-, oxazin-, polymethin-, quinolin-, thiazol-, triarylmetan- or xanthen dyes.

More preferred dyes for the process according to the invention are cationic oxazine dyes, mono- and disazo dyes, and hydrazon dyes.

Most preferred are dyes monoazo dyes of formula (1) 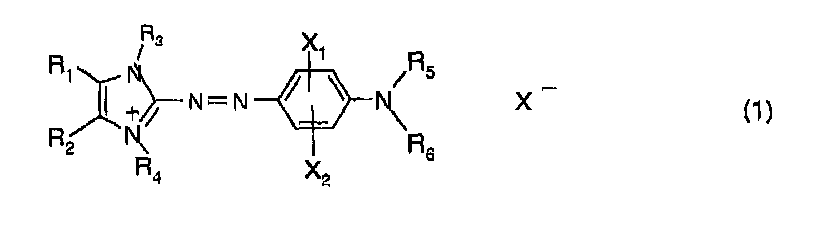 or hydrazon dyes of formula (2), 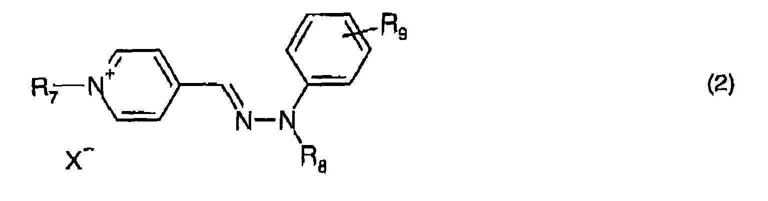 or diazo dyes of formula (3) 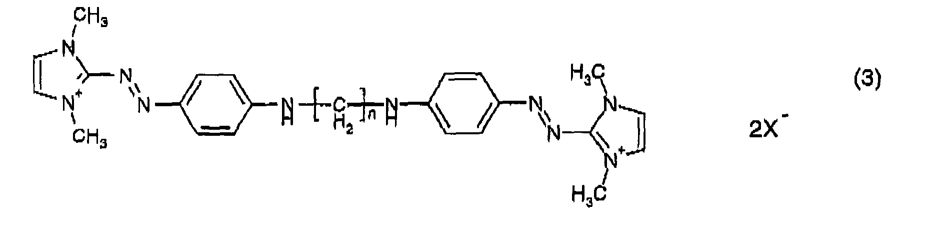 wherein
R₁ and R₂ are each independently of the other hydrogen, C₁-C₄alkyl, halogen or nitro, preferably hydrogen,
R₃ and R₄ are each independently of the other unsubstituted C₁-C₄alkyl or C₁-C₄alkyl substituted by OH, C₁-C₄alkoxy, halogen, CN or by phenyl, preferably unsubstituted C₁-C₄alkyl,
X₁ and X₂ are each independently of the other hydrogen, C₁-C₄alkyl, C₁-C₄alkoxy or halogen, preferably hydrogen,
R₅ is hydrogen or C₁-C₄alkyl,
R₆ is hydrogen, phenyl, C₁-C₁₂alkyl or C₅-C₈cycloalkyl, each of which may be unsubstituted or substituted by OH, C₁-C₄alkoxy, halogen, CN, R₆ is C₁-C₄alkyl substituted by phenyl or by C₅-C₈cycloalkyl,
or wherein R₅ and R₆, together with the nitrogen atom linking them, form a piperazine ring which is substituted by C₁-C₈alkyl or by phenyl on the nitrogen atom not bonded to the phenyl ring or which is quaternised at that nitrogen atom by means of two such groups, the
C₁-C₆alkyl and phenyl radicals mentioned as substituents on the nitrogen atom of the piperazine ring being unsubstituted or substituted by OH, C₁-C₄alkoxy, halogen, CN or by phenyl, and
wherein
R₇ and R₈ are each independently of the other a C₁-C₈alkyl radical or an unsubstituted or substituted benzyl radical, and
R₈ is hydrogen, C₁-C₈alkyl, C₁-C₈alkoxy, cyanide or halide, preferably hydrogen; and
n is a whole number in the range from 2 to 12, especially from 2 to 6;
and wherein X⁻ is an anion.

In the context of the present invention, alkyl radicals are understood as being straight-chain and branched alkyl radicals, for example methyl, ethyl, n- and iso-propyl and n-, sec- and tert-butyl, pentyl, hexyl, heptyl, octyl, nonyl, decyl, undecyl or dodecyl.

The alkyl radicals and the cycloalkyl radicals can be mono- or poly-substituted, for example by hydroxy, carboxy, halogen, cyano, phenyl or by C₁-C₄alkoxy.

The alkyl radicals can be interrupted by hetereoatoms, such as N; S or O.

The alkoxy radicals can contain from 1 to 12 carbon atoms, preferably from 1 to 4 carbon atoms. Examples of suitable alkoxy radicals are methoxy, ethoxy, propoxy, isopropoxy, n-butoxy, isobutoxy, tert-butoxy, n-pentyloxy or n-hexyloxy. The alkoxy groups can likewise be substituted, for example by the groups mentioned as possible substituents for the alkyl radicals, especially by hydroxy or by C₁-C₄alkoxy,

For the process according to the invention, special preference is given to the dyes of formulae 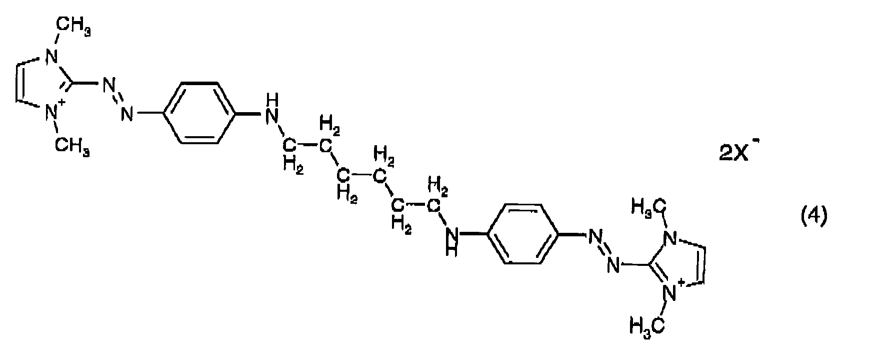 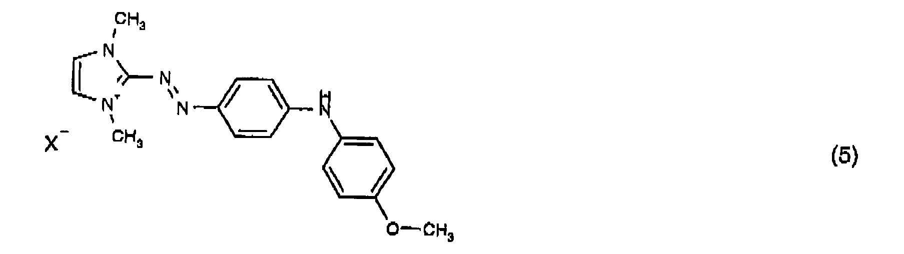 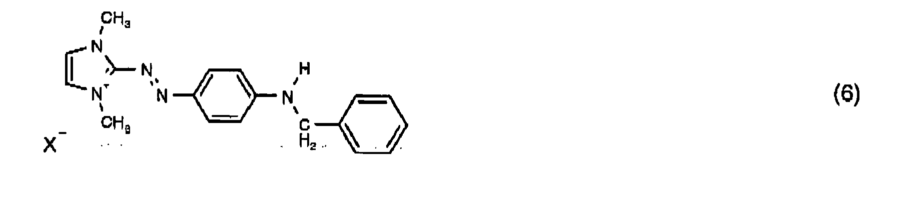 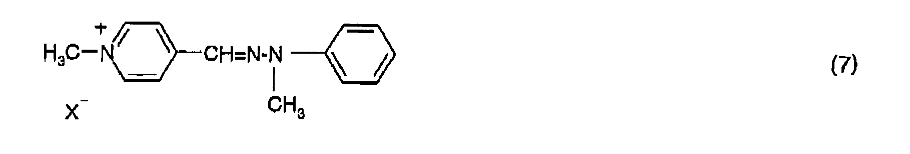 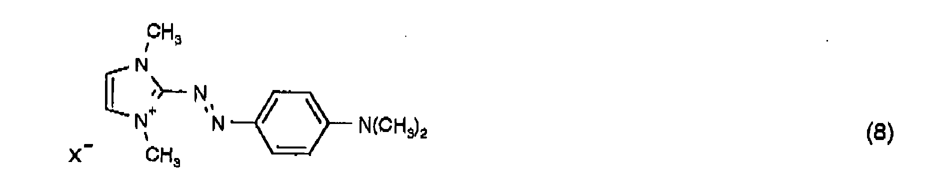 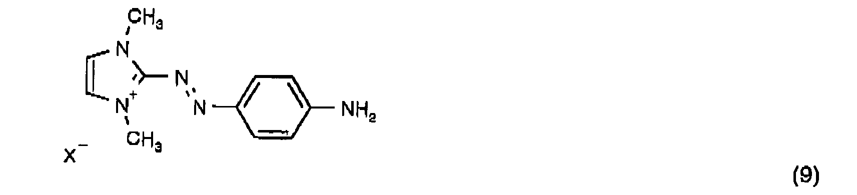 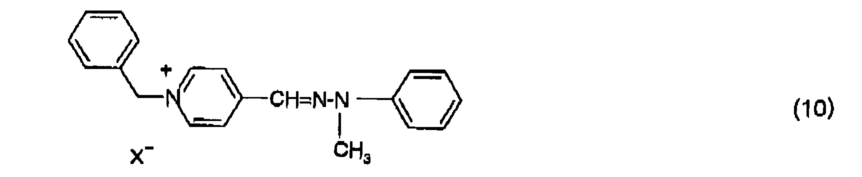 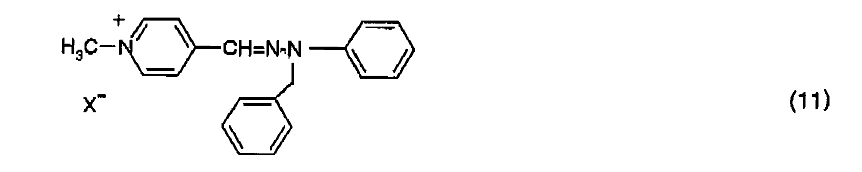 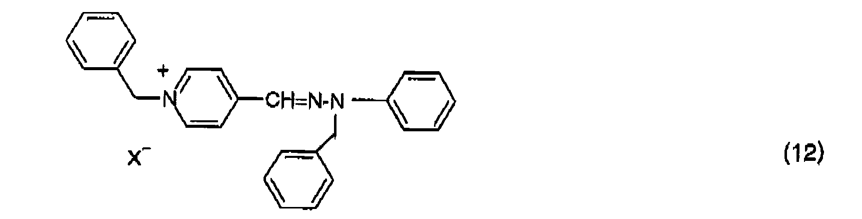 and 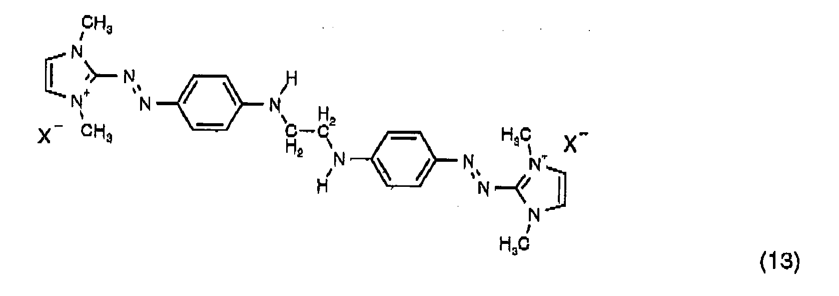 wherein X⁻ is an anion.

The cationic dyes are known or can be prepared in a manner known *per se.*

Examples of anions of inorganic acids are halides, such as iodide, bromide and especially chloride, and also sulfate, borofluorate, hydrogen sulfate, methyl sulfate, phosphate and perchlorate.

The anions of organic acids are, for example, formate, acetate, propionate, butyrate, monochloroacetate, trifluoroacetate, tartrate, oxalate, maleate, acrylate, succinate, citrate, lactate, methanesulfonate and ethanesulfonate, with formate, acetate, propionate and trifluoroacetate being especially preferred.

Suitable alkali metal salts are, for example, lithium, sodium and potassium salts, with potassium salts being especially preferred.

The alkali metal salts can either be added directly or alternatively they can first be produced in the reaction solution by reacting the corresponding alkali metal carbonate or bicarbonate *In situ* with the desired acid.

The alkali metal salt of the organic acid is normally used in an equimolar amount or in a slight excess relative to the molar mass of the inorganic anion, generally from 1 to 1.3 mol of alkali metal salt per mol of inorganic anion.

Reacting the sparingly water-soluble salt of the cationic dye with the anion of an inorganic acid and the alkali metal salt of an organic acid is carried out, for example, at a temperature of approximately from 0°C to the boiling temperature of the used alcohol, preferably from 15 to 100°C and most preferably in the temperature range of the boiling point of the used alcohol,

The cationic dye is dissolved or suspended in the alcohol so that its concentration is, for example, approximately from 2 to 80 % by weight, based on the total weight of the solution or suspension. The alkali metal salt of the organic acid is then added all at once or, preferably, in several portions.

Alcohols suitable for the process according to the invention are, for example, methanol, ethanol, n- and iso-propanol and also n-, iso- and tert-butanol. Special preference is given to methanol, ethanol and, especially, isopropanol.

When the reaction is complete, the alkali metal salt of the inorganic acid that has formed is separated off in the conventional manner, for example by filtration, optionally in the hot state or after cooling.

Customary, the alKali metal salt of the inorganic acid is separated in the hot state,

in general the separation is conducted by filtration.

Usually, after the filtration in the hot state, the filtrate is cooled and the desired compound crystallized. In general, the crystallized compound is then be isolated by filtration.

If the desired compound is also very soluble in the alcohol after cooling, it may be of advantage to remove the alcohol by distillation, optionally under reduced pressure. Decomposition of the organic cation is normally prevented thereby. In the case of products that are very sensitive to hydrolysis, it is recommended not to concentrate them to dryness by evaporation but to ensure that the dilution is always sufficient by addition of water before the end of the distillation. The aqueous solution can then optionally be lyophilised, if a dry product is required.

The newly formed alkali metal salt can optionally be separated off by crystallisation.

The resulting readily soluble salts of the cationic dye are suitable for the customary uses of cationic compounds, where, by virtue of their good solubility, especially in water, they permit the preparation of concentrated solutions.

The following Examples serve to illustrate the invention. Unless specified otherwise, parts and percentages relate to weight.

### Example 1:

26 g of the dye of formula 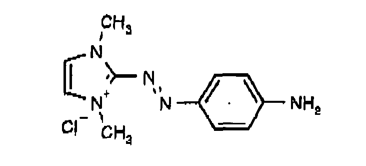 are heated to 60°C in 60 g of methanol. 10 g of potassium acetate are added thereto and stirring is carried out for 4 hours at 60°C. The mixture is then allowed to cool to room temperature and is filtered. The filter cake is then washed with 5 g of methanol, yielding a residue of 6.5 g of almost colourless salt, which consists almost entirely of potassium chloride. The methanolic mother liquor is concentrated to dryness by evaporation in a rotary evaporator, yielding 29 g of a dark-violet powder which is substantially the acetate of the dye that was used and has a chloride content of only 1.6 % (the starting product contains 14.1 %) and a water solubility of over 25 %. The water solubility of the chloride of the dye used is only 3.7 %.

### Example 2:

50 g of the same dye as in Example 1 are heated to 60°C in 190 g of isopropanol, and 23.4 g of potassium acetate are then added thereto. The temperature is raised to 80°C and maintained for 7 hours. The hot solution is then filtered through a pre-heated suction filter and the residue is washed with a small amount of isopropanol. About 14 g of almost colourless salt (potassium chloride) are obtained. Upon cooling to room temperature, the acetate of the dye crystallises from the mother liquor in the form of attractive crystals. Filtration, washing with a small amount of isopropanol and drying yield 38.5 g in very pure form and with a chloride content of only 0.6 %. The mother liquor is concentrated to dryness by evaporation *in vacuo* at a maximum of 50°C, yielding 21 g of a mixture of about 85 % acetate of the dye and 15 % potassium acetate. It is possible to prepare from the two fractions a clear, stable, 30 % aqueous solution.

### Example 3:

10,3 g of the same starting material as in Example 1 and 3.0 g of potassium carbonate are suspended in 50 g of isopropanol and heated to 60°C, 4 g of anhydrous lactic acid are then added thereto, and stirring is carried out for 4 hours at 60°C. The mixture is then diluted with a further 64 g of isopropanol and the temperature is raised to 80°C in order to dissolve all the dye. Clarifying filtration is carried out through a suction filter pre-heated to 80°C, and the residue is then washed with 18 g of Isopropanol, yielding a potassium chloride residue of 2.47 g, which corresponds to about 94 % of the expected amount. The filtrate is allowed to cool, with stirring, whereupon the lactate of the dye precipitates in crystalline form. Filtration, washing and drying yield 8.84 g of dark dye powder whose solubility in water is over 20 %, whereas the solubility of the starting product is only 3.7 %.

### Example 4:

The procedure is exactly as in Example 3 at first, but 3.3 g of propionic acid are added instead of the lactic acid and the temperature is immediately raised to 80°C. After 2 hours, clarifying filtration is carried out through a pre-heated suction filter, and the residue is washed with 65 g of isopropanol. The largely colourless clarified residue is 2.85 g and consists of 44,7 % chloride. The filtrate is concentrated to about 1/3 as gently as possible in a rotary evaporator and then allowed to cool, with stirring. The resulting dye is filtered off with suction, washed with 63 g of isopropanol and dried, yielding 8.2 g of crystal powder having a chloride content of only 1.4 % (about 1/10 of the original chloride content). The solubility of the propionate of the dye in water is likewise over 30 %.

### Example 5:

10.7 g of the dye of formula 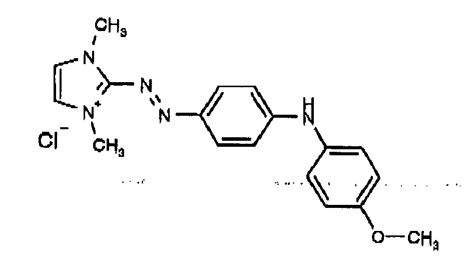 are suspended in 70 g of isopropanol, together with 2,6 g of potassium formate, and heated to 80°C. From about 62°C, the starting materials are largely dissolved and salt crystals are immediately observed on the wall of the flask. After 4 hours, clarifying filtration is carried out through a pre-heated suction filter, and the residue is then washed with 47 g of isopropanol.

The dry residue weighs 2,04 g and consists almost entirely of potassium chloride. Upon cooling, the formate of the dye crystallises from the filtrate in the form of violet crystals; yield 7.4 g. The mother liquor is concentrated to dryness by evaporation *in vacuo* and yields a further 3.75 g of the formate of the dye of almost equal purity. The chloride contents of those two fractions are only 0.5 % and 0.23 %, respectively, which likewise Indicates an anion exchange of over 95 %. Accordingly, their water solubility is over 30 %, whereas that of the chloride used is only 0.35 %.

### Example 6:

10.3 g of the dye chloride from Example 1 are suspended in 50 g of isopropanol, with 3.0 g of potassium carbonate and 11.5 g of stearic acid, and heated to 60°C. After 4 hours, the temperature is raised to 80°C. a further 200 g of isopropanol are added, and clarifying filtration is carried out through a heated suction filter at 80°C. 3.1 g of almost colourless salt remain on the filter. Upon cooling to room temperature, the stearate of the dye precipitates from the mother liquor in the form of a crystalline precipitate. The latter is filtered off, washed with 30 g of isopropanol and dried, yielding 10.7 g of a brown-violet dye powder. By concentration of the mother liquor it is readily possible to obtain a further 5 g of product of equally good quality.

### Example 7:

4.0 g of the oxazine dye of formula 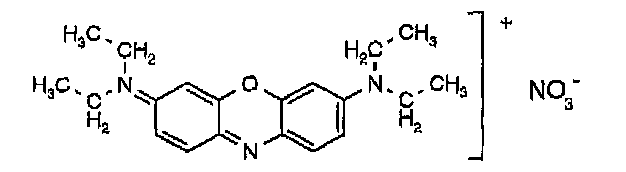 are placed, together with 0,72 g of potassium carbonate, in 13 g of isopropanol. 1,2 g of trifluoroacetic acid are added to the highly fluid suspension, whereupon the mixture becomes viscous. However, it rapidly becomes highly fluid again upon heating. Stirring is carried out for 4 hours at a temperature of 60°C, the temperature is then raised to 80°C, and the precipitated salt is filtered off while hot. The residue is rinsed with a small amount of isopropanol. Upon cooling, with stirring, the trifluoroacetate of the dye precipitates from the mother liquor in the form of a crystalline precipitate. The latter is filtered off, washed with a small amount of isopropanol and dried, yielding 1.9 g of a dye powder whose solubility in water is over 1.5 %, whereas the solubility of the starting product is less than 0.4 %. Neither the isolated product nor the mother liquor, which still contains a large amount of product, exhibit any decomposition. From the nitrate that is still present (about 3 %) it is possible to conclude that the exchange has taken place to the extent of about 82 %, A further 2.2 g of dye of equal quality can be obtained from the mother liquor by gentle concentration.

### Example 8:

4 g of the dye of formula 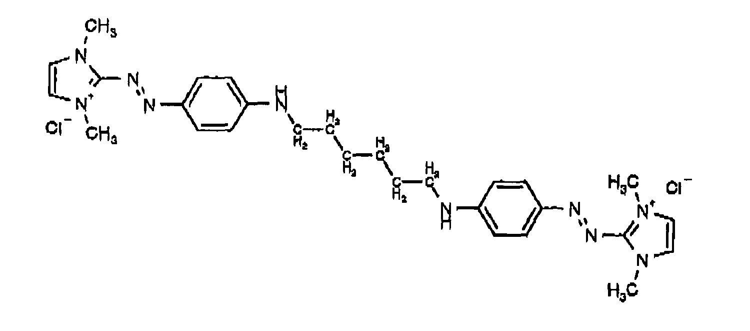 and 1.5 g of potassium acetate are suspended in 20 g of isopropanol and stirred for 5 hours at 60°C. Clarifying filtration is then carried out while hot. The salt residue is 1.12 g. The acetate of the dye crystallises from the mother liquor. Filtration and washing with isopropanol yield 3,43 g (dry) of dark crystal powder whose solubility in water has increased one hundred fold from < 0.03 % to 3 %,

### Example 9:

If in Example 8 the potassium acetate is replaced by 1.3 g of potassium formate and heating is carried out for 4 hours at 80°C, there are obtained, with an otherwise identical procedure and working up, 3,54 g of the formate of the dye, whose solubility in water is about 1.2 %. The salt residue from the clarifying filtration is 1.0 g.

### Example 10:

1,29 g of potassium acetate are added to 3 g of the compound of formula 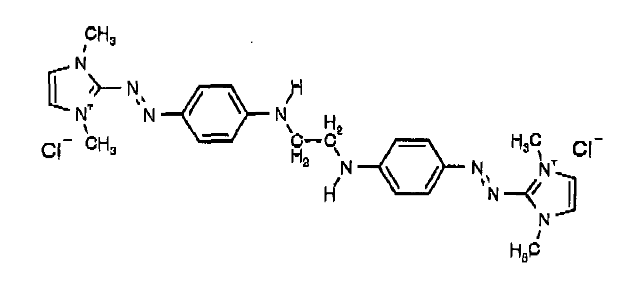 in 15 g of methanol, and stirring is carried out for 4 hours at a temperature of 55°C. A highly fluid suspension is obtained. Upon cooling, no dye precipitates and the clarifying filtration can therefore be carried out at room temperature; clarified residue 1.1 g. The mother liquor is concentrated to dryness by evaporation under gentle conditions at 50°C and 70 mbar, yielding 3.1 g of the acetate of the dye which, in contrast to the starting product, is excellently soluble In water.

### Example 11:

By means of the procedure described in Example 1 it is also possible to convert the chloride of the cationic dye of the following formula into the acetate:

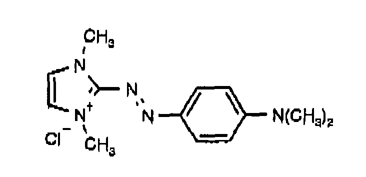

### Example 12:

By means of the procedure described in Example 1 it is also possible to convert the chloride of the cationic dye of the following formula into the acetate:

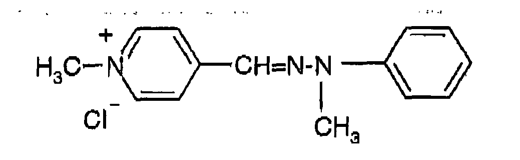

### Example 13:

By means of the procedure described in Example 1 it is also possible to convert the chloride of the cationic dye of the following formula into the acetate: 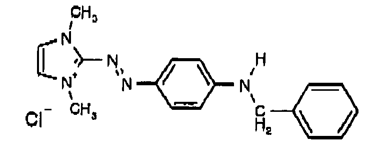 dyes and oxidization agents, especially for semi permanent dyeing and permanent dyeing, are:
Disperse Violet 4, Picramic acid, N,N'-Bis-(2-Hydroxyethyl) -2-nitro-p-phenylendiamine, HC Yellow No. 5, HC Blue No. 2, HC Yellow No. 2, 2-Chloro-5-nitro-N-hydroxyethyl-p-phenylendiamine, HC Red No. 3, 4-Amino-3-nitrophenol, Basic Blue 99, 2-Hydroxyethyl Picramic acid, HC Yellow No. 6, Hydroxyethyl-2-nitro-p-toluidine, 2-Amino-6-chloro-4-nitrophenol, 4-Hydroxypropylamino-3-nitrophenol, Basic Red 2, HC Red No. 16 and HC Blue No. 16.

Further preferred cationic dyes for the combination with a compound of formula (1), (2) or (3) according to the present invention are described in the following references
in WO 95/01772, especially on page 2, line 7 to page 4, line 1, and especially on page 4, line 35 to page 8, line 21 and on pages 11 to 27, or
in WO 01/66646, especially on page 1, line 18 to page 3, line 16, and preferred from page 16, line 20 to page 22, and cationic dyes as described on pages 10 to 17, or
in EP 970 685, especially on page 2, line 44 to page 9, line 56 and preferably on page 9, line 58 to page 48, line 12, or
direct dyes are described in DE-A-19 713 698 , especially page 2, line 61 to page 3, line 43, or
direct dyes and oxidizing agent are described in WO 97/20545, especially on page 1, lines 4 to 10, in particular on page 3, lines 24 to 32, and on page11, line 6 to page 13, line 19, especially with direct dyes are described on page 5, line 28 to page 8, line 20, or
cationic dyes and anionic UV-absorbers are described in EP 1 166 752, especially on page 3, line 20 to page 4, line 21, in particular with UV absorber on page 4, lines 26 to 3, and especially on page7, line 47 to page 9, line 56.

More preferred for a combination with a cationic dye of formula (1), (2) or (3) are cationic dyes, such as Basic Yellow 87, Basic Orange 31 or Basic Red 51, or cationic dyes as described in WO 01/66646, especially cationic dye of example 4, or cationic dyes as described in WO 02/31056, especially cationic dye of example 6, compound of formula 106, or cationic dye of formula (3) as described in EP-A-714,954 or a yellow cationic dyes of formula (1) 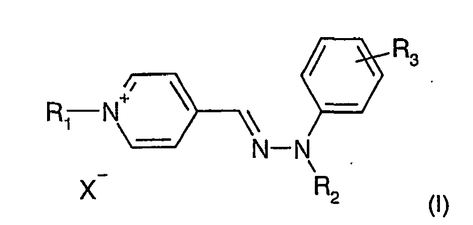 wherein
R₁ and R₂ are each independently of the other a C₁-C₈alkyl radical or an unsubstituted or substituted benzyl radical,
R₃ is hydrogen, C₁-C₈alkyl, C₁-C₈alkoxy, cyanide or halide, preferably hydrogen, and
X⁻ is an anion, and preferably a compound of formula (I)
wherein
R₁ is methyl, R₂ is benzyl, R₃ is hydrogen, and X⁻ is an anion, or
wherein
R₁ is benzyl, R₂ is benzyl, R₃ is hydrogen, and X⁻ is an anion, or
wherein
R₁ is benzyl, R₂ is methyl, R₃ is hydrogen, and X⁻ is an anion, which is defined as given above.

The process for the preparation of compounds of formula (I) comprises reacting a phenylhydrazine with a 4-pyridylaldehyde in the presence of an acid to form a hydrazone, which is optionally alkylated or benzylated.

Further preferred for a combination with a cationic dye of formula (1), (2) or (3) are cationic nitroaniline and anthraquinone dyes for the combination a compound of formula (1), (2) or (3) according to the invention, for example those described in the following patent specifications: US-5 298 029, especially in column 2, line 33 to column 5, line 38;
US-5 360 930, especially in column 2, line 38 to column 5, line 49; US-5 169 403, especially in column 2, line 30 to column 5, line 38; US-5 256 823, especially in column 4, line 23 to column 5, line 15; US-5 135 543, especially in column 4, line 24 to column 5, line 16;
EP-A-818 193, especially on page 2, line 40 to page 3, line 26; US-5 486 629, especially in column 2, line 34 to column 5, line 29; and EP-A-758 547, especially on page 7, line 48 to page 8, line 19.
In addition, preferred are combinations of a compound of formula (1), (2) or (3) according to the invention with further cationic dyes or with other dyes. Preferred are mixtures as given in the below references, with the proviso that one cationic dye is replaced by a compound of formula (1), (2) or (3) according to the present invention.
- mixtures of at least two cationic dyes as described in WO 95/01772, especially on page 8, line 34 to page 10, line 22 with the given preferences, or
- combinations of Pyrazolo-[1,5-a]-pyrimidines with at least one cationic dye as described in EP 998,908, especially on page 2, line 34 to line 42, with preferred Pyrazolo-[1,5-a]-pyrimidines as described in EP 998,908, especially on page 2, line 48 to page 4, line 3, and with preferred cationic direct dyes as described in EP 998,908, especially on page 4, line 22 to page 47, line 24, or
- combinations of cationic dyes as described in FR-2788432, especially on page 53, line 1 to page 63, line 23, especially a combination of cationic dyes with Arianors in FR-2788432, especially on pages 51 to 52, or especially a combination with at least one Basic Brown 17, Basic brown 16, Basic Red 76 and Basic Red 118, and/or at least one Basic Yellow 57, and/or at least one Basic Blue 99, or
- combinations of direct dyes and/or an oxidation dye and oxidizing agents in the form of permanent-wave fixing solution, especially with direct dyes as described in DE-A-19 713 698 , especially page 4, line 65 to page 35, line 59, or
- combinations of cationic dyes and an oxidation dye of the developer compound type and oxidizing agents as described in EP 850 638, especially on page 2, line 27 to page 7, line 46 and preferred on page 7, line 20 to page 9, line 26, or
- combinations of an extemporaneous mixture of a composition (A) containing one or more oxidation dye precursors and optionally one or more couplers, and of a composition (B), in powder form, containing one or more direct dye, preferably cationic, optionally dispersed in an organic pulverulent excipient and/or a mineral pulverulent excipient, and a composition (C) containing one or more oxidizing agent as described in US 6,190,421, especially in column 2, lines 2 to 1, and preferably with oxidation dye precursors as described in column 2, line 35 to column 5, line 13, and preferably with direct dyes as described in column 5, line 30 to column 7, line 14, or
- a ready-to-use composition comprising, at least one oxidation base, at least one cationic direct dye and at least one enzyme of 2-electron oxidoreductase type in the presence of at least one donor for the said enzyme as described in US 6,228,129, especially in column 26, line 26 to column 27, line 9 with cationic direct dyes as described in column 8, line 17 to column 13, line 65, especially those as described in column 20, line 11 to line 19, in column 23, line 61 to column 24, line 25, or
- compositions of at least one direct cationic dye and at least one nitrated benzene dye as described in WO 99/20235 on page 2, line 1 to page 7, line 9, and on page 39, line 1 to page 40b line 11, with cationic direct dyes as described on page 8, line 12 to page 25 line 6, and nitro benzene direct dyes as described on page 26, line 7 to page 30, line 15, or
- compositions of at least one direct cationic dye and at least one autooxidisable oxidation dye, especially benzene, indol and indoline derivatives as described in WO 99/20234, with in preferred direct dyes as given on page 2, line 17 to page 26, line 4, and autooxidisable oxidation dye as described especially on page 26, line 10 to page 28, line 15, or
- oxidation dyeing compositions of at least one direct dye and at least one meta-Aminophenol derivative and at least one developer compound and an oxidizing agent as described in EP 850 636, especially on page 5, line 41 to page 7, line 52, and preferably on page 19, line 50 to page 22, line 12, with preferred direct dye as described on page 18, lines 1 and 2 in connection with page 7, line 53 to page 17, line 55, and with preferred meta-Aminophenol derivatives as described on page 7, line 47 to line 52, and with preferred developer compounds as described on page 6, line 10 to page 7, line 46, or
- oxidation dyeing compositions of at least one direct dye and at least one developer compound selected from the group of para-Phenylenediamine derivatives and Bis-Phenylalkylenediamine and, and at least one coupler compound selected from the group of meta-Diphenols and an oxidizing agent, as described in EP-A-850 637, especially on page 6, line 50 to page 8, line 44, or
- oxidation dyeing compositions with cationic couplers, as described in WO 99/48856, especially on page 9, line 16 to page 13, line 8, and page 11, line 20 to page 12, line 13, or
- cationic dye and e.g. a pyrazolo-(1,5-a)-pyrimidine derivatives, as described in EP 998 908, especially on page 2, line 34 to page 4, line 23, or
- arianoren and/or oxidative dyes, as described in FR-2 788 432, especially on page 2, line 16 to page 3, line 16, and page 5, line 19 to page 14, line 8, and combinations with cationic dyes as described on page 14, line 23 and following, or
- oxidative dye precursors (unsaturated aldehyde and coupler compounds), as described in German Patent Application 197 172 24, especially unsaturated aldehydes as described on page 2, line 50 to line 66 and page 3 line 8 to line 12 are used as developer compounds, and primary and secondary amino group compounds, nitrogen-containing heterocyclic compounds, amino acids, oligopeptids, aromatic hydroxy compounds, CH-active compounds as described on page 3, line 42 to page 5 line 25 are used as coupler compounds.

Further preferred for the combination with a compound of formula (1), (2) or (3) are cationic azo dyes, e.g. according to GB-A-2 319 776, as well as the oxazine dyes described in DE-A-29 912 327 and mixtures thereof with the other direct dyes mentioned therein.

More preferred for a combination with a cationic dye of formula (1), (2) or (3) are cationic dyes such as Basic Yellow 87, Basic Orange 31 or Basic Red 51, or as described in WO 01/66646, especially cationic dye of example 4, or as described in WO 02/31056, especially cationic dye of example 6, compound of formula 106.

Especially preferred for a combination with a cationic dye of formula (1), (2) or (3) are direct dye mixtures comprising a dye of formula (1) of WO 01/66646, especially a direct dye of example 4, and/or or a dye of formula (2) of WO 02/31056, especially a direct dye of example 6, and/or Basic Yellow 87, and/or Basic Red 51, and/or Basic Orange 31.

No particular limitation is imposed on the acid dye used in the present invention so far as it is a water-soluble acid dye.

A further embodiment of the present invention concerns the combination of a compound of formula (1), (2) or (3) according to the invention with acid dyes, for example from the group of the compounds known by the international names (Color index), or trade names.

Preferred acid dyes for a combination with a cationic dye of formula (1), (2) or (3) are described in US Patent 6,248,314, they include Red Color No. 120, Yellow Color No. 4, Yellow Color No. 5, Red Color No. 201, Red Color No. 227, Orange Color No. 205, Brown Color No. 201, Red Color No. 502, Red Color No. 503, Red Color No. 504, Red Color No. 506, Orange Color No. 402, Yellow Color No. 402, Yellow Color No. 406, Yellow Color No. 407, Red Color No. 213, Red Color No. 214, Red Color No. 3, Red Color No. 104, Red Color No. 105(1), Red Color No. 106, Green Color No. 2, Green Color No. 3, Orange Color No. 207, Yellow Color No. 202(1), Yellow Color No. 202(2), Blue Color No. 202, Blue Color No. 203, Blue Color No. 205, Blue Color No. 2, Yellow Color No. 203, Blue Color No. 201, Green Color No. 201, Blue Color NO. 1, Red Color No. 230(1), Red Color No. 231, Red Color No. 232, Green Color No. 204, Green Color No. 205, Red Color No. 401, Yellow Color No. 403(1), Green Color No. 401, Green Color No. 402, Black Color No. 401 and Purple Color No. 401, especially Black Color No. 401, Purple Color 401, Orange Color No. 205.

These acid dyes may be used either single or in any combination thereof.

Preferably they are incorporated in dyeing composition for human hair in a proportion of 0.001 - 5% by weight (hereinafter indicated merely by "%"), particularly 0.005 - 4%, more particularly 0.2 - 3% based on the total weight of the composition, from the viewpoint of practical use in that a sufficient hair-dyeing effect is achieved, and the hand skin is scarcely smeared.

A further embodiment of the present invention concerns the combination of a compound of formula (1), (2) or (3) according to the invention with uncharged dyes, for example from the group of the nitroanilines, nitrophenylenediamines, nitroaminophenols, anthraquinones, indophenols, phenazines, phenothiazines, bispyrazolons or bispyrazol aza derivatives or methines.

In addition, the present invention concerns the combination of a compound of formula (1), (2) or (3) according to the invention with oxidation dyes.

Suitable oxidation dyes are described for example in
- German Patent Application 19 94 450, especially on page 6, line 6 to line 64, or
- German Patent Application 19 959 479, especially in column 2, line 6 to column 3, line 11, or
   - in the series "Dermatology", edited by Ch. Culnan, H. Maibach, Verlag Marcel Dekker Inc., New York, Basle, 1986, Vol. 7, Ch. Zviak, The Science of Hair Care, chapter 8, on pages 264 - 267 (oxidation dyes), or
- in German Patent Application 19 717 224; unsaturated aldehydes as described on page 2, line 50 to line 66 and on page 3 line 8 to line 12 are used as developer compounds, and primary and secondary amino group compounds, nitrogen-containing heterocyclic compounds, amino acids, oligopeptids, aromatic hydroxy compounds, CH-active compounds as described on page 3, line 42 to page 5 line 8 are used as coupler compounds.

Preferred oxidation dye precursors of the developer type for a combination with a cationic dye of formula (1), (2) or (3) are for example primary aromatic amines, which are substituted in the para- or ortho- position with a substituted or unsubstituted hydroxy- or amino residue, or diaminopyridine derivatives, heterocyclic hydrazones, 4-aminopyrazol derivatives, 2,4,5,6-tetraaminopyrimidin derivatives, or unsaturated aldehydes as described in German Patent Application 19 717 224, especially on page 2, line 50 to line 66 and on page 3 line 8 to line 12, or cationic developer compounds as described in WO 00/43367, especially on page, 2 line 27 to page 8, line 24, in particular on page 9, line 22 to page 11, line 6.
Also very suitable for a combination with a cationic dye of formula (1), (2) or (3) according to the invention are developer dyes in their physiological compatible acid addition salt form, such as hydrochloride or sulfate. Developer dyes, which have aromatic OH substituents are also suitable in their salt form with base, such as alkalimetalphenolates.

Preferred developer compounds are:
1,4-diamino-benzene (p-phenylendiamine), 1,4-diamino-2-methyl-benzene (p-toluylendiamine), 1,4-diamino-2,6-dimethyl-benzene, 1,4-diamino-2,5-dimethyl-benzene, 1,4-diamino-2,3-dimethyl-benzene, 2-chloro-1,4- diaminobenzene, 4-phenylamino-aniline, 4-dimethylamino-aniline, 4-diethylamino-aniline, hydroxyethyl-p-phenylendiamine, 1-(2'-hydroxyethyl)-2,5-diaminobenzene, N,N-bis-(2-hydroxyethyl)-p-phenylendiamine, 4-[(2-methoxyethyl)amino]-aniline, 4-[(3-hydroxypropyl)amino]-aniline, hydroxypropyl-bis-(N-hydroxyethyl-p-phenylenediamine) hydrochloride, 1,4-diamino-2-(2-hydroxyethyl)-benzene, 1,4-diamino-2-(1-methylethyl)- benzene, 2-(2,5- diaminophenoxy)-ethanol, 1,3-bis[(4-aminophenyl)(2-hydroxyethyl)amino]-2-propanol, bis-(2-hydroxy-5-aminophenyl)-methane,1,4-bis-(4-aminophenyl)-diazacycloheptane, 1,8-bis(2,5-diaminophenoxy)-3,6-dioxaoctan, 1,10-bis-(2,5-diaminophenyl)-1,4,7,10-tetraoxadecane, hydroxyethyl-3,4-methylenedioxyaniline, p-aminophenol, o-aminophenol, m-aminophenol, 2-amino-6-methyl-phenol, 4-methylaminophenol sulfate, 4-amino-m-cresol, 6-amino-m-cresol, 6-amino-m-cresol, 2-amino-4-hydroxyethylaminoanisole, 2-amino-5- methyl-phenol, 4-amino-3-methylphenol, 4-methylamino-phenol, 2-aminomethyl-4-aminophenol, 4-amino-2-[(2-hydroxyethyl)-amino]methyl-phenol, 4- amino-2-(2-hydroxyethoxy)-phenol, 4-amino-2-(methoxymethyl)-phenol, 4-amino-2-(2-hydroxyethyl)-phenol, 2-hydroxymethylamino-4-aminophenol, bis-(4-aminophenyl)amine, 4-amino-3-fluorphenol, 2-hydroxymethyl-4-aminophenol, 4-amino-2-(diethylamino)- methyl)-phenol, 5-amino-salicylsäure, 2,5-diamino-pyridine, 2-amino-3-hydroxy-pyridine, 2,6-dimethoxy-3,5-diamino-pyridine, 2,4,5,6-tetraaminopyrimidine, 2-hydroxy-4,5,6-triaminopyrimidine, 4-hydroxy-2,5,6-triaminopyrimidine, 2,4-dihydroxy- 5,6-diaminopyrimidine, 2-dimethylamino-4,5,6-triaminopyrimidine, 2,5,6-triamino-4-(1 H)-pyrimidone, further 4,5-diaminopyrazol derivatives as described in EP 0 740 741 or WO 94/08970, especially 4,5-diamino-1-(2-hydroxyethyl)-1 H- pyrazol, 4,5-diamino-1-(1-methylethyl)-1 H-pyrazol, 4,5-diamino-1-[(4-methylphenyl)methyl]-1H-pyrazol, 1-[(4-chlorophenyl)methyl]-4,5-diamino-1 H-pyrazol, 4,5-diamino-1-methyl-1 H-pyrazol.

More preferred developer dyes are p-phenylendiamine, p-toluylendiamine, p-aminophenol, m-aminophenol, o-aminophenol, N,N-bis-(2-hydroxyethyl)-p-phenylendiamine sulfate, 2-amino-4-hydroxyethylaminoanisole sulfate, hydroxyethyl-3,4-methylenedioxyaniline, 1-(2'-hydroxyethyl)-2,5-diaminobenzene, 2,6-dimethoxy-3,5-diamino-pyridine, hydroxypropyl-bis-(N-hydroxyethyl-p-phenylenediamine) hydrochloride, hydroxyethyl-p-phenylendiamine sulfate,4-amino-3-methylphenol, 4-methylaminophenol sulfate, 2-aminomethyl-4-aminophenol, 4,5-diamino-1-(2-hydroxyethyl)-1 H- pyrazol, 4-amino-m-cresol, 6-amino-m-cresol, 5-amino-6-chloro-cresol, 2,4,5,6-tetraaminopyrimidine, 2-hydroxy-4,5,6-triaminopyrimidine, 4-hydroxy-2,5,6-triaminopyrimidine sulfate.

Preferred oxidation dye precursors of the coupler type for a combination with a cationic dye of formula (1), (2) or (3) are for example m-phenylendiamine derivatives, naphthole, resorcine and resorcine derivatives, pyrazolone and m-aminophenol derivatives.

Especially preferred coupler compounds for a combination with a cationic dye of formula (1), (2) or (3) are N-(3-dimethylamino-phenyl)-urea, 4-amino-2-hydroxytoluene, 2-methyl-5-hydroxyethylaminophenol, 2,4-diaminophenoxyethanol, 2-amino-4-[(2-hydroxyethyl)amino]-anisole, p-aminophenol, m-aminophenol and its derivatives, especially 5-amino-2-methylphenol, 5-(3- hydroxypropylamino)-2-methylphenol, 3-amino-2-chloro-6-methylphenol, 2-hydroxy-4- aminophenoxyethanol, 2,6-dimethyl-3-aminophenol, 3-trifluoroacetylamino-2-chloro-6- methylphenol, 5-amino-4-chloro-2-methylphenol, 5-amino-4-methoxy-2-methylphenol, 5- (2'-hydroxyethyl)-amino-2-methylphenol, 3-(diethylamino)-phenol, N-cyclopentyl-3-aminophenol, 1,3-dihydroxy-5-(methylamino)-benzene, 3-(ethylamino)-4-methylphenol and 2,4-dichloro-3-aminophenol, or
o-aminophenol and its derivatives, such as 5-methyl-2-(1-methylamino)-phenol, 3-dimethylamino- phenol, 3-diethylamino-phenol, 5-amino-2-methyl-phenol, 5-amino-4-fluor-2-methyl-phenol, 5-amino-4-methoxy-2-methyl-phenol, 5-amino-4-ethoxy-2-methyl-phenol, 3-amino-2,4-dichlor-phenol, 5-amino-2,4-dichlor-phenol, 3-amino-2-methyl-phenol, 3-amino-2-chlor-6-methyl-phenol, 3-amino-phenol, 2-[(3-hydroxyphenyl)amino]-acetamide, 5-[(2-hydroxyethyl)amino]-2- methyl-phenol, 3-[(2-hydroxyethyl)amino]-phenol, 3-[(2-methoxyethyl)amino]-phenol, 5-amino-2-ethyl-phenol, 2-(4-amino-2-hydroxyphenoxy)-ethanol, 5-[(3-hydroxypropyl)amino]-2- methyl-phenol, 3-[(2,3-dihydroxypropyl)amino]-2-methyl-phenol, 3-[(2-hydroxyethyl)amino]-2-methyl-phenol, m-diaminobenzene and its derivatives such as 2,4-diaminophenoxyethanol, 1,3-bis-(2,4-diaminophenoxy)-propane, 1-methoxy-2-amino-4-(2'-hydroxyethylamino)benzene, 1,3-bis-(2,4-diaminophenyl)-propane, 3-[(2-aminoethyl)amino]-aniline, 1,3-di(2,4-diaminophenoxy)-propane, 1,3-diamino-2,4-dimethoxy-benzene, 2,6-bis(2-hydroxyethyl)amino-toluene, di(2,4-diaminophenoxy)-methane, 3-[di(2-hydroxyethyl)amino]-aniline, 2,6-bis-(2-hydroxyethylamino)-1-methylbenzene and 1-amino-3-bis-(2'-hydroxyethyl)-aminobenzene, o-diaminobenzene and its derivatives such as 3,4-diaminobenzoic acid and 2,3-diamino-1-methylbenzene, 2,4-diamino-1-fluor-5-methyl- benzene, 2,4-diamino-1-methoxy-5-methylbenzene, 1-(2-aminoethoxy)-2,4- diaminobenzene, 2-amino-1-(2-hydroxyethoxy)-4-methylamino-benzene, 2,4-diaminophenoxy-acetic acid, 2,4-diamino-1-ethoxy-5- methylbenzene, 3-[(2-hydroxyethyl)amino]-aniline, 3,4-diamino-benzoic acid, 3,4-dihydro-6-hydroxy-1,4(2H)-benzoxazine, 6-amino-3,4-dihydro-1,4(2H)- benzoxazine, 2,4-diamino-1,5-di(2-hydroxyethoxy)-benzene, 2,4-diamino-1-(2-hydroxyethoxy)-5-methyl-benzene, 4-amino-2-di[(2-hydroxyethyl)amino]-1-ethoxy-benzene 2,4-di[(2-hydroxyethyl)amino]-1,5-dimethoxybenzene, 3,4-dihydro-6-hydroxy-1,4(2H)-benzoxazine, 6-amino-3,4-dihydro-1,4(2H)- benzoxazine,
di- or trihydroxybenzene derivatives such as resorcine, resorcinmonomethylether, 2-methylresorcine, 5-methylresorcine, 2,5-dimethylresorcine, 1-chloro-2,4-dihydroxy-benzene, 2-chlororesorcine, 4-chlororesorcine, 2, 6-dihydroxyethylaminotoluene, 1,2-dichlor-3,5-dihydroxy-4-methyl-benzene, 1,5-dichlor-2,4-dihydroxy- benzene, 1,3-dihydroxy-2-methylbenzene, pyrogallol and 1,2,4-trihydroxybenzene, pyridine derivatives such as 2,6-diamino-pyridine, 2,6-dihydroxypyridine, 2-amino-3-hydroxypyridine, 2-amino-5-chloro-3-hydroxypyridine, 5-amino-4-chloro-2-methyl-phenol, 3-diamino-6- methoxy-pyridine, 3-amino-2-methylamino-6-methoxypyridine, 2,6- dihydroxy-3,4-dimethylpyridine, 2,6-dihydroxy-4-methylpyridine, 2,6-diaminopyridine, 2,3-diamino-6-methoxypyridine, 2,6-diamino-3,5-dimethoxy-pyridine, and 3,5-diamino-2,6-dimethoxypyridine, naphthaline derivatives such as 1-naphthol, 2-methyl-1-naphthol, 2-hydroxymethyl-1-naphthol, 2-hydroxyethyl-1-naphthol, 1,5-dihydroxynaphthaline, 1,6-dihydroxynaphthaline, 1,7-dihydroxynaphthaline, 1,8-dihydroxynaphthaline, 2,7-dihydroxynaphthaline and 2,3-dihydroxynaphthaline, 2-methyl-1-naphthol-acetat,
morpholine derivatives such as 6-hydroxybenzomorpholine and 6-aminobenzomorpholine, chinoxaline derivatives such as 6-methyl-1,2,3,4-tetrahydrochinoxaline,
pyrazol derivatives such as -phenyl-3-methylpyrazol-5-one, 3-methyl-1-phenyl-5-pyrazolone, indol derivatives such as 4-hydroxyindol, 5-hydroxy-indol, 6-hydroxyindol and 7-hydroxyindol, 2,3-indolindione, 5,6-dihydroxy-indol, 5,6-dihydroxy-indoline,
methylendioxybenzene derivates such as 1-hydroxy-3,4-methylendioxybenzene, 1-amino-3,4-methylendioxybenzene and 1-(2'-hydroxyethyl)-amino-3,4-methylendioxybenzene, 3,4-methylendioxy-phenol, 3,4-methylendioxy-aniline, 5-[(2-hydroxyethyl)amino]-1,3-benzodioxol, 6-brom-1-hydroxy-3,4-methylendioxy-benzene, or
cationic coupler compounds as described in FR 2 794 644, especially on page 11, line 20 to page 15, line 34, and on page 17, lines 4 to 12, page 178, line 33 to page 18, line 24.

More especially preferred coupler compounds for a combination with a cationic dye of formula (1), (2) or (3) are toluene-2,5-diamine sulfate, 1-naphthol, 1,5-, 2,7- and 1,7-dihydroxynaphthaline, 3-aminophenol, 5-amino-2-methylphenol, 2-amino-3-hydroxypyridine, resorcinol, 4-chlororesorcine, 2-chloro-6-methyl-3-aminophenol, 2,6-dihydroxyethylaminotoluene, 2-methyl-5-dihydroxyethylaminophenol, 2,4-diaminophenoxyethylol hydrochloride, 2-methylresorcine, 5- methylresorcine, 2,5-dimethylresorcine, 3,4-methylenedioxyphenol, 2-amino-4-hydroxyethylaminoanisole sulfate, 2,6-di-(beta -hydroxyethylamino)-toluene, 4-amino-2-hydroxytoluene, 6-hydroxyindol, 2-amino-3hydroxypyridine, 2,6-dimethoxy-3,5-pyridinediamine hydrochloride and 2,6-dihydroxy-3,4-dimethylpyridine.

Most preferred coupler compounds for a combination with a cationic dye of formula (1), (2) or (3) are 2-chloro-6-methyl-3-aminophenol, 5-amino-2- methylphenol, 2-amino-3-hydroxypyridine, 2,6-di-(beta -hydroxyethylamino)-toluol, 2- methylresorcine and 1-naphthol.
Further, preferred for a combination with a cationic dye of formula (1), (2) or (3) are:
- the developer/-coupler combination 2,4,5,6-Tetraaminopyrimidine and 2-Methylresorcine are preferred for assessing of red shades, or
- p-Toluenediamine and 4-Amino-2-hydroxytoluene for assessing of blue-violet shades, or
- p-Toluenediamine and 2-Amino-4-hydroxyethylaminoanisole for assessing of blue shades,or
- p-Toluenediamine and 2,4-Diamino-phenoxyethynol for assessing of blue shades, or
- 3-Methyl-4-aminophenol and 4-Amino-2-hydroxytlouene for assessing of orange shades, or
- p-Toluenediamine and resorcine for assessing of brown-green shades, or
- p-Toluenediamine and 1-Naphthol for assessing of blue-violet shades, or
- p-Toluenediamine and 2-methylresorcine for assessing of brown-gold shades.

Further, one preferred embodiment of the present invention concerns the combination of a compound of formula (1), (2) or (3) according to the present invention with autooxidizable compounds, such as, for example benzene, indol, or indoline, especially 5,6-dihydroxyindol or 5,6-dihydroxyindoline derivatives as described in WO 99/20234, especially on page 26, line 10 to page 28, line 15, or in WO 00/28957 on page 2, third paragraph.

Preferred autooxidizable benzene derivatives for a combination with a cationic dye of formula (1), (2) or (3) are:
1,2,4-trihydroxybenzene, 1-methyl-2,4,5-trihydroxybenzene, 2,4-diamnio-6-methylphenol, 2-amino-4-methylaminophenol, 2,5-diamino-4-methyl-phenol, 2,6-diamino-4-diethylamino-phenol, 2,6-diamino-1,4-dihydroxybenzen, and the salts of these compounds, which are accessible with acid.

Preferred autooxidizable indol derivatives for a combination with a cationic dye of formula (1), (2) or (3) are:
5,6-dihydroxyindol, 2-methyl-5,6-dihydroxyindol, 3-methyl-5,6-dihydroxyindole, 1-methyl-5,6-dihydroxyindol, 2,3-dimethyl-5,6-dihydroxyindol, 5-methoxy-6-dihydroxyindol, 5-acetoxy-6-hydroixyindol, 5,6-diacetoxyindol, acid of 5,6-dihydroxyindol-2-carbonacid, and the salts of these compounds, which are accessible with acid.

Preferred autooxidizable indoline derivatives for a combination with a cationic dye of formula (1), (2) or (3) are:
5,6-dihydroxyindoline, 1-methyl-5,6-dihydroxyindoline, 1-ethyl-5,6-dihydroxyindoline, and the salts of these compounds, which are accessible with acid.
A compound of formula (1), (2) or (3) according to the present invention can also be combined with at least two different developers and at least one coupler compound, or with at least two different couplers and at least one developer compound. Such combinations are for example described in German Patent Application 197 172 24, especially on page 3, line 31 to page 5, line 8.

In addition, a compound of formula (1), (2) or (3) according to the present invention may also be combined with naturally occurring dyes, such as, for example, henna red, henna neutral, henna black, camomile blossom, sandalwood, black tea, Rhamnus frangula bark, sage, campeche wood, madder root, catechu, sedre and alkanet root. Such colouring methods are described, for example, in EP-A-404 868, especially on page 3, line 55 to page 4, line 9.

Further, a compound of formula (1), (2) or (3) may also be combined with capped diazotised compounds.

Capped diazonium compounds that come into consideration include, for example, antidiazotates of formula 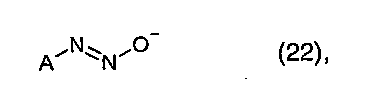 diazosulfonates of formula 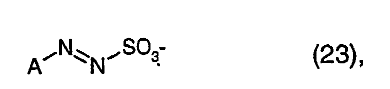 triazenes of formula 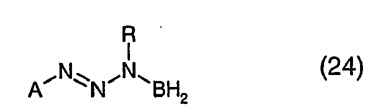 and also cyclic triazenes of formula 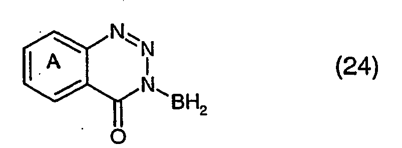 or

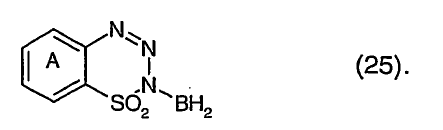

In formula (22) to (25):
A is the radical of an unsubstituted or substituted, aromatic or heterocyclic aryl,
B is the radical of an unsubstituted or substituted, water-soluble, aliphatic or aromatic aryl and
R is an unsubstituted or substituted alkyl group,
it being necessary for at least one of the groups to contain a water-solubilising radical.

As a water-solubilising radical there comes into consideration, for example, SO₃H, COOH, OH or a quaternised ammonium radical of formula 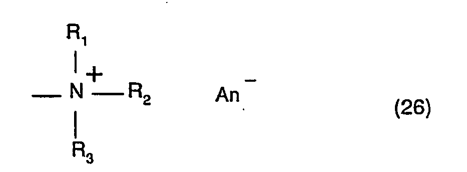 wherein R₁, R₂ and R₃ are each independently of the others unsubstituted or substituted alkyl and An is an anion.

According to the invention, alkyl groups R, R₁, R₂ and R₃ are to be understood generally as open-chain or branched alkyl radicals, for example methyl, ethyl, n- and iso-propyl or n-, sec- and tert-butyl.

Such alkyl radicals may be mono- or poly-substituted, for example by hydroxy, carboxy, halogen, cyano or C₁-C₄alkoxy.

Preferably, the alkyl groups are unsubstituted and each has from 1 to 4, especially 1 or 2, carbon atoms.

As anion An there come into consideration both inorganic and organic anions, for example halide, such as chloride, bromide or iodide, sulfate, hydrogen sulfate, methyl sulfate, formate, acetate or lactate.

The anion is generally governed by the preparation process. Preferably, it is chloride, hydrogen sulfate, sulfate, methosulfate or acetate.

A is the radical of an unsubstituted or substituted, aromatic or heterocyclic aryl. Suitable radicals include, for example, unsubstituted or substituted radicals of benzene, radicals at position 1- or 2- of naphthalene, radical at position 2- of thiophene, radical at position 2-of 1,3-thiazole, radical at position 5- of 1,2-thiazole, radical at position 2-of 1,3-benzothiazole, radical at position 1- of 2,3-benzothiazole, radical at position 2-of aminoimidazole, radical at position 2-of 1,3,4-thiadiazole, radical at position 2-of 1,3,5-thiadiazole, radical at position 2-of 1,3,4-triazole, radical at positions 3-, 7- or 8-of pyrazole, radical at position 2-of benzimidazole, radical at position 2-of benzopyrazole, radical at positions 2- or 4-of pyridine, radical at positions 2-,3-,4-, 7- or 8-of quinoline, radical at position 2-of aminopyrimidine and radical at position 3-of aminoisoxazole, radical at position 5-of aminoquinoline, radical at position 4-of aminodiphenylamine, radical at position 2-of aminodiphenyl ether and radical at position 4-of aminoazobenzene.

Such radicals may be mono- or poly-substituted, for example by C₁-C₄alkyl, C₁-C₄alkoxy, C₁-C₄alkylthio, halogen, for example fluorine, bromine or chlorine, nitro, trifluoromethyl, CN, SCN, C₁-C₄alkylsulfonyl, phenylsulfonyl, benzylsulfonyl, di-C₁-C₄alkylaminosulfonyl, C₁-C₄-alkylcarbonylamino, C₁-C₄alkoxysulfonyl or by dihydroxy-C₁-C₄alkylaminosulfonyl.

B is the radical of an unsubstituted or substituted, water-soluble, aliphatic or aromatic aryl, suitable aliphatic aryls including especially those carrying a carboxylic acid or sulfonic acid group, for example starting materials for B radicals are: methylaminoacetic acid (sarcosine), methylaminobutyric acid, methylaminopropionic acid, ethylaminoacetic acid, ethylaminobutyric acid, 1-methylaminoethane-2-sulfonic acid, 1-ethylaminoethane-2-sulfonic acid and 1-methylaminopropane-3-sulfonic acid.

As starting materials for B radicals come into consideration especially aniline compounds and aminonaphthalene compounds, especially those carrying a carboxylic acid or sulfonic acid group. The amino group of such compounds may be unsubstituted but is preferably substituted, for example by unsubstituted or substituted C₁-C₄alkyl, suitable substituents thereof being especially hydroxy or carboxy.

Suitable coupling components include, for example, the coupling components customarily used for azo dyes and known from the relevant literature, for example those of the benzene series, the naphthalene series, the open-chain methylene-active compounds (for example acylacetarylamides) and the heterocyclic series.

The compounds of formula (22), (23), (24) and (25) are known, or can be prepared in a manner known *per se.*

The compounds of formula (23) wherein B is the radical of an aliphatic aryl are likewise known or can be prepared in a manner known *per se.*

The compounds of formula (27) 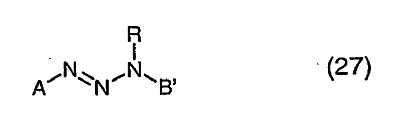 wherein
A is the radical of an unsubstituted or substituted, water-soluble, aromatic or heterocyclic aryl,
B' is the radical of an unsubstituted or substituted, water-soluble, aliphatic or aromatic aryl, and
R is an unsubstituted or substituted alkyl group, can likewise be prepared in a manner known *per se*.

For A and R, the definitions and preferred meanings indicated for formula (22) to (25) apply and, for B', the definitions and preferred meanings indicated for B in formula (22) to (25) apply in so far as they relate to aromatic amines.
The compounds of formula (27) can be prepared, for example, by diazotising an amine of formula A-NH₂ in customary manner and coupling it to an amine of formula B'-NHR, there coming into consideration as amines B'-NHR only those compounds which couple at the nitrogen atom and not at a carbon atom of the aromatic ring. Such compounds are preferably aniline derivatives substituted in the 4 positions.

The present invention also describes formulations, which are used for the colouration of keratin fibres, especially human hair. The formulations are applicable on human hair in different technical forms. The specific technical form may be chosen in view of the envisaged application and/or dye or dye composition. Technical forms of formulation are for example a solution, especially a thickened watery or watery alcoholic solution, a cream, foam, shampoo, powder, a gel, or an emulsion.

Preferred forms of formulations are ready to use compositions or a multi-compartment dyeing device or 'kit' or any of the multi-compartment packaging systems with compartments as described for example as described in US 6,190,421, column 2, lines 16 to 31.

It is of advantage to prepare compositions of dyes, which are not stable to reduction, with oxidizing agent free compositions just before the dyeing process.

One preferred embodiment of the present invention concerns the formulation of dyes, especially those of formula (1), (2) or (3) in powder form.

The colouring compositions according to the invention may furthermore comprise any active ingredient, additive or adjuvant known for such preparations.

Adjuvants that are suitable for such formulations are in general customary in the field hair-colouring, such as for example surfactants or tensides, solvents, bases, acids, perfumes, polymeric adjuvant, thickeners and light stabilisers.

Preferred combinations of the colouring compositions according to the invention with adjuvant used in the colouring of hair, are combination of direct dyes with oxidizing agents to achieve lightened colouration; wherein oxidizing agents especially described in WO 97/20545, especially page 9, lines 5 to 9, combination of direct dyes and/or an oxidation dye and oxidizing agents in the form of permanent-wave fixing solution, especially oxidizing agents as described in DE-A-19 713 698 , especially page 4, lines 52 to 55, or EP-A-1 062 940, especially page 6, lines 41 to 47, (and in the equivalent WO 99/40895), oxidation dyes in the presence of oxidoreductase enzyme, as described in WO 99/17730, especially page 4, line 11 to page 13, line 28, and WO 99/36034, especially pages 3 to 15, combination of cationic dyes with polyols or polyethers; polyols or polyethers as described in EP-A-962 219, especially page 27, lines 14 to 38, thickening polymers, as described in EP-A-970 684, especially page 48, line 16 to page 51, line 4,
sugar-containing polymers, as described in EP-A-970 687, especially page 28, line 17 to page 29, line 23,
quaternary ammonium salts, as described in WO 00/10517, especially page 44, line 16 to page 46, line 23,
anionic surfactants, as described in WO 00/10518, especially page 45, line 11 to page 48, line 3,
non-ionic surfactants, as described in WO 00/10519, especially page 45, line 11 to page 50, line 12, or
silicones, as described in WO 00/12057, especially page 45, line 9 to page 55, line 2. oxidative agent or laser and direct dyes, as described in EP-920 856, especially on page 2, line 31 to page 53 line 36, and on page 49, line 38 to page 50, line 41, with direct dyes as described on page 3, line 54 to page 48, line 52, or
direct dyes in the presence of cationic amphotere, substantive polymer, as described in EP-953 334, especially on page 2, line 39 to page 7, line 44, with direct dyes as described on page 8, line 54 to page 27, line 16, and polymers as described on page 27, line 17 to page 30, line 14, or
direct dyes formulations with polymer thickener on the basis of acrylic acid, as described in EP-970 685, especially on page 2, line 39 to page 10, line 1, with direct dyes as described on page 10, line 7 to page 48, line 15, with polymers as described on page 48, line 17 to page 49, line 28.

The colouring composition according to the invention in many cases comprises at least one surfactant. Suitable surfactants are anionic, zwitterionic, ampholytic, non-ionic and cationic surfactants. In many cases, however, it has proved advantageous to select the surfactants from anionic, zwitterionic and non-ionic surfactants.

Anionic surfactants suitable for use in the colouring compositions according to the invention include all anionic surface-active substances that are suitable for use on the human body. Such substances are characterised by an anionic group that imparts water solubility, for example a carboxylate, sulfate, sulfonate or phosphate group, and a lipophilic alkyl group having approximately from 10 to 22 carbon atoms. In addition, glycol or polyglycol ether groups, ester, ether and amide groups and also hydroxy groups may be present in the molecule. The following are examples of suitable anionic surfactants, each in the form of sodium, potassium or ammonium salts or mono-, di- or tri-alkanolammonium salts having 2 or 3 carbon atoms in the alkanol group:
- linear fatty acids having from 10 to 22 carbon atoms (soaps),
- ether carboxylic acids of formula R-O-(CH₂-CH₂-O)ₓ-CH₂-COOH, in which R is a linear alkyl group having from 10 to 22 carbon atoms and x = 0 or from 1 to 16,
- acyl sarcosides having from 10 to 18 carbon atoms in the acyl group,
- acyl taurides having from 10 to 18 carbon atoms in the acyl group,
- acyl isothionates having from 10 to 18 carbon atoms in the acyl group,
- sulfosuccinic mono- and di-alkyl esters having from 8 to 18 carbon atoms in the alkyl group and sulfosuccinic monoalkylpolyoxyethyl esters having from 8 to 18 carbon atoms in the alkyl group and from 1 to 6 oxyethyl groups,
- linear alkane sulfonates having from 12 to 18 carbon atoms,
- linear α-olefin sulfonates having from 12 to 18 carbon atoms,
- α-sulfo fatty acid methyl esters of fatty acids having from 12 to 18 carbon atoms,
- alkyl sulfates and alkyl polyglycol ether sulfates of formula R'-O(CH₂-CH₂-O)_{x'}-SO₃H, in which R' is a preferably linear alkyl group having from 10 to 18 carbon atoms and x' = 0 or from 1 to 12,
- mixtures of surface-active hydroxysulfonates according to DE-A-3 725 030, especially page 3, lines 40 to 55,
- sulfated hydroxyalkylpolyethylene and/or hydroxyalkylenepropylene glycol ethers according to DE-A-3 723 354, especially page 4, lines 42 to 62,
- sulfonates of unsaturated fatty acids having from 12 to 24 carbon atoms and from 1 to 6 double bonds according to DE-A-3 926 344, especially page 2, lines 36 to 54,
- esters of tartaric acid and citric acid with alcohols which are addition products of approximately from 2 to 15 molecules of ethylene oxide and/or propylene oxide with fatty alcohols having from 8 to 22 carbon atoms, or
- anionic surfactants, as described in WO 00/10518, especially page 45, line 11 to page 48, line 3.

Preferred anionic surfactants are alkyl sulfates, alkyl polyglycol ether sulfates and ether carboxylic acids having from 10 to 18 carbon atoms in the alkyl group and up to 12 glycol ether groups in the molecule, and also especially salts of saturated and especially unsaturated C₈-C₂₂carboxylic acids, such as oleic acid, stearic acid, isostearic acid and palmitic acid.
Surface-active compounds that carry at least one quaternary ammonium group and at least one -COO⁽⁻⁾ or -SO₃⁽⁻⁾ group in the molecule are termed zwitterionic surfactants. Zwitterionic surfactants that are especially suitable are the so-called betaines, such as the N-alkyl-N,N-dimethylammonium glycinates, for example cocoalkyldimethylammonium glycinate, N-acylaminopropyl-N,N-dimethylammonium glycinates, for example cocoacylaminopropyldimethylammonium glycinate, and 2-alkyl-3-carboxymethyl-3-hydroxyethylimidazolines having from 8 to 18 carbon atoms in the alkyl or acyl group and also cocoacylaminoethylhydroxyethylcarboxymethyl glycinate. A preferred zwitterionic surfactant is the fatty acid amide derivative known by the CTFA name cocoamidopropyl betaine.

Ampholytic surfactants are to be understood as meaning surface-active compounds that, in addition to a C₈-C₁₈-alkyl or -acyl group, contain at least one free amino group and at least one -COOH or -SO₃H group in the molecule and are capable of forming internal salts. Examples of suitable ampholytic surfactants include N-alkylglycines, N-alkylpropionic acids, N-alkylaminobutyric acids, N-alkyliminodipropionic acids, N-hydroxyethyl-N-alkylamidopropylglycines, N-alkyltaurines, N-alkylsarcosines, 2-alkylaminopropionic acids and alkylaminoacetic acids, each having approximately from 8 to 18 carbon atoms in the alkyl group. Ampholytic surfactants to which special preference is given are N-cocoalkylaminopropionate, cocoacylaminoethylaminopropionate and C₁₂-C₁₈acylsarcosine.

Non-ionic surfactants are described in WO 00/10519, especially page 45, line 11 to page 50, line 12.
Non-ionic surfactants contain as the hydrophilic group, for example, a polyol group, a polyalkylene glycol ether group or a combination of polyol and polyglycol ether groups.

Such compounds are, for example:
- addition products of from 2 to 30 mol of ethylene oxide and/or from 0 to 5 mol of propylene oxide with linear fatty alcohols having from 8 to 22 carbon atoms, with fatty acids having from 12 to 22 carbon atoms and with alkylphenols having from 8 to 15 carbon atoms in the alkyl group,
- C₁₂-C₂₂ fatty acid mono- and di-esters of addition products of from 1 to 30 mol of ethylene oxide with glycerol,
- C₈-C₂₂alkyl-mono- and -oligo-glycosides and ethoxylated analogues thereof,
- addition products of from 5 to 60 mol of ethylene oxide with castor oil and hydrogenated castor oil,
- addition products of ethylene oxide with sorbitan fatty acid esters,
- addition products of ethylene oxide with fatty acid alkanolamides.

Examples of cationic surfactants that can be used in the colouring compositions according to the invention are especially quaternary ammonium compounds. Preference is given to ammonium halides, such as alkyltrimethylammonium chlorides, dialkyldimethylammonium chlorides and trialkylmethylammonium chlorides, for example cetyltrimethylammonium chloride, stearyltrimethylammonium chloride, distearyldimethy-lammonium chloride, lauryldimethylammonium chloride, lauryldimethylbenzylammonium chloride and tricetylmethylammonium chloride. Further cationic surfactants that can be used in accordance with the invention are quaternised protein hydrolysates.

Also suitable in accordance with the invention are cationic silicone oils, such as, for example, the commercially available products Q2-7224 (manufacturer: Dow Corning; a stabilised trimethylsilylamodimethicone), Dow Corning 929 emulsion (comprising a hydroxylamino-modified silicone, which is also referred to as amodimethicone), SM-2059 (manufacturer:
General Electric), SLM-55067 (manufacturer: Wacker) and also Abil^{®}-Quat 3270 and 3272 (manufacturer: Th. Goldschmidt; diquaternary polydimethylsiloxanes, quaternium-80), or silicones, as described in WO 00/12057, especially page 45, line 9 to page 55, line 2.

Alkylamidoamines, especially fatty acid amidoamines, such as the stearylamidopropyldimethylamine obtainable under the name Tego Amid^{®} 18, are distinguished not only by a good conditioning action but also especially by their good biodegradability.

Quaternary ester compounds, so-called "esterquats", such as the methyl hydroxyalkyldialkoyloxyalkylammonium methosulfates marketed under the trademark Stepantex^{®}, are also very readily biodegradable.
An example of a quaternary sugar derivative that can be used as cationic surfactant is the commercial product Glucquat^{®}100, according to CTFA nomenclature a "lauryl methyl gluceth-10 hydroxypropyl dimonium chloride". The alkyl-group-containing compounds used as surfactants may be single substances, but the use of natural raw materials of vegetable or animal origin is generally preferred in the preparation of such substances, with the result that the substance mixtures obtained have different alkyl chain lengths according to the particular starting material used.

The surfactants that are addition products of ethylene and/or propylene oxide with fatty alcohols or derivatives of such addition products may either be products having a "normal" homologue distribution or products having a restricted homologue distribution. "Normal" homologue distribution is to be understood as meaning mixtures of homologues obtained in the reaction of fatty alcohol and alkylene oxide using alkali metals, alkali metal hydroxides or alkali metal alcoholates as catalysts. Restricted homologue distributions, on the other hand, are obtained when, for example, hydrotalcites, alkali metal salts of ether carboxylic acids, alkali metal oxides, hydroxides or alcoholates are used as catalysts. The use of products having restricted homologue distribution may be preferred.

Further preferred active ingredients of formulation according to the present invention, adjuvants and additives are as follows:
- non-ionic polymers, for example vinylpyrrolidone/vinyl acrylate copolymers, polyvinylpyrrolidone and vinylpyrrolidone/vinyl acetate copolymers and polysiloxanes,
- cationic polymers, such as quaternised cellulose ethers, polysiloxanes having quaternary groups, dimethyldiallylammonium chloride polymers, copolymers of dimethyldiallylammonium chloride and acrylic acid, as available commercially under the name Merquat^{®} 280 and the use of which in hair colouring is described, for example, in DE-A-4 421 031, especially page 2, lines 20 to 49, or EP-A-953 334, especially page 27, line 17 to page 30, line 11, acrylamide/dimethyldiallylammonium chloride copolymers, diethyl-sulfate-quaternised dimethylaminoethyl methacrylate/vinylpyrrolidone copolymers, vinylpyrrolidone/imidazolinium methochloride copolymers,
- quaternised polyvinyl alcohol,
- zwitterionic and amphoteric polymers, such as, for example, acrylamidopropyl-trimethylammonium chloride/acrylate copolymers and octylacrylamide/methyl methacrylate/tert-butylaminoethyl methacrylate/2-hydroxypropyl methacrylate copolymers,
- anionic polymers, such as, for example, polyacrylic acids, crosslinked polyacrylic acids, vinyl acetate/crotonic acid copolymers, vinylpyrrolidone/vinyl acrylate copolymers, vinyl acetate/butyl maleate/isobornyl acrylate copolymers, methyl vinyl ether/maleic anhydride copolymers and acrylic acid/ethyl acrylate/N-tert-butyl acrylamide terpolymers,
- thickeners, such as agar, guar gum, alginates, xanthan gum, gum arabic, karaya gum, locust bean flour, linseed gums, dextrans, cellulose derivatives, e.g. methyl cellulose, hydroxyalkyl cellulose and carboxymethyl cellulose, starch fractions and derivatives, such amylose, amylopectin and dextrins, clays, e.g. bentonite or fully synthetic hydrocolloids such as, for example, polyvinyl alcohol,
- structuring agents, such as glucose and maleic acid,
- hair-conditioning compounds, such as phospholipids, for example soya lecithin, egg lecithin, and cephalins, silicone oils, and also conditioning compounds, for example such as those described in DE-A-19 729 080, especially page 2, lines 20 to 49, EP-A-834 303, especially page 2, line 18 to page 3, line 2, or EP-A-312 343, especially page 2, line 59 to page 3, line 11,
- protein hydrolysates, especially elastin, collagen, keratin, milk protein, soya protein and wheat protein hydrolysates, condensation products thereof with fatty acids and also quaternised protein hydrolysates,
- perfume oils, dimethyl isosorbitol and cyclodextrins,
- solubilisers, such as ethanol, isopropanol, ethylene glycol, propylene glycol, glycerol and diethylene glycol,
- anti-dandruff active ingredients, such as piroctones, olamines and zinc Omadine,
- further substances for adjusting the pH value,
- active ingredients such as panthenol, pantothenic acid, allantoin, pyrrolidonecarboxylic acids and salts thereof, plant extracts and vitamins,
- cholesterol,
- light stabilisers and UV absorbers, as described, for example, in EP-A-819 422, especially page 4, lines 34 to 37,
- consistency regulators, such as sugar esters, polyol esters or polyol alkyl ethers,
- fats and waxes, such as spermaceti, beeswax, montan wax, paraffins, fatty alcohols and fatty acid esters,
- fatty alkanolamides,
- polyethylene glycols and polypropylene glycols having a molecular weight of from 150 to 50 000, for example such as those described in EP-A-801 942, especially page 3, lines 44 to 55,
- complexing agents, such as EDTA, NTA and phosphonic acids,
- swelling and penetration substances, such as polyols and polyol ethers, as listed extensively, for example, in EP-A-962 219, especially page 27, lines 18 to 38, for example glycerol, propylene glycol, propylene glycol monoethyl ether, butyl glycol, benzyl alcohol, carbonates, hydrogen carbonates, guanidines, ureas and also primary, secondary and tertiary phosphates, imidazoles, tannins, pyrrole,
- opacifiers, such as latex,
- pearlising agents, such as ethylene glycol mono- and di-stearate,
- propellants, such as propane-butane mixtures, N₂O, dimethyl ether, CO₂ and air, and also
- antioxidants,
- polyols or polyethers, as described in EP-A-962 219, especially page 27, lines 14 to 38,
- thickening polymers, as described in EP-A-970 684, especially page 48, line 16 to page 51, line 4,
- sugar-containing polymers, as described in EP-A-970 687, especially page 28, line 17 to page 29, line 23,
- quaternary ammonium salts, as described in WO 00/10517, especially page 44, line 16 to page 46, line 23.

In the context of the present invention, oxidizing agents are understood to be any oxidizing agent customarily used for oxidative hair colouring, for example dilute hydrogen peroxide solutions, hydrogen peroxide emulsions or hydrogen peroxide gels, alkaline earth metal peroxides, organic peroxides, such as urea peroxides, melamine peroxides, or alkalimetalbromat fixations are also applicable if a shading powder on the basis of semi-permanent, direct hair dyes is used.

Preferred oxidizing agent is hydrogen peroxide, preferred in about 2 to 30 % by weight, more preferred in 3 to 20% by weight, and most preferred in 6 to 12% by weight of the total weight of a watery composition such as a solution, dispersion, a gel or emulsion.

The watery composition can comprise all customary components, which are used for the different applications of oxidizing agent compositions as described in K. Schrader, "Grundlagen und Rezepturen der Kosmetika", 2. Aufl. (1989), page 832-840.

Further preferred oxidizing agents are oxidizing agents to achieve lightened colouration, as described in WO 97/20545, especially page 9, lines 5 to 9,
oxidizing agents in the form of permanent-wave fixing solution, as described in DE-A-19 713 698 , especially page 4, lines 52 to 55, and lines 60 and 61 or EP-A-1 062 940, especially page 6, lines 41 to 47, (and in the equivalent WO 99/40895).

An oxidizing agents may be present in the colouring compositions according to the invention preferably in an amount of from 0.01 % to 6 %, especially from 0.01 % to 1 %, based on the total dyeing composition.

Preferred catalysts are metal ions, such as for example Zn²⁺, Cu²⁺, Fe²⁺, Fe³⁺, Mₙ²⁺, Mₙ⁴⁺, Li⁺, Mg²⁺, Ca²⁺ and Al³⁺, preferably Zn²⁺, Cu²⁺ and Mn²⁺.

The metal ions are applicable in any physiological suitable salts form. Preferred salts are acetate, sulfate, halogenide, lactate and tartrate.

Alkalimetalsulfits, such as sodium-, potassium-, lithium-sulfite, Alkalimetaldisulfits, such as sodium-, potassium-, lithium-disulfite, ascorbic acid, tert.-Butylhydrochinon and Ammoniumthiolactat.

In general, the coloration with an oxidative agent is conducted in the presence of a base. Bases are for example ammonia, alkali metal carbonates, earth metal carbonates, alkanol amines, such as for example mono-, di- or triethanolamine, alkali metal hydroxides, earth metal hydroxides, compounds of the formula 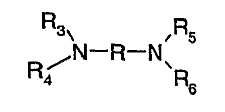 wherein,
R is a propyl residue, which substituted with OH or C₁-C₄-alkyl,
R₃, R₄, R₅ and R₆ are independently or dependently from each other hydrogen, C₁-C₄-alkyl or hydroxy-(C₁-C₄)-alkyl.

Alkali metal is for example sodium, potassium or lithium.
Earth metal is for example magnesium or calcium.

Acids are inorganic or organic acids, such as hydrochloride, tartrat acid, citric acid, ascorbic acid and phosphor acid.

The use of UV absorbers can effectively protect natural and dyed hair from the damaging rays of the sun and increase the wash fastness of dyed hair.

Preferred UV absorbers the colouring compositions according to the invention are:
- cationic benzotriazole UV absorbers as for example described in WO 01/36396 especially on page 1, line 20 to page 2, line 24, and preferred on page 3 to 5, and on pages 26 to 37, or
- cationic benzotriazole UV in combination with antioxidants as described in WO 01/36396, especially on page 11, line 14 to page 18, or
- UV absorbers in combination with antioxidants as described in US Patent 5 922 310, especially in column 2, lines 1 to 3,
- UV absorbers in combination with antioxidants as described in US Patent 4 786 493, especially in column 1, 42 to column 2, line 7, and preferred in column 3, 43 to column 5, line 20, or
- combination of UV absorbers as described in US Patent 5 830 441, especially in column 4, lines 53 to 56, or
- combination of UV absorbers as described in WO 01/36396, especially on page 11, lines 9 to 13, or
- triazine derivatives provide effective UV protection as described in WO 98/22447, especially on page 1, line 23 to page 2, line 4, and preferred on page 2, line 11 to page 3, line 15 and most preferred on pages 6 to 7, and 12 to 16, or
- combination of the cosmetic formulations as described in WO 98/22447 with one or more than one further UV filter as described in the following patents:
(Abbreviations T: table, R: row, Comp: compound, Ex: compound(s) of patent example, p = page; pp = pages)

| | |
|---|---|
| EP 895776 | Comp. in Rows 48-58, p 3; R 25+33, p 5 |
| WO 9220690 | Polymeric comp in Examples 3-6 |
| EP 1000950 | Comp. in Table 1, pp 18-21 |
| EP 1060734 | T 1-3, pp 11-14 |
| EP 1059082 | Ex 1; T 1, pp 9-11 |
| EP 1008586 | Ex 1-3, pp 13-15 |
| EP 1005855 | T3, p13 |
| EP 1129695 | Ex 1-7, pp 13-14 |
| EP 967200 | Ex 2; T 3-5, pp 17-20 |
| EP 945125 | T 3 a+b, pp 14-15 |
| EP 924246 | T2, p 9 |
| EP 911020 | T 2, p 11-12 |
| EP 916335 | T 2-4, pp 19-41 |
| EP 852137 | T 2, pp 41-46 |
| EP 858318 | T1, p6 |
| EP 826361 | T 1, pp 5-6 |
| EP 503338 | T 1, pp 9-10 |
| WO 9301164 | T 1+2, pp 13-22 |
| EP 823418 | Ex 1-4, pp 7-8 |
| WO 9714680 | Ex 1-3, p 10 |
| EP 1027883 | Compound VII, p 3 |
| EP 832641 | Ex 5+6 p 7; t 2, p 8 |
| US 5338539 | Ex 1-9, pp 3+4 |
| EP 517103 | Ex 3,4,9,10 pp 6-7 |
| EP 1123934 | T3, p10 |
| EP 1027883 | Comp I-VI, p 3 |
| EP 969004 | Ex 5, T 1, pp 6-8 |
| US 5801244 | Ex 1-5, pp 6-7 |
| EP 832642 | Ex 22, T 3 pp, 10-15; T 4, p 16 |
| US 5346691 (EP 570838) | Ex 40, p 7; T 5, p 8 |
| EP 517104 | Ex 1, T 1, pp 4-5; Ex 8, T 2, pp 6-8 |
| WO 200149686 | Ex 1-5, pp 16-21 |
| EP 944624 | Ex 1+2, pp13-15 |
| EP 933376 | Ex 1-15, pp 10-21 |
| EP 863145 | Ex 1-11, pp 12-18 |
| EP 780382 | Ex 1-11, pp 5-7 |
| EP 626950 | All examples |
| EP 1081140 | Ex 1-9, pp 11-16 |
| WO 9217461 | Ex 1-22, pp 10-20 |
| WO 0168047 | Tables on pp 85-96 |
| EP 613893 | Ex 1-5 + 15, T 1, pp 6-8 |
| EP 1064922 | Compounds 1-34, pp 6-14 |
| EP 1028120 | Ex 1-5, pp 5-13 |
| EP 1008593 | Ex 1-8, pp 4-5 |
| EP 669323 | Ex 1-3, p 5 |
| EP 1108712 | 4,5-Dimorpholino-3-hydroxypyridazine |
| JP 2000319629 | CAS Regno. 80142-49-0,137215-83-9, 307947-82-6 |
| EP 420707 B1 | Ex 3, p 13 (80142-49-0) |
| US 5635343 | All examples |
| EP 1167358 | All examples |

A preferred embodiment of the present invention concerns the combination of a compound of formula (1), (2) or (3) with UV absorbers.
Preferred UV absorbers are described in WO 98/22447.
Preferred cosmetic formulations contain a combination of a compound of formula (1), (2) or (3) with UV absorbers and one or more than one further UV protective of the following substance classes:
p-aminobenzoic acid derivatives, for example 4-dimethylaminobenzoic acid 2-ethylhexyl ester;
salicylic acid derivatives, for example salicylic acid 2-ethylhexyl ester;
benzophenone derivatives, for example 2-hydroxy-4-methoxybenzophenone and its 5-sulfonic acid derivative;
dibenzoylmethane derivatives, for example 1-(4-tert-butylphenyl)-3-(4-methoxyphenyl)-propane-1,3-dione;
diphenylacrylates, for example 2-ethylhexyl 2-cyano-3,3-diphenylacrylate, and 3-(benzofuranyl) 2-cyanoacrylate;
3-imidazol-4-ylacrylic acid and esters;
benzofuran derivatives, especially 2-(p-aminophenyl)benzofuran derivatives, described in EP-A-582 189, US-A-5 338 539, US-A-5 518 713 and EP-A-613 893;
polymeric UV absorbers, for example the benzylidene malonate derivatives described in EP-A-709 080;
cinnamic acid derivatives, for example the 4-methoxycinnamic acid 2-ethylhexyl ester and
isoamyl ester or cinnamic acid derivatives described in US-A-5 601 811 and WO 97/00851; camphor derivatives, for example 3-(4'-methyl)benzylidene-bornan-2-one, 3-benzylidene-bornan-2-one, N-[2(and 4)-2-oxyborn-3-ylidene-methyl)-benzyl]acrylamide polymer, 3-(4'-trimethylammonium)-benzylidene-bornan-2-one methyl sulfate, 3,3'-(1,4-phenylenedimethine)-bis(7,7-dimethyl-2-oxo-bicyclo[2.2.1]heptane-1-methanesulfonic acid) and salts, 3-(4'-sulfo)benzylidene-bornan-2-one and salts; camphorbenzalkonium methosulfate;
hydroxyphenyltriazine compounds, for example 2-(4'-methoxyphenyl)-4,6-bis(2'-hydroxy-4'-n-octyloxyphenyl)-1,3,5-triazine; 2,4-bis{[4-(3-(2-propyloxy)-2-hydroxy-propyloxy)-2-hydroxy]-phenyl}-6-(4-methoxyphenyl)-1,3,5-triazine; 2,4-bis{[4-(2-ethyl-hexyloxy)-2-hydroxy]-phenyl}-6-[4-(2-methoxyethyl-carboxyl)-phenylamino]-1,3,5-triazine; 2,4-bis{[4-(tris(trimethylsilyloxysilylpropyloxy)-2-hydroxy]-phenyl}-6-(4-methoxyphenyl)-1,3,5-triazine; 2,4-bis{[4-(2"-methylpropenyloxy)-2-hydroxy]-phenyl}-6-(4-methoxyphenyl)-1,3,5-triazine; 2,4-bis{[4-(1',1',1',3',5',5',5'-heptamethyltrisilyl-2"-methyl-propyloxy)-2-hydroxy]-phenyl}-6-(4-methoxyphenyl)-1,3,5-triazine; 2,4-bis{[4-(3-(2-propyloxy)-2-hydroxy-propyloxy)-2-hydroxy]-phenyl}-6-[4-ethylcarboxy)-phenylamino]-1,3,5-triazine;
benzotriazole compounds, for example 2,2'-methylene-bis(6-(2H-benzotriazol-2-yl)-4-(1,1,3,3-tetramethylbutyl)-phenol;
trianilino-s-triazine derivatives, for example 2,4,6-trianiline-(p-carbo-2'-ethyl-1'-oxy)-1,3,5-triazine and the UV absorbers disclosed in US-A-5 332 568, EP-A-517 104, EP-A-507 691, WO 93/17002 and EP-A-570 838;
2-phenylbenzimidazole-5-sulfonic acid and salts thereof;
methyl o-aminobenzoates;
physical sunscreens coated or not as titanium dioxide, zinc oxide, iron oxides, mica, MnO, Fe₂O₃, Ce₂O₃, Al₂O₃, ZrO₂. (surface coatings: polymethylmethacrylate, methicone (methylhydrogenpolysiloxane CAS 9004-73-3), dimethicone, isopropyl titanium triisostearate (CAS 61417-49-0), metal soaps as magnesium stearate (CAS 4086-70-8), perfluoroalcohol phosphate as C9-15 fluoroalcohol phosphate (CAS 74499-44-8; JP 5-86984 , JP 4-330007)). The primary particle size is an average of 15nm-35nm and the particle size in dispersion is in the range of 100nm - 300nm.
aminohydroxy-benzophenone derivatives disclosed in DE 10011317, EP 1133980 and EP 1046391
phenyl-benzimidazole derivatives as disclosed in EP 1167358
The UV absorbers described in "Sunscreens", Eds. N.J. Lowe, N.A.Shaath, Marcel Dekker, Inc. , New York and Basle or in Cosmetics & Toiletries (107), 50ff (1992) also can be used as additional UV protective substances.

Synergistic effects are observed when UV absorbers are used in combination with antioxidants. Examples of antioxidants that can be used are listened in WO 01/36396 (pages 11-18), US Patent 5 922 310 and US Patent 4 786 493.

Further preferred UV absorbers used in addition to the uncharged and cationic benzotriazole UV absorbers in the formulations without limitation to those listed in the following are benzophenone-type substances such as benzophenone-1, benzophenone-2, benzophenone-3, benzophenone-4, benzophenone-5 (sodium salt) or benzotriazol-type substances such as benzenesulfonic acid, 3-(2H-benzotriazol-2-yl)-4-hydroxy-5-(1-methylpropyl)-, monosodium salt; 2-(5-chloro-2H-benzotriazol-2-yl)-6-(1,1-dimethylethyl)-4-methyl-phenol; 2-(2H-benzotriazol-2-yl)-6-dodecyl-4-methyl-phenol, branched and linear. Typical ingredients in the oil phase of emulsions (water in oil, oil in water or triple emulsion) or used in hair oils can be chosen from the following substance groups without limiting the kind of lipophilic ingredients to those substances:

Suitable cosmetic preparations may contain usually from 0.05 to 40 % by weight, preferably from 0.1 to 20 % by weight, based on the total weight of the composition, of one or more UV absorbers.

Preferred are the cosmetic preparations contain at least one triazine derivative UV absorber, for example, from 0.1 to 40 % by weight, preferably from 0.1 to 20 % by weight and especially from 0.5 to 10 % by weight, based on the total weight of the composition, and the cosmetic preparations contain at least one cationic benzotriazole from 0.05-20% by weight, preferred from 0.1-20% by weight, based on the total weight of the composition. Typical cosmetic formulations containing uncharged and/or cationic benzotriazoles and/or antioxidants alone or in combinations are rinse-off products (e.g. shampoos, hair rinses, conditioners etc.),
Suitable cosmetic formulations are:
- cosmetic hair-treatment preparations, e.g. hair-washing preparations in the form of shampoos and conditioners, hair-care preparations, e.g. pre-treatment preparations or leave-on products such as sprays, creams, gels, lotions, mousses and oils, or
- hair tonics, styling creams, styling gels, pomades, hair rinses, treatment packs, intensive hair treatments, hair-structuring preparations, e.g. hair-waving preparations for permanent waves (hot wave, mild wave, cold wave), hair-straightening preparations, liquid hair-setting preparations, hair foams, hairsprays, bleaching preparations, e.g. hydrogen peroxide solutions, lightening shampoos, bleaching creams, bleaching powders, bleaching pastes or oils, temporary, semi-permanent or permanent hair colourants, preparations containing self-oxidising dyes, or natural hair colourants, such as henna or camomile.
The final formulations listed may exist in a wide variety of presentation forms, for example:
- in the form of liquid preparations as a W/O, O/W, O/W/O, W/O/W or PIT emulsion and all kinds of microemulsions,
- in the form of a gel,
- in the form of an oil, a cream, milk or lotion,
- in the form of a powder, a lacquer, a tablet or make-up,
- in the form of a stick,
- in the form of a spray (spray with propellant gas or pump-action spray) or an aerosol,
- in the form of a foam, or
- in the form of a paste.

Of special importance as cosmetic preparations for the hair are the above-mentioned preparations for hair treatment, especially hair-washing preparations in the form of shampoos, hair conditioners, hair-care preparations, e.g. pre-treatment preparations, hair tonics, styling creams, styling gels, pomades, hair rinses, treatment packs, intensive hair treatments, hair-straightening preparations, liquid hair-setting preparations, hair foams and hairsprays. Of special interest are hair-washing preparations in the form of shampoos.

A shampoo has, for example, the following composition: from 0.01 to 5 % by weight of a UV absorber according to the invention, 12.0 % by weight of sodium laureth-2-sulfate, 4.0 % by weight of cocoamidopropyl betaine, 3.0 % by weight of sodium chloride, and water ad 100%.

A further embodiment of the present invention concerns micronised UV absorbers, for example:
- wet-grinding with a hard grinding medium, for example zirconium silicate and a protective surfactant or a protective polymer in water or in a suitable organic solvent;
- spray-drying from a suitable solvent, for example aqueous suspensions or suspensions containing organic solvents, or true solutions in water, ethanol, dichloroethane, toluene or N-methylpyrrolidone etc.;
- by the expansion according to the RESS process (Rapid Expansion of Supercritical Solutions) of supercritical fluids (e.g. CO₂ in which the UV filter or filters is/are dissolved, or the expansion of fluid carbon dioxide together with a solution of one or more UV filters in a suitable organic solvent;
- by reprecipitation from suitable solvents, including supercritical fluids (GASR process = Gas Anti-Solvent Recrystallisation / PCA process = Precipitation with Compressed Anti-solvents).

As grinding apparatus for the preparation of the micronised organic UV absorbers there may be used, for example, a jet mill, ball mill, vibratory mill or hammer mill, preferably a highspeed mixing mill. The grinding is preferably carried out with a grinding aid, for example an alkylated vinylpyrrolidone polymer, a vinylpyrrolidone/vinyl acetate copolymer, an acyl glutamate, an alkyl polyglucoside, ceteareth-25 or a phospholipid.

The micronised UV absorbers so obtained usually have an average particle size that is from 0.02 to 2 µm, preferably from 0.05 to 1.5 µm, and more especially from 0.1 to 1.0 µm.

The UV absorbers can also be used dry in powder form. For that purpose the UV absorbers are subjected to known grinding methods, such as vacuum atomization, countercurrent spray-drying etc. Such powders have a particle size of from 0.1 µm to 2µm. To avoid the occurrence of agglomeration, the UV absorbers can be coated with a surface-active compound prior to the pulverisation process, for example with an anionic, non-ionic or amphoteric surfactant, e.g. a phospholipid or a known polymer, such as PVP, or an acrylate.
The colouring compositions according to the invention may further comprise antimicrobial agents.

Preferred antimicrobial preservatives and antimicrobial actives used in formulations (in most cases the INCI name of the antimicrobial substances is mentioned):
formaldehyde and paraformaldehyde, hydroxy biphenyls and its salts such as orth ophenylphenol, zinc pyrithion, chlorobutanol, hydroxy benzoic acids and their salts and esters such as methyl paraben, ethyl paraben, propyl paraben, butyl paraben, dibromo hexamidine and its salts including isothionate (4,4'-hexamethylenedioxy-bis(3-bromo-benzamidine) and 4,4'-hexamethylenedioxy-bis(3-bromo-benzamidinium 2-hydroxyethanesulfonate), mercury, (aceto-O)phenyl (especially phenyl mercuric acetate) and Mercurate(2-),(orthoborate(3-)-O)phenyl, dihydrogene (especially phenyl mercuric borate), 1,3-bis(2-ethylhexyl)-hexahydro-5-methyl-5-pyrimidine (Hexetidin), 5-bromo-5-nitro-1,3-dioxan, 2-bromo-2-nitro-1,3-propandiol, 2,4-dichlorobenzyl alcohol, 3,4,4' trichlorocarbanilide (Trichlorcarban), p-chloro-m-cresol, 2,4,4'-trichloro 2-hydroxy diphenylether (triclosan), 4,4'-dichloro 2-hydroxy diphenylether, 4-chloro-3,5-dimethylphenol (Chloroxylenol), imidazolidinyl urea, poly-(hexamethylene biguanide) hydrochloride, 2-phenoxy ethanol (phenoxyethanol), hexamethylene tetramine (Methenamine), 1-(3-chloroallyl)-3,5,7-triaza-1-azoniaadamantanchloride (Quaternium 15), 1-(4-chlorophenyoxy)-1-(1-imidazolyl)3,3-dimethyl-2-butanone (Climbazole), 1,3-bis(hydroxymethyl)-5,5-dimethyl-2,4-imidazolidinedione (DMDM hydantoin), benzyl alcohol, 1,2-dibromo-2,4-dicyano butane , 2,2' methylene-bis(6-bromo-4-chloro phenol) (bromochlorophene), methylchloroisothiazolone, methylisothiazolone, octylisothiazolone, benzylisothiazolone, 2-benzyl-4-chlorophenol (Chlorophenone), chloracetamide, chlorhexidine, chlorhexidine acetate, chlorhexidine gluconate, chlorhexidine hydrochloride, 1-phenoxy-propane-2-ol (phenoxyisopropanol), 4,4-dimethyl-1,3-oxazolidine (dimethyl oxazolidine), diazolidinyl urea, 4,4'-hexamethylenedioxybisbenzamidine and 4,4'-hexamethylenedioxybis(benzamidinium-2-hydroxyethanesulfonate), glutaraldehyde (1,5-pentanedial), 7-ethylbicyclooxazolidine, 3-(4-chlorophenoxy)-1,2-propandiol (chlorophenesin), phenylmethoxymethanol and ((phenylmethoxy)methoxy)-methanol (benzylhemiformal), N-alkyl(C12-C22)trimethyl ammoniumbromide and -chloride (cetrimonium bromide, cetrimonium chloride), benzyl-dimethyl-(4-(2-(4-(1,1,3,3-tetramethylbutyl)-phenoxy)-ethoxy)-ethyl)-ammoniumchloride (benzethonium chloride), Alkyl-(C8-C18)-dimethyl-benzylammonium chloride, - bromide and saccharinate (benzalkonium chloride, benzalkonium bromide, benzalkonium saccharinate), benzoic acid and its salts and esters, propionic acid and its salts, salicylic acid and its salt, sorbic acid and its salts, sodium iodiate, inorganic sulfites and bisulfites such as sodium sulfite, dehydroacetic acid , formic acid, mercurate(1-ethyl)2-mercaptobenzoate(2-)-O,S-,hydrogene (Thiomersal or Thiomerosal), 10-undecylenic acid and its salts, octopirox (piroctone olamine), sodium hydroxy methyl-aminoacetate (sodium hydroxymethylglycinate), 3-iodo-2-propynyl butylcarbamate, 10-undecylenic acid, sulfur. Combinations with natural antimicrobials or chemically modified natural substances with antimicrobial activities such as chitosans and chitosan derivatives, farnesol, plant extracts such as clove oil, blue cypres oil etc. can be also used.

For use on human hair, the dyeing compositions can usually be incorporated into an aqueous cosmetic carrier. Suitable aqueous cosmetic carriers include, for example, creams, sprays, emulsions, gels, powders and also surfactant-containing foaming solutions, e.g. shampoos or other preparations, that are suitable for use on keratin-containing fibers. Such forms of use are described in detail in Research Disclosure 42448 (August 1999). If necessary, it is also possible to incorporate the dyeing compositions into anhydrous carriers, as described, for example, in US-3 369 970, especially column 1, line 70 to column 3, line 55. The dyeing compositions according to the invention are also excellently suitable for the colouring method described in DE-A-3 829 870 using a colouring comb or a colouring brush.

Further carriers for dying compositions are for example described in "Dermatology", edited by Ch. Culnan, H. Maibach, Verlag Marcel Dekker Inc., New York, Basle, 1986, Vol. 7, Ch. Zviak, The Science of Hair Care, chapter 7, pages 248-250, especially on page 243, line 1 to page 244, line 12.

Suitable formulations of cationic dyes, which can be used in the colouring compositions according to the invention, are described for example in
in WO 95/01772, especially on page 11, line 29 to page 12, line 7, or
in WO 01/66646, especially on page 7, line 1 to page 22, and preferred from page 16, line 20 to page 22, or
direct dyes as described in DE-A-19 713 698 , especially page 3, line 51 to page 4, line 29 and page 4, line 65 to page 5, line 60, or
direct dyes and oxidizing agent as described in WO 97/20545, especially on page 9, line 1 to page 11, line 4, in particular on page 11, line 6 to page 13, line 19.

Preferred formulations of cationic dyes with other dyes, which can be used in the colouring compositions according to the invention, are:
combinations of Pyrazolo-[1,5-a]-pyrimidines with at least one cationic dye as described in EP 998,908, especially on page 47, line 3 to page 49, line 26, and preferred on page 51, line 4 to page 52, line 5, or
combinations of cationic dyes as described in FR-2788432, especially on page 53, line 1 to page 63, line 23, especially a combination of cationic dyes with Arianors in FR-2788432, especially on pages 51 to 52, or especially a combination with at least one Basic Brown 17, Basic brown 16, Basic Red 76 and Basic Red 118, and/or at least one Basic Yellow 57, and/or at least one Basic Blue 99, or
combinations of direct dyes and/or an oxidation dye and oxidizing agents in the form of permanent-wave fixing solution, especially with direct dyes as described in DE-A-19 713 698 especially page 4, line 65 to page 35, line 59, or
combinations of cationic dyes and an oxidation dye of the developer compound type and oxidizing agents as described in EP 850 638, especially on page 2, lines 3 to 12 and line 30 to page 14, and page 28, line 35 to page 30, line 20, preferred on page 30, line 25 to page 32, line 30, or
ready-to-use dyeing compositions and multicompartment device for dyeing keratin fibers comprising combinations of an extemporaneous mixture of a composition (A) containing one or more oxidation dye precursors and optionally one or more couplers, and of a composition (B), in powder form, containing one or more direct dye, preferably cationic, optionally dispersed in an organic pulverulent excipient and/or a mineral pulverulent excipient, and a composition (C) containing one or more oxidizing agent as described in US 6,190,421, especially in column 2, line 20 to line 31 in column 7, line 15 to column 8, line 43, and
preferably in column 8, line 55 to column 9, line 56, and preferably with direct dyes as described in column 5, line 30 to column 7, line 14, or
a ready-to-use composition comprising, at least one oxidation base, at least one cationic direct dye and at least one enzyme of 2-electron oxidoreductase type in the presence of at least one donor for the said enzyme as described in US 6,228,129, especially in column 2, line 16 to column 25, line 55, and a multi-compartment dyeing device as described in column 26, lines 13 to 24, especially in column 26, line 26 to column 27, line 9, or
a ready-to-use composition comprising compositions of at least one direct cationic dye and at least one nitrated benzene dye as described in WO 99/20235 especially on page 1, line 25 to page 8, line 5, and on page 30, line 17 to page 34 line 25, with cationic direct dyes as described on page 8, line 12 to page 25 line 6, and a multi-compartment dyeing device as described on page 35, lines 21 to 27, especially on page 36, line 1 to page 37, or
a ready-to-use composition or a multi-compartment dyeing device comprising compositions of at least one direct cationic dye and at least one autooxidisable oxidation dye, especially benzene, indol and indoline derivatives as described in WO 99/20234, especially on page 26, line 5 to page 32, line 18, or
oxidation dyeing compositions of at least one direct dye and at least one meta-Aminophenol derivative and at least one developer compound and an oxidizing agent as described in EP 850 636, especially on page 18, line 1 to page 22, line 11, or
oxidation dyeing compositions of at least one direct dye and at least one developer compound selected from the group of para-Phenylenediamine derivatives and Bis-Phenylalkylenediamine and, and at least one coupler compound selected from the group of meta-Diphenols and an oxidizing agent, as described in EP-A-850 637, especially on page 19, line 24 to page 22, line 57,
cationic dye and e.g. a pyrazolo-(1,5-a)-pyrimidine derivatives, as described in EP 998 908, especially on page 47, line 25 to page 50, line 29, or
oxidative dye precursors (unsaturated aldehyde and coupler compounds), as described in German Patent Application 197 172 24, especially on page 3, line 36 to page 9 line 64.

Cationic dyes, especially compound of formula (1), (2) or (3) may be present in the colouring compositions according to the invention preferably in an amount of from 0.001 % to 5 %, especially from 0.01 % to 1 %, based on the total dyeing composition.

The pH value of the ready-to-use dyeing preparations is usually from 2 to 11, preferably from 5 to 10.

The constituents of the aqueous carrier are used in the colouring compositions to the invention in the amounts customary for that purpose; for example emulsifiers may be used in concentrations of from 0.5 to 30 % by weight and thickeners in concentrations of from 0.1 to 25 % by weight of the total dyeing composition.

If direct dyes, especially of compound of formula (1), (2) or (3) are used together with oxidation dyes and/or the addition salts thereof with an acid, they may be stored separately or together.

It is preferred to store the oxidation dyes and direct dyes, which are not stable to reduction, separately.

They may be stored in a liquid to paste-like preparation (aqueous or non-aqueous) or in the form of a dry powder.

When the dyes and adjuvants are stored together in a liquid preparation, the preparation should be substantially anhydrous in order to reduce reaction of the compounds.

When they are stored separately, the reactive components are intimately mixed with one another only immediately before use. In the case of dry storage, before use a defined amount of hot (from 50 to 80°C) water is usually added and a homogeneous mixture prepared.

One preferred method of applying direct dyes containing formulations on hair is by using a multi-compartment dyeing device or "kit" or any other multi-compartment packaging system, as described for example in WO 97/20545 on page 4, line 19 to line 27.

The colouring compositions according to the invention may combined with a suitable ready-to-use composition for the oxidation dyeing of keratin fibers, in particular human keratin, comprising an oxidizing agent, at least one direct dye, especially compound of formula (1), (2) or (3) and at least one oxidation dye precursor, as described in US 6,190,421, in column 1, line 65 to column 3, line 65, especially in column 10, line 62 to column 12, line 65. Preferably, such a ready-to-use composition is prepared according to a first preferred embodiment by a process which comprises a preliminary step which involves separately storing, on the one hand, a composition (A) comprising, in a medium which is suitable for dyeing, at least one developer compound, especially selected from para-phenylenediamines and bis(phenyl)alkylenediamines, and the acid-addition salts thereof, at least one coupler, especially selected from meta-phenylenediamines and the acid-addition salts thereof, and at least one cationic direct dye, especially compound of formula (I), on the other hand, a composition (B) containing, in a medium which is suitable for dyeing, at least one oxidizing agent and mixing them together at the time of use before applying this mixture to the keratin fibers.

According to a second preferred embodiment for the preparation of the ready-to-use dye composition, the process includes a preliminary step which involves separately storing, on the one hand, a composition (A) comprising, in a medium which is suitable for dyeing, at least one developer compound, especially selected from para-phenylenediamines and bis(phenyl)alkylenediamines, and the acid-addition salts thereof, at least one coupler compound, especially selected from meta-phenylenediamines and the acid-addition salts thereof; on the other hand, a composition (A') comprising, in a medium which is suitable for dyeing, at least one cationic direct dye, especially compound of formula (I), and, lastly, a composition (B) containing, in a medium which is suitable for dyeing, at least one oxidizing agent as defined above, and mixing them together at the time of use before applying this mixture to the keratin fibers.

The composition (A') used according to this second variant of the process in accordance with the invention can optionally be in powder form, the cationic direct dye(s) in accordance with the invention itself (themselves) constituting, in this case, all of the said composition (A') or optionally being dispersed in an organic and/or inorganic pulverulent excipient.

When it is present in the composition A', the organic excipient can be of synthetic or plant origin and is selected in particular from crosslinked and non-crosslinked synthetic polymers, polysaccharides such as celluloses and modified or unmodified starches, as well as natural products containing them such as sawdust and plant gums (guar gum, carob gum, xanthan gum, etc.).

When it is present in the composition (A'), the inorganic excipient can contain metal oxides such as titanium oxides, aluminium oxides, kaolin, talc, silicates, mica and silicas.

An very suitable excipient in the colouring compositions according to the invention is sawdust.
The powdered composition (A') can also contain binders or coating products in an amount, which preferably does not exceed approximately 3% by weight relative to the total weight of the said composition (A').

These binders are preferably selected from oils and liquid fatty substances of inorganic, synthetic, animal or plant origin.

The composition (A') may optionally also contain other adjuvants, in powdered form, in particular surfactants of any kind, hair conditioners such as, for example, cationic polymers, etc.

Another subject of the invention is a multi-compartment dyeing device or "kit" or any other multi-compartment packaging system, as described for example in US 6,228,129, especially in column 26, lines 13 to 24, especially in column 26, line 26 to column 27, line 9, or. A first compartment which contains the composition (A) as defined above, an optional second compartment contains the composition (A') as defined above, when it is present, and a third compartment contains the oxidizing composition (B) as defined above. These devices can be equipped with means which allow the desired mixture to be applied to the hair, such as the devices described in French patent FR-2,586,913, the disclosure of which is specifically incorporated by reference herein.

An oxidizing agent, which may be added to the colouring compositions according to the invention containing composition, comprises an oxidizing agent and a base.

Further, this composition comprises for this oxidizing agent containing composition customary adjuvant and additives.

The formulations are for example a solution, especially a thickened watery or watery alcoholic solution, a cream, foam, a gel, a powder or an emulsion.
In general, preference is given to a cream formulation, a gel formulation or a foam formulation, and especially a foam formulation.

But, if stability- or solubility- problems arise it may of advantage to use powder formulation as for example described in DE 197 13 698, page 2, line 26 to 54 and page 3, line 51 to page 4, line 25, and page 4, line 41 to page 5 line 59.
The oxidizing agent (calculated as hydrogen peroxide) is present in this composition in 0.5 to 12% by weight, in particular from 1 to 6% by weight based on the totals weight of the oxidizing agent containing composition.
The pH-value of the oxidizing agent containing composition is usually about 2 to 7, and in particular about 3 to 6.

An oxidizing agent free composition, which may be added to the colouring compositions according to the invention, comprises a developer compound and a coupler compound and a reduction agent, or
a developer compound or/and optionally a reduction agent, or
a coupler compound and a reduction agent.

Further, an oxidizing agent free composition may additionally comprise a direct dye as for example described in German Patent Application 199 59 479, column 3, line 12 to line 16.

Additionally, the oxidizing agent free composition usually comprises customary adjuvant and additives. Preferred are those, which are described in German Patent Application, in column 3, line 17 to line 41.

The pH-value of the oxidizing agent free composition is usually about 3 to 11, and in particular about 5 to 10, and most particular about 9 to 10.

For adjusting the pH-value organic or inorganic acids, as for example described in German Patent Application 199 59 479, column 3, line 46 to line 53 are suitable.

The colouring compositions according to the invention may also be combined with hair dye compositions comprising an acid dye. Hair dye compositions comprising an acid dye are known. For example, they are described in "Dermatology", edited by Ch. Culnan, H. Maibach, Verlag Marcel Dekker Inc., New York, Basle, 1986, Vol. 7, Ch. Zviak, The Science of Hair Care, chapter 7, pages 248-250, especially on page 253 and 254.
The hair dye compositions comprising an acid dye have a pH of 2-6, preferably 2-5, more preferably 2.5-4.0. If the pH is too low, the resulting composition may roughen the hair, scalp and hand skin due to an acid component in some cases. If the pH is too high, the penetration accelerating effect on the acid dye is lowered.

A compound of formula (1), (2) or (3) according to the present invention may also readily be used in combination with other dyes and/or adjuvants used in the colouring of hair, for example
- acid dye and an alkylene carbonate, as described in US patent 6,248,314, especially in examples 1 and 2, or
- acid hair dye compositions comprise various kinds of organic solvents represented by benzyl alcohol as a penetrant solvent have good penetrability into hair, as described in Japanese Patent Application Laid-Open Nos. 210023/1986 and 101841/1995, or
- acid hair dye compositions with a water-soluble polymer or the like to prevent the drooping of the hair dye composition, as described for example in Japanese Patent Application Laid-Open Nos. 87450/1998, 255540/1997 and 245348/1996, or
- acid hair dye compositions with a water-soluble polymer of aromatic alcohols, lower alkylene carbonates, or the like as described in Japanese Patent Application Laid-Open No. 53970/1998 and Japanese Patent Invention No. 23911/1973.

Preferred keratin fibers are human hair.

The dyes or dye precursors are suitable for all-over colouring of the hair, that is to say when colouring the hair on a first occasion, and also for re-colouring subsequently, or colouration of locks or parts of the hair.

The dyes or dye precursors are applied to hair for example through massage in by hand, a comb, a brush, or a bottle, or a bottle, which is combined with a comb or a nozzle.

In general, the dyes or dye precursors are applied to the hair in a formulation with further components, like adjuvants or additional dyes or dye precursors.

After the application of the dyeing composition the dyed hair is customary rinsed.
Customary, the rinsing is conducted with water.

In a suitable embodiment of the processes of the present invention for dyeing human hair, the dyeing composition is not rinsed off, but washed off with a commercially available hair shampoo.
In general, the dyed hair is dried after rinsing and/or washing.

Customary, drying is conducted with hot air by means of a drier or the like, since color migration to clothes and the like becomes scarcely caused.

A very suitable process for dyeing keratin fibers comprises contacting the keratin fibers under alkaline conditions with at least one capped diazotized compound and a coupler compound, with the proviso that the pH is adjusted in the range from 2 to 6 in the last process step.
Adjusting the pH is achieved in conventional manner by adding an acid as described for example in EP 962218, especially on page 3, lines 12 to 16.

Acids are for example tartaric acid or citric acid, a citric acid gel, a suitable buffer solution with optionally an acid dye.

Preferred technical forms of acids are a solution, a gel, a cream, foam, a conditioner, a emulsion, a shampoo and more preferred a shampoo or a conditioner.

In the context of the present invention, the expression "alkaline condition", denotes to all process steps without those wherein acid conditions are explicitly described.

In the processes for colouring according to the invention, whether or not colouring is to be carried out in the presence of a further dye will depend upon the colour shade to be obtained.

In the context of the present invention, the expression "a further dye", denotes preferably an oxidation dye, a diazotised compound, a capped diazotised compound and/or coupler compound, or acid dye, especially selected a cationic, anionic or uncharged direct dye, especially a cationic dye selected from the group of the cationic dyes as described in WO 95/01772, especially on page 2, line 7 to page 4, line 1, and preferred on page 4, line 35 to page 8, line 21 with the given preferences, and as described in WO 01/66646, especially on page 1, line 18 to page 3, line 16, or a mixture of at least two cationic dyes as described in WO 95/01772, especially on page 8, line 34 to page 10, line 22.

The processes of the present invention for dyeing keratin fibers, in particular human hair, comprise after contacting the keratin fiber with at least a compound of formula (1), (2) or (3), and then
leaving the fibers to stand, and then rinsing the fibers.
The process for dyeing is for example described in WO 01/66646 on page 15, line 32 to page 16, line 2.

Usually, the dyeing compositions are usually applied to the hair in an amount of from 50 to 100 g.

This composition is left on the fiber at 15 to 45°C for 5 to 30 minutes, and in particular for 10 to 20 minutes at 20 to 30°C.

Further preferred does a process for dyeing keratin fibres comprise contacting the keratin fibers with at least one direct dye, a base and an oxidizing agent.

Compositions comprising at least one direct dye, especially compound of formula (1), (2) or (3) and an oxidizing agent, are for example described in WO 97/20545, on page 3, line 24 to page 11, line 4, and especially on page 4, line 9 to 17.

The composition comprising at least one direct dye, especially at least a compound of formula (1), (2) or (3), a base and an oxidizing agent is prepared by mixing at least one direct dye and a base, and then just before the dyeing of the hair, adding an oxidizing agent.

Alternatively, the oxidizing agent can be applied simultaneously with a composition comprising at least one dye, such as a compound of formula (1), (2) or (3) and a base.

Preferably, the process for dyeing keratin fibres with at least on direct dye comprises using a multi-compartment dyeing device or 'kits' as described for example in WO 97/20545, especially on page 4, line 19 to line 27.

Suitable processes for enlightening dyeing, wherein a compound of formula (1), (2) or (3) according to the invention can be used in combination with an oxidative agent are described in WO 97/20545, on page 11 to page 13.

Further preferred are processes for dyeing keratine fibers, especially compound of formula (1), (2) or (3) with further cationic dyes, according to processes as described
in WO 95/01772, especially on page 10, line 24 to page 11, line 16, and especially on page 11, line 29 to page 28, or
in WO 01/66646, especially on page 1, line 18 to page 3, line 16, and preferred from page 16, line 20 to page 22, or
in EP 970 685, especially on page 50, lines 15 to 43, and preferred from page 50, line 46 to page 51, line 40, or
in DE-A-19 713 698 , especially page 5, lines 26 to 60, or
a process of dyeing with direct dyes and oxidizing agent is described in WO 97/20545, especially on page 10, line 10 to page 11, line 55 and preferably on page 11, line 6 to page 13, line 19.

Further preferred are processes for dyeing keratine fibers, especially compound of formula (1), (2) or (3) with other dyes, which can be combined with a compound of formula (1), (2) or (3) according to the present invention, are:
mixtures of at least two cationic dyes as described in WO 95/01772, especially on page 11, lines 1 to 15, or
combinations of Pyrazolo-[1,5-a]-pyrimidines with at least one cationic dye as described in EP 998,908, especially on page 50, lines 15 to 28, or
combinations of cationic dyes as described in FR-2788432, especially on page 49, line 28 to page 52, and preferred on page 50, lines 16 to 28, or
combinations of direct dyes and/or an oxidation dye and oxidizing agents in the form of permanent-wave fixing solution, especially with direct dyes as described in DE-A-19 713 698, especially on page 2, lines 12 to 23, especially on page 4, line 65 to page 5, line 59, or combinations of cationic dyes and an oxidation dye of the developer compound type and oxidizing agents as described in EP 850 638, especially on page 29, line 42 to page 30, line 20 and preferred on page 30, line 25 to page 32, line 30, or
combinations of an extemporaneous mixture of a composition (A) containing one or more oxidation dye precursors and optionally one or more couplers, and of a composition (B), in powder form, containing one or more direct dye, preferably cationic, optionally dispersed in an organic pulverulent excipient and/or a mineral pulverulent excipient, and a composition (C) containing one or more oxidizing agent as described in US 6,190,421, especially in column 8, lines 43 to 52, and preferably in column 8, line 55 to column 9, line 55, or
a ready-to-use composition comprising, at least one oxidation base, at least one cationic direct dye and at least one enzyme of 2-electron oxidoreductase type in the presence of at least one donor for the said enzyme as described in US 6,228,129, especially in column 25, line 56 to column 27, line 9, or
a ready-to-use composition or multi-compartment dyeing device comprising compositions of at least one direct cationic dye and at least one nitrated benzene dye as described in WO 99/20235 on page 34, line 27 to page 37, or
a ready-to-use composition or multi-compartment dyeing device comprising compositions of at least one direct cationic dye and at least one autooxidisable oxidation dye, especially benzene, indol and indoline derivatives as described in WO 99/20234, especially on page 32, line 20 to page 35, oxidation dyeing compositions of at least one direct dye and at least one meta-Aminophenol derivative and at least one developer compound and an oxidizing agent as described in EP 850 636, especially on page 18, line 1 to page 22, line 11, or oxidation dyeing compositions of at least one direct dye and at least one developer compound selected from the group of para-Phenylenediamine derivatives and Bis-Phenylalkylenediamine and, and at least one coupler compound selected from the group of meta-Diphenols and an oxidizing agent, as described in EP-A-850 637, especially on page 19, line 24 to page 22, line 57,
cationic dye and e.g. a pyrazolo-(1,5-a)-pyrimidine derivatives, as described in EP 998 908, especially on page 47, line 25 to page 50, line 29, or
arianors and/or oxidative dyes, as described in FR-2 788 432, especially on page 2, line 16 to page 3, line 16, and page 5, line 19 to page 14, line 8, and combinations with cationic dyes as described on page 14, line 23 and following, or
oxidative dye precursors (unsaturated aldehyde and coupler compounds), as described in German Patent Application 197 172 24, especially on page 3, line 36 to page 9 line 64.

The processes of colouring of keratin fibers, especially human hair, with a compound of formula (1), (2) or (3) according to the present invention may be combined with other direct dyes and oxidative dyes.

In a preferred embodiment of the present invention the process for dyeing keratin fibers with direct dyes and oxidative dyes, in particular human hair, comprises
a) contacting the keratin fibers with an oxidizing agent, optionally containing at least a compound of formula (1), (2) or (3),
b) then contacting the keratin fibers with an oxidizing agent free composition, optionally containing at least a compound of formula (1), (2) or (3),
   or

a) contacting the keratin fibers with an oxidizing agent free composition, optionally containing at least a compound of formula (1), (2) or (3),
b) then contacting the keratin fibers with an oxidizing agent, optionally containing least a compound of formula (1), (2) or (3), with the proviso that at least in one of the process steps a) or b) a compound of formula (1), (2) or (3) is present.

The process of colouring with a compound of formula (1), (2) or (3) according to the present invention may combined with a process for dyeing keratin fibers with direct dyes and oxidative dyes, which comprises contacting the keratin fibers with least a compound of formula (1), (2) or (3), then contacting the keratin fibers with an oxidizing agent free composition.

Such process is for example described in DE 199 41 450, especially on page 5, lines 50 to 58, and on page 8, line 31 to 46.

Oxidizing agent is usually applied in form of an oxidizing agent containing composition. Oxidizing agent free composition containing at least one coupler compound, at least one developer compound, a base and a reduction agent.

Customary, the oxidizing agent containing composition is evenly applied in a sufficient amount related to the amount of hair, usually with 30 to 200 g.

In general, the oxidizing agent containing composition is left on the fiber at 15 to 45°C for 0 to 15 minutes, and in particular for 0 to 5 minutes.

Then the oxidizing agent free composition is applied to the hair.

In general, the direct dye and oxidizing agent free composition is left on the fiber at 15 to 50°C for 5 to 45 minutes, and in particular for 10 to 25 minutes.
The coupler and developer compounds of the oxidizing agent free composition can be applied simultaneously or in succession. Preferred is a simultaneous application.

One preferred embodiment of the process is to wash the hair with shampoo and or a weak acid, such as citric acid or tartrate acid.

The direct dyes, which are stable to reduction can stored together with the oxidizing agent free compositions and are applicable as composition.

It is of advantage to prepare compositions of direct dyes, which are not stable to reduction, with oxidizing agent free compositions just before the dyeing process.

Further, a direct dye and an oxidizing agent free composition can be applied simultaneously or in succession.

A further process for the coloration of keratin fiber with direct dyes and oxidation dyes, which can be used in combination with a compound of formula (1), (2) or (3) according to the invention, comprises mixing at least a compound of formula (1), (2) or (3) and optionally at least one coupler compound and at least one developer compound, and an oxidizing agent, which optionally contains at least one further direct dye, and
then contacting the keratin fibers with the mixture as prepared in step a).

A further suitable process for the coloration of keratin fiber with direct dyes and oxidation dyes, which can be used in combination with a compound of formula (1), (2) or (3) according to the invention, comprises mixing at least one autooxidable compound and at least one developer compound and at least one compound of formula (1), (2) or (3), and
then contacting the keratin fibers with the mixture prepared in step a).

The present invention relates also to a process, in which a dye of formula (1), (2) or (3), or cationic dye of formula (1), (2) or (3) prepared according to a process according to the invention, or a composition according to the invention, and,
in succession in any desired order, or simultaneously,
a capped diazonium compound and a water-soluble coupling component,
are applied to the material to be dyed under conditions in which coupling does not take place initially, and then the capped diazonium compound disposed on the material is caused to react with the coupling component.

The first step of the dyeing method according to the invention comprises applying to the material to be dyed, in succession in any order or simultaneously, a capped diazonium compound and a water-soluble coupling component and, optionally, a cationic direct dye, the application being carried out under such conditions that coupling does not take place initially. This is effected, for example, by immersing the material in a solution comprising the capped diazonium compound or the coupling component and, optionally, a cationic direct dye, and then, if desired after rinsing and intermediate drying, immersing the material in a solution of the second component. Preferably, however, the capped diazonium compound and the coupling component and, optionally, a cationic direct dye, are present together in one solution. Such solutions can also be applied to the material by spraying or by similar measures, care having to be taken that penetration is adequate unless it is desired to dye only the upper layers. In that first step, the diazonium compound and the coupling component should not yet react with each other, and that is preferably achieved by maintaining a pH value of from 8 to 12, preferably from 9 to 11.

In the second step, the diazonium compound and the coupling component are then caused to react, preferably by lowering the pH to a value of from 5 to 2, especially from 3 to 4. The pH value is lowered in customary manner by addition of an acid or a suitable buffer solution, especially citric acid or citric acid gel. If desired, a cationic direct dye may be used in the second step. In any event, it is necessary for a cationic direct dye to be used in one of steps 1 and 2 of the process according to the invention.
The dyed material is then finished in conventional manner, for example by rinsing with water and subsequently drying.

Further, in the present invention especially preferred is a process for dyeing keratin fibers, in particular human hair, with capped diazotised compounds, which comprises, contacting the keratin fibers, under alkaline conditions, with at least one capped diazotised compound and a coupler compound, and optionally an oxidising agent, and optionally in the presence of a further dye, and optionally with at least a compound of formula (1), (2) or (3), then adjusting the pH in the range of 6 to 2 by treatment with acid, optionally in the presence of a further dye, especially with, and optionally at least a compound of formula (1), (2) or (3), with the proviso that at least in one step d) or e) at least a compound of formula (1), (2) or (3) is present.
The capped diazotised compound and coupler compound and optionally the oxidizing agent, can be applied in any desired order successively, or simultaneously.
Preferably, however, the capped diazotised compound and the coupler compound are applied simultaneously, in a single composition.

Customary the dyeing composition is applied to the hair in an amount of from 50 to 100 g.

In the context of the present invention, the expression "alkaline conditions" denotes a pH in the range from 8 to 10, preferably 9-10, especially 9.5-10.

Adding bases, for example sodium carbonate, ammonia or sodium hydroxide, to the hair or to the dye precursors, the capped diazotised compound and/or the water-soluble coupling component, or to colouring compositions comprising the dye precursors, customarily achieve the alkaline conditions.
In the second stage, the diazotised compound and the coupler compound are then caused to react, preferably by lowering the pH by adding an acid to a value of from 6 to 2, especially from 3 to 4.

Acids are for example tartaric acid or citric acid, a citric acid gel, a suitable buffer solution with optionally an acid dye.

Preferred technical forms of acids are a solution, a gel, a cream, foam, a conditioner, a emulsion, a shampoo and more preferred a shampoo or a conditioner.

The ratio of the amount of alkaline colouring composition applied in the first stage to that of acid colouring composition applied in the second stage is preferably about from 1:3 to 3:1, especially about 1:1.

This first alkaline and then acid dyeing compositions each are left on the fiber at 15 to 45°C for 5 to 60 minutes, and in particular for 5 to 45 minutes at 20 to 30°C.

A preferred embodiment of the process for dyeing keratin fibres with capped diazotised compounds and a coupler compound, comprises contacting the keratin fibres with more than one capped diazotised compound and/or more than one coupler compound.

Preferred is a process of the present invention for the coloration of keratin fiber with capped diazotised compounds and at least a compound of formula (1), (2) or (3), comprises mixing, under alkaline conditions, at least one with capped diazotised compound and at least one coupler compound and, optionally with at least a compound of formula (I), and optionally at least one developer compound; and an oxidizing agent, and, optionally with at least a compound of formula (1), (2) or (3), and
then contacting the keratin fibers with the mixture as prepared in step a), and then adjusting the pH in the range of 6 to 2 by treatment with acid, optionally in the presence of a further dye, with the proviso that at least in one of the process a compound of formula (1), (2) or (3) is present.

More preferred is a process for dyeing keratin fibres with at least one capped diazotized compound, which comprises mixing under alkaline conditions, at least one capped diazotized compound and at least a compound of formula (1), (2) or (3), a base and an oxidizing agent, and then contacting the keratin fibers with the mixture as prepared in step a), then adjusting the pH in the range of 6 to 2 by treatment with acid, optionally in the presence of a further dye.

Further, preferred is a process for the coloration of keratin fiber with capped diazotised compounds comprising
mixing under alkaline conditions, at least one with capped diazotised compound and at least one coupler compound and optionally at least a compound of formula (1), (2) or (3), and optionally at least one developer compound, and optionally at least one autooxidable compound, and
then contacting the keratin fibers with the mixture prepared in step a), then adjusting the pH in the range of 6 to 2 by treatment with acid, and and optionally at least a compound of formula (1), (2) or (3), and optionally in the presence of a further dye, with the proviso that at least in one of the process a compound of formula (1), (2) or (3) is present.

A further preferred process for the two-step direct dyeing of keratin fibres, which can be used in combination with a compound of formula (1), (2) or (3) according to the invention, is characterized in that,
contacting the keratin fibers with an oxidizing agent or an oxidizing agent containing composition,
then contacting the keratin fibers with at least one capped diazotised compound and at least a coupler compound and a compound of formula (1), (2) or (3), and optionally an oxidizing agent free composition,
then adjusting the pH in the range of 6 to 2 by treatment with acid, optionally in the presence of a further dye.
or
contacting the keratin fibers with at least one capped diazotised compound and a coupler compound and a compound of formula (1), (2) or (3), and optionally an oxidizing agent free composition,
then contacting the keratin fibers with an oxidizing agent or an oxidizing agent containing composition,
then adjusting the pH in the range of 5 to 2 by treatment with acid, optionally in the presence of a further dye.

The present invention also concerns a process for the colouration of keratin fibers, especially human hair, with acid dyes, which can be used in combination with a compound of formula (1), (2) or (3) according to the invention.

The process comprises
contacting the keratin fiber with an acid dye and at least a compound of formula (1), (2) or (3).

Customary, the dyeing composition comprising an acid dye is applied to the hair in an amount of from 50 to 100 g.
This in a composition is left on the fiber at 15 to 45°C for 1 to 30 minutes, and in particular for 0 to 15 minutes at 20 to 30°C.

Preferably the hair is rinsed and than washed with shampoo and more preferably not rinsed, but washed with shampoo.

The shampoo used herein includes a shampoo comprising 5-20% of a usual anionic surfactant such as an alkylsulfate or polyoxyethylene alkylsulfate.

Further, the present invention relates to a method of dyeing keratin-containing fibres with the cationic reactive dyes, which comprises treating the fibres with the cationic reactive dyes defined at the beginning or with the dye compositions according to the invention.

A preferred embodiment of the method according to the invention for dyeing keratin-containing fibres comprises treating the fibres with a dyeing solution, prepared according to the process of the invention, comprising a tinctorially effective amount of a cationic reactive dye of formula (1), (2) or (3).

The cationic reactive dyes defined at the beginning are present in the dye compositions according to the invention preferably in a tinctorially effective amount of from 0.001 % to 5 %, especially from 0.01 % to 1 %, based on the total dyestuff.

The keratin-containing fibres are usually treated with the dyeing solution for about 30 minutes at 20-25°C.

A further preferred embodiment of the present invention relates to a method of dyeing hair, which comprises treating the hair with
a) an acidic or alkaline permanent-wave solution, and
b) then with a dyeing solution, prepared according to the process of the invention, comprising a cationic reactive dye of formula (1), (2) or (3).

Usually the keratin-containing fibres are treated with the permanent-wave solution for about 3-10 minutes, preferably for 4-6 minutes, at 20-25°C.

It is generally advisable to rinse the hair after treatment with the dyeing solution and/or permanent-wave solution.

The present invention relates also to the use of the cationic reactive dyes defined at the beginning or of the dye compositions according to the invention for dyeing keratin fibers, wool, leather, silk, cellulose or polyamides, especially for dyeing hair.

A preferred embodiment of the process according to the invention for dyeing keratin-containing fibres comprises treating the fibres with a dyeing solution, prepared according to the process of the invention, comprising a tinctorially effective amount of a cationic reactive dye.

The cationic reactive dyes are present in the dye compositions according to the invention preferably in a tinctorially effective amount of from 0.001 % to 5 %, especially from 0.01 % to 1 %, based on the total dyestuff.

The keratin-containing fibres are usually treated with the dyeing solution for about 30 minutes at 20-25°C.

A further preferred embodiment of the present invention relates to a method of dyeing hair, which comprises treating the hair with
a) an acidic or alkaline permanent-wave solution, and
b) then with a dyeing solution, prepared according to the process of the invention, comprising a cationic reactive dye.

Usually the keratin-containing fibres are treated with the permanent-wave solution for about 3-10 minutes, preferably for 4-6 minutes, at 20-25°C.

A preferred embodiment of the present invention relates to a method of dyeing hair, which comprises treating the hair with a mixture of
a) an acidic or alkaline permanent-wave solution comprising a thiol derivative, and
b) a dyeing solution comprising a cationic dichlorotriazine reactive dye or a cationic monofluoromonochloropyrimidine reactive dye.

The dyes according to the invention are distinguished by brilliant shades. They are suitable for dyeing organic material, such as keratin, wool, leather, silk, cellulose or polyamides, especially keratin-containing fibres, cotton or nylon, and preferably human hair. The dyeings obtained are distinguished by their depth of shade and their good fastness to washing properties, such as, for example, fastness to light, shampooing and rubbing. The stability and storage stability of the dyes according to the invention are excellent. They are accordingly especially suitable for dyeing under oxidising and reducing conditions. The advantage of the new dyes according to the present invention is their stability against reduction agents e. g. sodium sulfite and ascorbic acid. Therefore you can combine them with oxidation dyes in one emulsion.

The following Examples serve to illustrate the processes for colouration without limiting the processes thereto. Unless specified otherwise, parts and percentages relate to weight. The amounts of dye specified are relative to the material being coloured.

### Examples G (Application)

Compositions A, B, C, D, E, F and G for colouring human hair according to the following table below.

| Compositions | A | B | C | D1-D20** | E6-E20**** | F* | G4-G13*** |
|---|---|---|---|---|---|---|---|
| cetyl stearyl alcohol | 11.00 | 11.00 | 11.00 | 11.00 | 11.00 | | 11.00 |
| oleth-5 | 5.0 | 5.0 | 5.0 | 5.0 | 5.0 | | 5.0 |
| oleic acid | 2.5 | 2.5 | 2.5 | 2.5 | 2.5 | | 2.5 |
| stearic acid monoethanolamide | 2.5 | 2.5 | 2.5 | 2.5 | 2.5 | | 2.5 |
| coconut fatty acid monoethanolamide | 2.5 | 2.5 | 2.5 | 2.5 | 2.5 | | 2.5 |
| sodium lauryl sulfate | 1.7 | 1.7 | 1.7 | 1.7 | 1.7 | | 1.7 |
| 1,2-propanediol | 1.0 | 1.0 | 1.0 | 1.0 | 1.0 | | 1.0 |
| ammonium chloride | 0.5 | 0.5 | 0.5 | 0.5 | 0.5 | | 0.5 |
| EDTA, tetrasodium salt | 0.2 | 0.2 | 0.2 | 0.2 | 0.2 | | 0.2 |
| perfume | 0.4 | 0.4 | 0.4 | 0.4 | 0.4 | | 0.4 |
| wheat protein hydrolysate | 0.2 | 0.2 | 0.2 | 0.2 | 0.2 | | 0.2 |
| silica | 0.1 | 0.1 | 0.1 | 0.1 | 0.1 | | 0.1 |
| 2,5-diaminotoluene sulfate | | | 0.7 | | 0.7 | | |
| 4-amino-2-hydroxytoluene | | | 0.5 | | 0.5 | | |
| 2,5,6-triamino-4-hydroxypyrimidine sulfate | | | 0.2 | | 0.2 | | |
| sodium sulfite | | | 1.0 | | 1.0 | | |
| ascorbic acid | | | 0.5 | | 0.5 | | |
| triazene of Example 15a | 9.33 | | | | | | |
| coupler of Example 15 | | 12.03 | | | | | |
| Direct dye ** | | | | 0.4 | | | |
| Direct dye *** | | | | | | | 0.4 |
| Direct dye **** | | | | | 0.4 | | |
| Black Color No. 401 | | | | | | 0.1 | |
| Purple Color 401 | | | | | | 0.0 5 | |
| Orange Color No. 205 | | | | | | 0.1 | |
| benzyl alcohol | | | | | | 2.0 | |
| ethylene carbonate | | | | | | 10 | |
| propylene carbonate | | | | | | 15 | |
| ethanol | | | | | | 10 | |
| lactic acid | | | | | | 3.5 | |
| sodium carbonate solution | | | | | | of pH 2.9 | |
| hydroxyethyl cellulose | | | | | | 1.5 | |
| ammonia (25 %) | 9.2 | 9.2 | 9.2 | 9.2 | 9.2 | | 9.2 |
| composition: pH | 9.8 | 9.8 | 9.8 | 9.8 | 9.8 | | 9.8 |
| water | ad 100 | ad 100 | ad 100 | ad 100 | ad 100 | ad 100 | ad 100 |

| | | | | | | | |
|---|---|---|---|---|---|---|---|
| *F:dye mixture known from US 6 248 314 **D1-D10 comprise the following dyes: in D1 the direct dye** is Basic Yellow 87; in D2 the direct dye** is Basic Orange 31; in D3 the direct dye** is or Basic Red 51; in D4 the direct dye** is the cationic dye of example 4 as described in WO 01/66646; in D5 the direct dye** is the cationic dye of example 6, compound of formula 106, as described WO 02/31056; in D6 the direct dye** has the following formula (I) 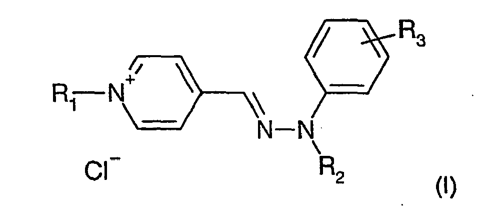 wherein R₁ is methyl, R₂ is benzyl, R₃ is hydrogen; in D7 the direct dye** is a compound of formula (I) wherein R₁ is benzyl, R₂ is benzyl, R₃ is hydrogen; in D8 the direct dye** is a compound of formula (I) wherein R₁ is benzyl, R₂ is methyl, R₃ is hydrogen. In D10 the direct dye** is a cationic dye of formula (3) as described in EP-A-714,954. **D11-D20 and ***G4-G13 comprise the following direct dyes: in D11 the direct dye** and in G4 direct dye*** is a compound of formula (4), wherein X⁻ is acetate; in D12 the direct dye** and in G5 direct dye*** is a compound of formula (5), wherein X⁻ is formate; in D13 the direct dye** and in G6 direct dye*** is a compound of formula (6), wherein X⁻ is acetate; in D14 the direct dye** and in G7 direct dye*** is a compound of formula (7), wherein X⁻ is acetate; in D15 the direct dye** and in G8 direct dye*** is a compound of formula (8), wherein X⁻ is acetate; in D16 the direct dye** and in G9 direct dye*** is a compound of formula (9), wherein X⁻ is acetate; in D17 the direct dye** and in G10 direct dye*** is a compound of formula (10), wherein X⁻ is succinate; in D18 the direct dye** and in G11 direct dye*** is a compound of formula (11), wherein X⁻ is lactate; in D19 the direct dye** and in G12 direct dye*** is a compound of formula (12), wherein X⁻ is acrylate; in D20 the direct dye** and in G13 direct dye*** is a compound of formula (13), wherein X⁻ is acetate; ****E6-E20: In E6 the direct dye**** is identical the direct dye ** of D6; in E7 the direct dye**** is identical the direct dye ** of D7; in E8 the direct dye**** is identical the direct dye** of D8; E9 comprises as direct dye Basic Yellow 87. in E10 the direct dye**** is identical the direct dye ** of D10; in E11 the direct dye**** is identical the direct dye ** of D11; in E12 the direct dye**** is identical the direct dye ** of D12; in E13 the direct dye**** is identical the direct dye ** of D13; in E14 the direct dye**** is identical the direct dye ** of D14; in E15 the direct dye**** is identical the direct dye ** of D15; in E16 the direct dye**** is identical the direct dye ** of D16; in E17 the direct dye**** is identical the direct dye ** of D17; in E18 the direct dye**** is identical the direct dye ** of D18; in E19 the direct dye**** is identical the direct dye ** of D19. in E20 the direct dye**** is identical the direct dye ** of D20. | | | | | | | |

### Example G/3:

A strand of middle blond undamaged human hair is coloured
3a) with a mixture of 20 g of 6 % hydrogen peroxide solution and a composition consisting of 5 g each of compositions A, B, C and G5, and alternatively
3b) first with 20 g of 6 % hydrogen peroxide solution and then with 5 g of a composition A, 5 g of composition B, 5 g of composition C and 5 g of composition G6, or
3c) first with 5 g of a composition A, 5 g of composition B, 5 g of composition C and 5 g of composition G13, and then with 20 g of 6 % hydrogen peroxide solution.

The colouring mixture is allowed to act on the hair for 30 minutes at about 22°C. 10 g of a 2 % strength aqueous citric acid gel are then applied to the strand. After contact for 5 minutes, the strand is rinsed thoroughly, shampooed and then dried.
A strong, intense, striking red coloration having good fastness to washing and fastness to rubbing properties is obtained.

### Example G/4:

A strand of blond undamaged human hair is coloured
4a) with a composition consisting of 5 g each of compositions A, B and G10, and
4b) alternatively the colouring mixture is allowed to act on the hair for 30 minutes at about 22°C. 10 g of a 2 % strength aqueous citric acid gel are then applied to the strand. After contact for 5 minutes, the strand is rinsed thoroughly, shampooed and then dried.

A strong, intense, striking red coloration having good fastness to washing and fastness to rubbing properties is obtained.

Example (4a/G9) is identical to Example 4a with the proviso that G10 is replaced by G9.
Example (4a/G13) is identical to Example 4a with the proviso that G10 is replaced by G13.

### Example G/5:

A strand of middle blond undamaged human hair is coloured
5a) with a mixture of 15 g of 6 % hydrogen peroxide solution and a composition consisting of 5 g each of compositions A, B and G6, and alternatively
5b) first with 15 g of 6 % hydrogen peroxide solution, and then with 5 g of a composition A, 5 g of composition, and B and 5 g of composition G6.
5c) first with 5 g of a composition A, 5 g of composition, and B and 5 g of composition G6, and then with 15 g of 6 % hydrogen peroxide solution.
The colouring mixture is allowed to act on the hair for 30 minutes at about 22°C. 10 g of a 2 % strength aqueous citric acid gel are then applied to the strand. After contact for 5 minutes, the strand is rinsed thoroughly, shampooed and then dried.
A strong, intense, striking red coloration having good fastness to washing and fastness to rubbing properties is obtained.

Example (5a/G5) is identical to Example 5a with the proviso that G6 is replaced by G5.

Example (5a/G10) is identical to Example 5a with the proviso that G6 is replaced by G10.

Example (5b/G9) is identical to Example 5b with the proviso that G6 is replaced by G9.

Example (5b/G13) is identical to Example 5b with the proviso that G6 is replaced by G13.

Example (5c/G5) is identical to Example 5c with the proviso that G6 is replaced by G5.

Example (5c/G13) is identical to Example 5c with the proviso that G6 is replaced by G13.

Example G/6:

A strand of middle blond undamaged human hair is coloured
6a) with a mixture of 15 g of 6 % hydrogen peroxide solution and a composition consisting of 5 g each of compositions A, B and G6, and alternatively
6b) first with 15 g of 6 % hydrogen peroxide solution, and then with 5 g of a composition A, 5 g of composition B and 5 g of composition G6, or
6c) first with 5 g of a composition A, 5 g of composition B and 5 g of composition G6, and then with 15 g of 6 % hydrogen peroxide solution.
The colouring mixture is allowed to act on the hair for 30 minutes at about 22°C. 10 g of a 2 % strength aqueous citric acid gel are then applied to the strand. After contact for 5 minutes, the strand is rinsed thoroughly, shampooed and then dried.
A strong, intense, striking red coloration having good fastness to washing and fastness to rubbing properties is obtained.

Example (6a/G5) is identical to Example 6a with the proviso that G6 is replaced by G5.

Example (6a/G13) is identical to Example 6a with the proviso that G6 is replaced by G13.

Example (6b/G9) is identical to Example 6b with the proviso that G6 is replaced by G9.

Example 6b/G10) is identical to Example 6b with the proviso that G6 is replaced by G10.

Example (6c/G5) is identical to Example 6c with the proviso that G6 is replaced by G5.

Example (6c/G13) is identical to Example 6c with the proviso that G6 is replaced by G13.

### Example G/9:

A strand of blond undamaged human hair is coloured
9a) with a mixture of 10 g of 6 % hydrogen peroxide solution and a composition consisting of 5 g each of compositions C and G6, and alternatively
9b) with 10 g of 6 % hydrogen peroxide solution and 5 g of composition C and 5 g of composition G6.

The colouring mixture is allowed to act on the hair for 30 minutes at about 22°C. 10 g of a 2 % strength aqueous citric acid gel are then applied to the strand. After contact for 5 minutes, the strand is rinsed thoroughly, shampooed and then dried.
A strong, intense, striking bluish red coloration having good fastness to washing and fastness to rubbing properties is obtained.

Example (9a/G5) is identical to Example 9a with the proviso that G6 is replaced by G5.

Example (9a/G13) is identical to Example 9a with the proviso that G6 is replaced by G3.

Example (9b/G9) is identical to Example 9b with the proviso that G6 is replaced by G9.

Example (9b/G10) is identical to Example 9b with the proviso that G6 is replaced by G10.

### Example G/10:

A strand of blond undamaged human hair is coloured
10a) with a mixture of 10 g of 6 % hydrogen peroxide solution and a composition consisting of 5 g each of compositions D1 and E11, and alternatively
10b) first with 10 g of 6 % hydrogen peroxide solution, and then with 5 g of composition D1 and 5 g of composition E11,or
10c) first with 5 g of composition D1 and 5 g of composition E11, and then with 10 g of 6 % hydrogen peroxide solution.

The colouring mixture is allowed to act on the hair for 30 minutes at about 22°C. 10 g of a 2 % strength aqueous citric acid gel are then applied to the strand. After contact for 5 minutes, the strand is rinsed thoroughly, shampooed and then dried.
A strong, intense, striking bluish red coloration having good fastness to washing and fastness to rubbing properties is obtained.

Example (10a/D2) is identical to Example 10a with the proviso that D1 is replaced by D2.

Example (10a/D3) is identical to Example 10a with the proviso that D1 is replaced by D3.

Example (10a/D4) is identical to Example 10a with the proviso that D1 is replaced by D4.

Example (10a/D5) is identical to Example 10a with the proviso that D1 is replaced by D5.

Example (10b/D2) is identical to Example 10b with the proviso that D1 is replaced by D2.

Example (10b/D3) is identical to Example 10b with the proviso that D1 is replaced by D3.

Example (10b/D4) is identical to Example 10b with the proviso that D1 is replaced by D4.

Example (10b/D5) is identical to Example 10b with the proviso that D1 is replaced by D5.

Example (10c/D2) is identical to Example 10c with the proviso that D1 is replaced by D2.

Example (10c/D3) is identical to Example 10c with the proviso that D1 is replaced by D3.

Example (10c/D4) is identical to Example 10c with the proviso that D1 is replaced by D4.

Example (10c/D5) is identical to Example 10c with the proviso that D1 is replaced by D5.

Example (10d/E12) is identical to Example 10a with the proviso that E11 is replaced by E12.

Example (10d/E19) is identical to Example 10a with the proviso that E11 is replaced by E10.

Example (10d/E16) is identical to Example 10a with the proviso that E11 is replaced by E7.

Example (10e/E15) is identical to Example 10b with the proviso that E11 is replaced by E12.

Example 10e/E12) is identical to Example 10b with the proviso that E11 is replaced by E10.

Example 10e/E16) is identical to Example 10b with the proviso that E11 is replaced by E7.

Example (10f/E12) is identical to Example 10c with the proviso that E11 is replaced by E12.

Example (10f/E19) is identical to Example 10c with the proviso that E11 is replaced by E10.

Example (10f/E16) is identical to Example 10c with the proviso that E11 is replaced by E7.

### Example G/11:

A strand of brown undamaged human hair is coloured
11a) with a mixture of 10 g of 6 % hydrogen peroxide solution and a composition consisting of 5 g each of compositions C and E11, and alternatively
11b) first with 10 g of 6 % hydrogen peroxide solution, and then with 5 g of composition C and 5 g of composition E11, or
11c) first with 5 g of composition C and 5 g of composition E11, and then with 10 g of 6 % hydrogen peroxide solution.

The colouring mixture is allowed to act on the hair for 30 minutes at about 22°C. 10 g of a 2 % strength aqueous citric acid gel are then applied to the strand. After contact for 5 minutes, the strand is rinsed thoroughly, shampooed and then dried.
A strong, intense, striking bluish red coloration having good fastness to washing and fastness to rubbing properties is obtained.

Example (11a/E12) is identical to Example 11 a with the proviso that E11 is replaced by E12.

Example (11a/E19) is identical to Example 11 a with the proviso that E11 is replaced by E10.

Example (11a/E16) is identical to Example 11 a with the proviso that E11 is replaced by E7.

Example (11b/E15) is identical to Example 11 b with the proviso that E11 is replaced by E10.

Example (11b/E19) is identical to Example 11 b with the proviso that E11 is replaced by E12.

Example (11b/E12) is identical to Example 11 b with the proviso that E11 is replaced by E7.

Example (11c/E12) is identical to Example 11c with the proviso that E11 is replaced by E12.

Example (11c/E19) is identical to Example 11 c with the proviso that E11 is replaced by E10.

Example (11c/E15) is identical to Example 11 c with the proviso that E11 is replaced by E7.

### Example G/12:

A strand of blond undamaged human hair is coloured
12a) with a mixture of 5 g of 6 % hydrogen peroxide solution and 5 g of composition G6, and alternatively
12b) first with 5 g of 6 % hydrogen peroxide solution, and then with 5 g of a composition G6, or
12c) first with 5 g of a composition G6, and then with 5 g of 6 % hydrogen peroxide solution.

The colouring mixture is allowed to act on the hair for 30 minutes at about 22°C.

After contact for 30 minutes, without being washed out, 10 g of a dye mixture F is applied to the hair. The hair is then combed through thoroughly, whereupon its pH becomes about 3. Then, after a contact period of 15 minutes, the hair is rinsed thoroughly with water and dried.

Example (12a/G5) is identical to Example 12a with the proviso that G6 is replaced by G5.

Example (12a/G13) is identical to Example 12a with the proviso that G6 is replaced by G13.

### Example G/13:

A strand of blond undamaged human hair is coloured with 10 g of composition G6,
The colouring mixture is allowed to act on the hair for 30 minutes at about 22°C.
After contact for 30 minutes, without being washed out, 10 g of a dye mixture F
is applied to the hair. The hair is then combed through thoroughly, whereupon its pH becomes about 3. Then, after a contact period of 15 minutes, the hair is rinsed thoroughly with water and dried.

Example (13/G5) is identical to Example G/13 with the proviso that G6 is replaced by G5.

Example (13/G9) is identical to Example G/13 with the proviso that G6 is replaced by G9.

### Example G/15

A strand of blond undamaged human hair is coloured with 10 g of a composition G6.
The colouring mixture is allowed to act on the hair for 30 minutes at about 22°C. After contact the strand is rinsed thoroughly, shampooed and then dried.
A strong, intense, striking red coloration having good fastness to washing and fastness to rubbing properties is obtained.

Example (15/G5) is identical to Example 15 with the proviso that G6 is replaced by G5.

Example (15/G9) is identical to Example 15 with the proviso that G6 is replaced by G9.

### Example G/16:

A strand of blond undamaged human hair is coloured
16a) with a mixture of 5 g of 6 % hydrogen peroxide solution and 5 g of composition G6, and alternatively
16b) first with 5 g of 6 % hydrogen peroxide solution, and then with 5 g of a composition G6, or
16c) first with 5 g of a composition G6, and then with 5 g of 6 % hydrogen peroxide solution.

The colouring mixture is allowed to act on the hair for 30 minutes at about 22°C. 10 g of a 2 % strength aqueous citric acid gel are then applied to the strand. After contact for 15 minutes, the strand is rinsed thoroughly, shampooed and then dried.
A strong, intense, striking red coloration having good fastness to washing and fastness to rubbing properties is obtained.

Example (16a/G5) is identical to Example 16a with the proviso that G6 is replaced by G5.

Example (16a/G9) is identical to Example 16a with the proviso that G6 is replaced by G9.

Example (16b/G10) is identical to Example 16b with the proviso that G6 is replaced by G10.

Example 16b/G13) is identical to Example 16b with the proviso that G6 is replaced by G13.

Example (16c/G13) is identical to Example 16c with the proviso that G6 is replaced by G13.

Example (16c/G5) is identical to Example 16c with the proviso that G6 is replaced by G5.

### Example G/22:

A strand of blond undamaged human hair is coloured with 10 g of a composition G6. Then the pH is adjusted in the range of pH 5 to 8 by adding citric acid.
The colouring mixture is allowed to act on the hair for 30 minutes at about 22°C. After contact the strand is rinsed thoroughly, shampooed and then dried.
A strong, intense, striking red coloration having good fastness to washing and fastness to rubbing properties is obtained.

Example (22/G5) is identical to Example G/22 with the proviso that G6 is replaced by G5.

Example (22/G13) is identical to Example G/22 with the proviso that G6 is replaced by G13.

### Example G/23:

A strand of brown undamaged human hair is coloured
11a) with a mixture of 15 g of 6 % hydrogen peroxide solution and a composition consisting of 5 g each of compositions C, D1 and E11, and alternatively
11b) first with 10 g of 6 % hydrogen peroxide solution, and then with 5 g each of compositions C, D1 and E11 or
11c) first with 5 g each of compositions C, D1 and E11, then with 10 g of 6 % hydrogen peroxide solution.

The colouring mixture is allowed to act on the hair for 30 minutes at about 22°C. 10 g of a 2 % strength aqueous citric acid gel are then applied to the strand. After contact for 5 minutes, the strand is rinsed thoroughly, shampooed and then dried. A strong, intense, striking bluish red coloration having good fastness to washing and fastness to rubbing properties is obtained.

Example (23a/D2) is identical to Example 23a with the proviso that D1 is replaced by D2.

Example (23a/D3) is identical to Example 23a with the proviso that D1 is replaced by D3.

Example (23a/D4) is identical to Example 23a with the proviso that D1 is replaced by D4.

Example (23a/D5) is identical to Example 23a with the proviso that D1 is replaced by D5.

Example (23b/D2) is identical to Example 23b with the proviso that D1 is replaced by D2.

Example (23b/D3) is identical to Example 23b with the proviso that D1 is replaced by D3.

Example (23b/D4) is identical to Example 23b with the proviso that D1 is replaced by D4.

Example (23b/D5) is identical to Example 23b with the proviso that D1 is replaced by D5.

Example (23c/D2) is identical to Example 23c with the proviso that D1 is replaced by D2.

Example (23c/D3) is identical to Example 23c with the proviso that D1 is replaced by D3.

Example (23c/D4) is identical to Example 23c with the proviso that D1 is replaced by D4.

Example (23c/D5) is identical to Example 23c with the proviso that D1 is replaced by D5.

Example (23a/E12) is identical to Example 23a with the proviso that E11 is replaced by E12.

Example (23a/E16) is identical to Example 23a with the proviso that E11 is replaced by E10.

Example (23a/E15) is identical to Example 23a with the proviso that E11 is replaced by E7.

Example (23b/E12) is identical to Example 23b with the proviso that E11 is replaced by E12.

Example (23b/E19) is identical to Example 23b with the proviso that E11 is replaced by E10.

Example (23b/E15) is identical to Example 23b with the proviso that E11 is replaced by E7.

Example (23c/E16) is identical to Example 23c with the proviso that E11 is replaced by E10.

Example (23c/E12) is identical to Example 23c with the proviso that E11 is replaced by E12.

Example (23c/E19) is identical to Example 23c with the proviso that E11 is replaced by E7.

Example (23a/D2/E712) is identical to Example 23a/D2 with the proviso that that E11 is replaced by E12.

Example (23a/D3/E15) is identical to Example 23a/D3 with the proviso that that E11 is replaced by E10.

Example (23a/D4/E9) is identical to Example 23a/D4 with the proviso that E11 is replaced by E7.

Example (23b/D2/E19) is identical to Example 23b/D2 with the proviso that that E11 is replaced by E10.

Example (23b/D3/E12) is identical to Example 23b/D3 with the proviso that that E11 is replaced by E12.

Example (23b/D4/E16) is identical to Example 23b/D4 with the proviso that E11 is replaced by E7.

Example (23c/D2/E12) is identical to Example 23c/D2 with the proviso that that E11 is replaced by E12.

Example (23c/D3/E15) is identical to Example 23c/D3 with the proviso that that E11 is replaced by E10.

Example (23c/D4/E19) is identical to Example 23c/D4 with the proviso that E11 is replaced by E7.

### Example G/24:

A strand of blond undamaged human hair is coloured
24a) with a mixture of 5 g of 6 % hydrogen peroxide solution and a composition consisting of 5 g of composition E11 and alternatively
24b) first with 5 g of 6 % hydrogen peroxide solution, and then with 5 g of composition 11or
25c) first with 5 g of composition E11 and then with 5 g of 6 % hydrogen peroxide solution.
The colouring mixture is allowed to act on the hair for 30 minutes at about 22°C. 10 g of a 2 % strength aqueous citric acid gel are then applied to the strand. After contact for 5 minutes, the strand is rinsed thoroughly, shampooed and then dried. A strong, intense, striking bluish red coloration having good fastness to washing and fastness to rubbing properties is obtained.

Example (24a/E12) is identical to Example 24a with the proviso that E11 is replaced by E12.

Example (24b/E19) is identical to Example 24a with the proviso that E11 is replaced by E10.

Example (24c/E15) is identical to Example 24a with the proviso that E11 is replaced by E7.

Example (24b/E16) is identical to Example 24b with the proviso that E11 is replaced by E10.

Example (24b/E15) is identical to Example 24b with the proviso that E11 is replaced by E7.

Example (24b/E12) is identical to Example 24b with the proviso that E11 is replaced by E12.

Example (25c/E16) is identical to Example 25c with the proviso that E11 is replaced by E10.

Example (25c/E19) is identical to Example 25c with the proviso that E11 is replaced by E7.

Example (25c/E12) is identical to Example 25c with the proviso that E11 is replaced by E12.

### Example G/25:

A strand of blond undamaged human hair is coloured with
25a) 5 g of a composition according to the following table

| | |
|---|---|
| behentrimonium chloride | 3.8 g |
| cetylalcohol | 4 g |
| phenoxyethanol and isobutylparaben | 0.5 g |
| perfume | 0.1 g |
| Direct Dye of formula (6), wherein X⁻ is acetate | 0.5 g |
| monoethanolamine | ad pH 6.5 |
| Water | ad100 |

The colouring mixture is allowed to act on the hair for 30 minutes at about 22°C. After contact the strand is rinsed and then dried.
A strong, intense, striking coloration having good fastness to washing and fastness to rubbing properties is obtained.

Example (25a/G5) is identical to Example 25a with the proviso that Direct Dye of formula (6), is replaced by Direct Dye of formula (5), wherein X⁻is formate.

Example (25a/G9) is identical to Example 25a with the proviso that Direct Dye of formula (6), is replaced by Direct Dye of formula (9), wherein X⁻ is acetate.

Example (25a/G13) is identical to Example 25a with the proviso that Direct Dye of formula (6), by Direct Dye of formula (13), wherein X⁻ is acetate.

### Example G/26:

### 26a)

2 g of a composition A according to the following table

| COMPOSITION A | |
|---|---|
| sodium stearat | 11.0 g |
| aluminium distearat | 2.7 g |
| sodium laurylsulfat(Duponol C) | 1.0 g |
| disperse silicic acid (Aerosil 200) | 9.1 g |
| hydroxypropylcellulose | 2.7 g |
| ammoniumpersulfat | 19.0 g |
| sodium metasilicat | 12.0 g |
| disodium salt of ethylentetraminacetic acid | 1.0 g |
| potassium persulfat | 31.5 g |

and 3.6 g of a composition B according to the following table

| COMPOSITION B | |
|---|---|
| water | 188 g |
| hydrogenperoxide | 12 g |

are mixed to a homogenous mixture. This homogenous mixture is allowed to act on a strand of blond undamaged human hair for 30 minutes at about 22°C. After contact the strand is rinsed, shampooed.
Then the hair is coloured with 5 g of a composition according to the following table

| | |
|---|---|
| behentrimonium chloride | 3.8 g |
| cetylalcohol | 4 g |
| phenoxyethanol and Isobutylparaben | 0.5 g |
| perfume | 0.1 g |
| Direct Dye of formula (6), wherein X⁻is acetate | 0.5 g |
| monoethanolamine | ad pH 6.5 |
| Water | ad100 |

The colouring mixture is allowed to act on the hair for 30 minutes at about 22°C. After contact the strand is rinsed and then dried.
A strong, intense, striking coloration having good fastness to washing and fastness to rubbing properties is obtained.

Example (26a/G5) is identical to Example 26a with the proviso that direct dye of formula (6), wherein X⁻ is chloride is replaced by direct dye of formula (5), wherein X⁻ is formate.

Example (26a/G9) is identical to Example 26a with the proviso that direct dye of formula (6), wherein X⁻ is chloride is replaced by direct Dye of formula (9), wherein X⁻ is acetate. Example (26a/G10) is identical to Example 26a with the proviso that direct dye of formula (6), wherein X⁻ is chloride is replaced by direct dye of formula (10), wherein X⁻ is acetate.

### Example G/27:

### 27a)

2 g of a composition A according to the following table

| COMPOSITION A | |
|---|---|
| sodium stearat | 11.0 g |
| aluminium distearat | 2.7 g |
| sodium laurylsulfat(Duponol C) | 1.0 g |
| disperse silicic acid (Aerosil 200) | 9.1 g |
| hydroxypropylcellulose | 2.7 g |
| ammoniumpersulfat | 19.0 g |
| sodium metasilicat | 12.0 g |
| disodium salt of ethylentetraminacetic acid | 1.0 g |
| potassium persulfat | 31.5 g |
| Direct Dye of formula (6), wherein X⁻ is acetate | 10 g |

and 4 g of a composition B according to the following table

| COMPOSITION B | |
|---|---|
| water | 188 g |
| hydrogenperoxide | 12 g |

are mixed to a homogenous mixture. This homogenous mixture is allowed to act on a strand of blond undamaged human hair for 30 minutes at about 22°C. After contact the strand is rinsed, shampooed and dried.

Example (27a/G5) is identical to Example 27a with the proviso that direct dye of formula (6),
wherein X⁻ is chloride is replaced by direct dye of formula (5), wherein X⁻ is formate Example (27a/G13) is identical to Example 27a with the proviso that direct dye of formula (6), wherein X⁻ is chloride is replaced by direct dye of formula (13), wherein X⁻ is acetate. Example (27a/G9) is identical to Example 27a with the proviso that direct dye of formula (6),
wherein X⁻ is chloride is replaced by direct dye of formula (6), wherein X⁻ is acetate.

### Example G/28:

### 28a)

2 g of a composition A according to the following table

| COMPOSITION A | |
|---|---|
| sodium stearat | 11.0 g |
| aluminium distearat | 2.7 g |
| sodium laurylsulfat(Duponol C) | 1.0 g |
| disperse silicic acid (Aerosil 200) | 9.1 g |
| hydroxypropylcellulose | 2.7 g |
| ammoniumpersulfat | 19.0 g |
| sodium metasilicat | 12.0 g |
| disodium salt of ethylentetraminacetic acid | 1.0 g |
| potassium persulfat | 31.5 g |
| Direct Dye of formula (6), wherein X⁻ is acetate | 10 g |

and 4 g of a composition B according to the following table

| COMPOSITION B | |
|---|---|
| water | 188 g |
| hydrogenperoxide | 12 g |

are mixed to a homogenous mixture. This homogenous mixture is allowed to act on a strand of blond undamaged human hair for 30 minutes at about 22°C. After contact the strand is rinsed and shampooed.
Then the hair is coloured with 5 g of a composition according to the following table

| | |
|---|---|
| behentrimonium chloride | 3.8 g |
| cetylalcohol | 4 g |
| phenoxyethanol and Isobutylparaben | 0.5 g |
| perfume | 0.1 g |
| Direct Dye of formula (6), wherein X⁻ is acetate | 0.5 g |
| monoethanolamine | ad pH 6.5 |
| Water | ad100 |

The colouring mixture is allowed to act on the hair for 30 minutes at about 22°C. After contact the strand is rinsed and then dried.
A strong, intense, striking coloration having good fastness to washing and fastness to rubbing properties is obtained.

Example (28a/G5) is identical to Example 28a with the proviso that direct dye of formula (6), wherein X⁻ is acetate is replaced by direct dye of formula (5), wherein X⁻ is formate:

Example (28a/G10) is identical to Example 28a with the proviso that direct dye of formula (6), wherein X⁻ is chloride is replaced by direct dye of formula (10), wherein X⁻ is succinate.

Example (28a/G9) is identical to Example 28a with the proviso that direct dye of formula (6), wherein X⁻ is chloride is replaced by direct dye of formula (9), wherein X⁻is acetate.

### Example G/30:

### 30a)

2 g of a composition A according to the following table

| COMPOSITION A | |
|---|---|
| sodium stearat | 11.0 g |
| aluminium distearat | 2.7 g |
| sodium laurylsulfat(Duponol C) | 1.0 g |
| disperse silicic acid (Aerosil 200) | 9.1 g |
| hydroxypropylcellulose | 2.7 g |
| ammoniumpersulfat | 19.0 g |
| sodium metasilicat | 12.0 g |
| disodium salt of ethylentetraminacetic acid | 1.0 g |
| potassium persulfat | 31.5 g |

is mixed with 2 g of a composition C,
and 4 g of a composition B according to the following table

| COMPOSITION B | |
|---|---|
| water | 188 g |
| hydrogenperoxide | 12 g |

to a homogenous mixture. This homogenous mixture is allowed to act on a strand of blond undamaged human hair for 30 minutes at about 22°C. After contact the strand is rinsed and shampooed.
Then the hair is coloured with 5 g of a composition according to the following table

| | |
|---|---|
| behentrimonium chloride | 3.8 g |
| cetylalcohol | 4 g |
| phenoxyethanol and lsobutylparaben | 0.5 g |
| perfume | 0.1 g |
| Direct Dye of formula (6), wherein X⁻ is acetate | 0.5 g |
| monoethanolamine | ad pH 6.5 |
| Water | ad100 |

The colouring mixture is allowed to act on the hair for 30 minutes at about 22°C. After contact the strand is rinsed and then dried.
A strong, intense, striking coloration having good fastness to washing and fastness to rubbing properties is obtained.

Example (30a/G5) is identical to Example 30a with the proviso that direct dye of formula (6), wherein X' is chloride is replaced by direct dye of formula (5), wherein X⁻ is formate.

Example (30a/G10) is identical to Example 30a with the proviso that direct dye of formula (6), wherein X'is chloride is replaced by direct dye of formula (10), wherein X⁻ is succinate.

Example (30a/G9) is identical to Example 30a with the proviso that direct dye of formula (6), wherein X⁻ is chloride is replaced by direct dye of formula (9), wherein X⁻ is acetate.

### Example G/31:

### 31a)

2 g of a composition A according to the following table

| COMPOSITION A | |
|---|---|
| sodium stearat | 11.0 g |
| aluminium distearat | 2.7 g |
| sodium laurylsulfat(Duponol C) | 1.0 g |
| disperse silicic acid (Aerosil 200) | 9.1 g |
| hydroxypropylcellulose | 2.7 g |
| ammoniumpersulfat | 19.0 g |
| sodium metasilicat | 12.0 g |
| disodium salt of ethylentetraminacetic acid | 1.0 g |
| potassium persulfat | 31.5 g |

is mixed with a 2.0 g of a composition E11
and 6 g of a composition B according to the following table

| COMPOSITION B | |
|---|---|
| water | 188 g |
| hydrogenperoxide | 12 g |

to a homogenous mixture. This homogenous mixture is allowed to act on a strand of blond undamaged human hair for 30 minutes at about 22°C. After contact the strand is rinsed, shampooed and dried.

Example (31a/E12) is identical to Example 31 a with the proviso that E11 is replaced by E12.

Example (31b/E15) is identical to Example 31 a with the proviso that E11 is replaced by E10.

Example (31c/E19) is identical to Example 31 a with the proviso that E11 is replaced by E7.

### Example G/32:

### 32a)

2 g of a composition A according to the following table

| COMPOSITION A | |
|---|---|
| sodium stearat | 11.0g |
| aluminium distearat | 2.7 g |
| sodium laurylsulfat(Duponol C) | 1.0 g |
| disperse silicic acid (Aerosil 200) | 9.1 g |
| hydroxypropylcellulose | 2.7 g |
| ammoniumpersulfat | 19.0 g |
| sodium metasilicat | 12.0 g |
| Di sodium salt of ethylentetraminacetic acid | 1.0 g |
| potassium persulfat | 31.5 g |

is mixed with a 2.0 g of a COMPOSITION C as given in example G and 2.0g of a direct dye of formula (6), wherein X⁻ is acetate and 8 g of a COMPOSITION B according to the
following table

| COMPOSITION B | |
|---|---|
| water | 188 g |
| hydrogenperoxide | 12 g |

to a homogenous mixture. This homogenous mixture is allowed to act on a strand of blond undamaged human hair for 30 minutes at about 22°C. After contact the strand is rinsed, shampooed and dried.

Example (32a/G5) is identical to Example 32a with the proviso that direct dye of formula (6),
wherein X⁻ is acetate is replaced by direct dye of formula (5), wherein X⁻ is formate.

Example (32a/G13) is identical to Example 32a with the proviso that direct dye of formula (6), wherein X⁻ is acetate is replaced by direct dye of formula (13), wherein X⁻ is acetate.

Example (32a/G9) is identical to Example 32a with the proviso that direct dye of formula (6),
wherein X⁻ is acetate is replaced by direct dye of formula (9), wherein X⁻ is acetate.

### Example G/33:

### 33a)

### Composition (A')

| | |
|---|---|
| polyglycerol alcohol with 2 mols of glycerol | 4.0g |
| polyglycerol alcohol wit h 4 mols of glycerol of 78% (M.A.) | 5.69g |
| oil acid | 3.0 g |
| oleic amine with 2 mols ethylenoxide available from ETHOMEEN 012 from AKZO | 7.0g |
| laurylamine succinamate of diethylaminopropylene, salt of sodium with 55%. | 3.0 g |
| oil alcohol | 5.0g |
| diethynolamide of oil acid | 12.0g |
| propylenglycol | 3.5g |
| ethylenalcohol | 7.0g |
| monobutylether of diethylenglycol | 0.5g |
| monomethylether of propylenglycol | 0.5g |
| sodium metabisulfite as solution a 35% | 0.455g |
| ammonium acetate | 0.8g |
| paraphenylendiamine | 0.35g |
| 1,3-dihydroxybenzene | 0.4g |
| 3-amino phenol | 0.03g |
| 2,4-diamino-1-(β-hydroxyethy)oxy)benzene, 2HCl | 0.012g |
| 1,3-bis-[(4-aminophenyl)2-hydroxyethyl)-amino]-2-propanol, 4HCl | 0.037g |
| 1,3-dihydroxy-2-methyl-benzene | 0.2g |
| antioxidant | qs |
| parfume | qs |
| ammonia 20% of NH₃ | 10.0 g |
| water | 100g |

### Composition (B')

| | |
|---|---|
| direct dye of formula (6), wherein X⁻ is acetate in powder form | 20g |
| oil of parafine | 3g |
| cationic polymeric powder (Merquat 280 Dry de Calgon) | 10g |
| sawdust | 100g |

### Composition (C'):

| | |
|---|---|
| hydrogenperoxide 20% by volume | |

Just before the colouration of human hair a mixture of 1 equivalent of weight of Composition (A'), 01 equivalent of weight of compostion (B') and 1 equivalent of weight of composition (C') is mixed.
The pH of the mixture is adjusted to 9.8.

The colouring mixture is applied on human grey hair. This mixture is allowed to act on a strand of blond undamaged human hair for 30 minutes. After contact the strand is rinsed, shampooed and dried.

Example (33a/G5) is identical to Example 33a with the proviso that direct dye of formula (6), wherein X⁻ is acetate is replaced by direct dye of formula (5), wherein X⁻ is formate.

Example (33a/G13) is identical to Example 33a with the proviso that direct dye of formula (6), wherein X⁻ is acetate is replaced by direct dye of formula (13), wherein X⁻ is acetate.

Example (33a/G9) is identical to Example 33a with the proviso that G6 is replaced by direct dye of formula (9), wherein X⁻is acetate.

### Example G/34:

### 34a)

### Composition (B')

| | |
|---|---|
| direct dye of formula (6), wherein X⁻ is acetate in powder form | 20g |
| Oil of parafine | 3g |
| cationic polymeric powder (Merquat 280 Dry de Calgon) | 10g |
| sawdust | 100g |

### Composition (C'):

| | |
|---|---|
| hydrogenperoxide 20% by volume | 100g |

Just before the colouration of human hair a mixture of 1 equivalent of weight of Composition (A'), and 1 equivalent of weight of composition (C') is mixed.
The pH of the mixture is adjusted to 9.8.
The colouring mixture is applied on human grey hair. This mixture is allowed to act on a strand of blond undamaged human hair for 30 minutes. After contact the strand is rinsed, shampooed and dried.

Example (34a/G5) is identical to Example 34a with the proviso that direct dye of formula (6),
wherein X⁻ is acetate is replaced by direct dye of formula (5), wherein X⁻ is formate.

Example (34a/G9) is identical to Example 34a with the proviso that direct dye of formula (6),
wherein X⁻ is acetate is replaced by direct dye of formula (9), wherein X⁻ is acetate.

Example (34a/G10) is identical to Example 34a with the proviso that direct dye of formula
(6), wherein X'is acetate is replaced by direct dye of formula (10), wherein X⁻ is succinate.

### Example G/35:

### 35a)

### Composition (B')

| | |
|---|---|
| direct dye of formula (6), wherein X⁻ is acetate, powder form | 20g |
| Oil of parafine | 3g |
| cationic polymeric powder (Merquat 280 Dry de Calgon) | 10g |
| sawdust | 100g |

### Composition (C'):

| | |
|---|---|
| hydrogenperoxide 20% by volume | 100g |

Just before the colouration of human hair a mixture of 1 equivalent of weight of Composition (A'), and 1 equivalent of weight of composition (C') is mixed.

The pH of the mixture is adjusted to 9.8 with ammonia 20% by volume.
The colouring mixture is applied on human grey hair. This mixture is allowed to act on a strand of blond undamaged human hair for 30 minutes. After contact the strand is rinsed, shampooed and dried.
Example (35a/G13) is identical to Example 35a with the proviso that direct dye of formula (6), wherein X⁻ is acetate is replaced by direct dye of formula (13), wherein X⁻is acetate.

Example (35a/G9) is identical to Example 35a with the proviso that direct dye of formula (6),
wherein X⁻ is acetate is replaced by direct dye of formula (9), wherein X⁻ is acetate.

Example (35a/G5) is identical to Example 35a with the proviso that direct dye of formula (6),
wherein X⁻ is acetate is replaced by direct dye of formula (5), wherein X⁻ is formate.

### Example 36:

26 g of the dye of formula 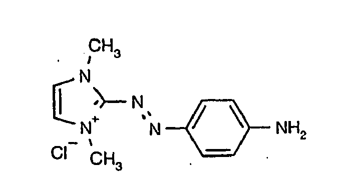 are heated to 60°C in 60 g of methanol. 10 g of potassium acetate are added thereto and stirring is carried out for 4 hours at 60°C. The mixture is then allowed to cool to room temperature and is filtered. The filter cake is then washed with 5 g of methanol, yielding a residue of 6.5 g of almost colourless salt, which consists almost entirely of potassium chloride. The methanolic mother liquor is concentrated to dryness by evaporation in a rotary evaporator, yielding 29 g of a dark-violet powder which is substantially the acetate of the dye that was used and has a chloride content of only 1.6 % (the starting product contains 14.1 %) and a water solubility of over 25 %. The water solubility of the chloride of the dye used is only 3.7 %.

### Example 37:

50 g of the same dye as in Example 36 are heated to 60°C in 190 g of isopropanol, and 23.4 g of potassium acetate are then added thereto. The temperature is raised to 80°C and maintained for 7 hours. The hot solution is then filtered through a pre-heated suction filter and the residue is washed with a small amount of isopropanol. About 14 g of almost colourless salt (potassium chloride) are obtained. Upon cooling to room temperature, the acetate of the dye crystallises from the mother liquor in the form of attractive crystals. Filtration, washing with a small amount of isopropanol and drying yield 38.5 g in very pure form and with a chloride content of only 0.6 %. The mother liquor is concentrated to dryness by evaporation *in vacuo* at a maximum of 50°C, yielding 21 g of a mixture of about 85 % acetate of the dye and 15 % potassium acetate. It is possible to prepare from the two fractions a clear, stable, 30 % aqueous solution.

### Example 38:

10.3 g of the same starting material as in Example 36 and 3.0 g of potassium carbonate are suspended in 50 g of isopropanol and heated to 60°C. 4 g of anhydrous lactic acid are then added thereto, and stirring is carried out for 4 hours at 60°C. The mixture is then diluted with a further 64 g of isopropanol and the temperature is raised to 80°C in order to dissolve all the dye. Clarifying filtration is carried out through a suction filter pre-heated to 80°C, and the residue is then washed with 18 g of isopropanol, yielding a potassium chloride residue of 2.47 g, which corresponds to about 94 % of the expected amount. The filtrate is allowed to cool, with stirring, whereupon the lactate of the dye precipitates in crystalline form. Filtration, washing and drying yield 8.84 g of dark dye powder whose solubility in water is over 20 %, whereas the solubility of the starting product is only 3.7 %.

### Example 39:

The procedure is exactly as in Example 38 at first, but 3.3 g of propionic acid are added instead of the lactic acid and the temperature is immediately raised to 80°C. After 2 hours, clarifying filtration is carried out through a pre-heated suction filter, and the residue is washed with 65 g of isopropanol. The largely colourless clarified residue is 2.85 g and consists of 44.7 % chloride. The filtrate is concentrated to about 1/3 as gently as possible in a rotary evaporator and then allowed to cool, with stirring. The resulting dye is filtered off with suction, washed with 63 g of isopropanol and dried, yielding 8.2 g of crystal powder having a chloride content of only 1.4 % (about 1/10 of the original chloride content). The solubility of the propionate of the dye in water is likewise over 30 %.

### Example 40:

10.7 g of the dye of formula 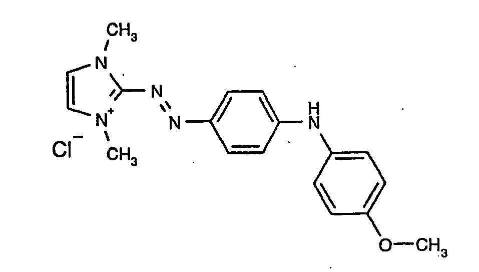 are suspended in 70 g of isopropanol, together with 2.6 g of potassium formate, and heated to 80°C. From about 62°C, the starting materials are largely dissolved and salt crystals are immediately observed on the wall of the flask. After 4 hours, clarifying filtration is carried out through a pre-heated suction filter, and the residue is then washed with 47 g of isopropanol. The dry residue weighs 2.04 g and consists almost entirely of potassium chloride. Upon cooling, the formate of the dye crystallises from the filtrate in the form of violet crystals; yield 7.4 g. The mother liquor is concentrated to dryness by evaporation *in vacuo* and yields a further 3.75 g of the formate of the dye of almost equal purity. The chloride contents of those two fractions are only 0.5 % and 0.23 %, respectively, which likewise indicates an anion exchange of over 95 %. Accordingly, their water solubility is over 30 %, whereas that of the chloride used is only 0.35 %.

### Example 41:

10.3 g of the dye chloride from Example 36 are suspended in 50 g of isopropanol, with 3.0 g of potassium carbonate and 11.5 g of stearic acid, and heated to 60°C. After 4 hours, the temperature is raised to 80°C, a further 200 g of isopropanol are added, and clarifying filtration is carried out through a heated suction filter at 80°C. 3.1 g of almost colourless salt remain on the filter. Upon cooling to room temperature, the stearate of the dye precipitates from the mother liquor in the form of a crystalline precipitate. The latter is filtered off, washed with 30 g of isopropanol and dried, yielding 10.7 g of a brown-violet dye powder. By concentration of the mother liquor it is readily possible to obtain a further 5 g of product of equally good quality.

### Example 42:

4.0 g of the oxazine dye of formula 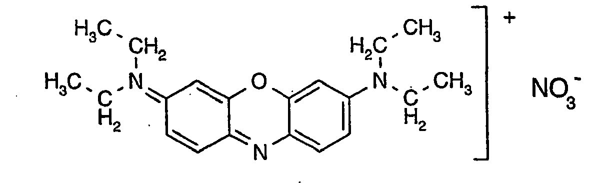 are placed, together with 0.72 g of potassium carbonate, in 13 g of isopropanol, 1.2 g of trifluoroacetic acid are added to the highly fluid suspension, whereupon the mixture becomes viscous. However, it rapidly becomes highly fluid again upon heating. Stirring is carried out for 4 hours at a temperature of 60°C, the temperature is then raised to 80°C, and the precipitated salt is filtered off while hot. The residue is rinsed with a small amount of isopropanol. Upon cooling, with stirring, the trifluoroacetate of the dye precipitates from the mother liquor in the form of a crystalline precipitate. The latter is filtered off, washed with a small amount of isopropanol and dried, yielding 1.9 g of a dye powder whose solubility in water is over 1.5 %, whereas the solubility of the starting product is less than 0.4 %. Neither the isolated product nor the mother liquor, which still contains a large amount of product, exhibit any decomposition. From the nitrate that is still present (about 3 %) it is possible to conclude that the exchange has taken place to the extent of about 82 %. A further 2.2 g of dye of equal quality can be obtained from the mother liquor by gentle concentration.

### Example 43:

4 g of the dye of formula 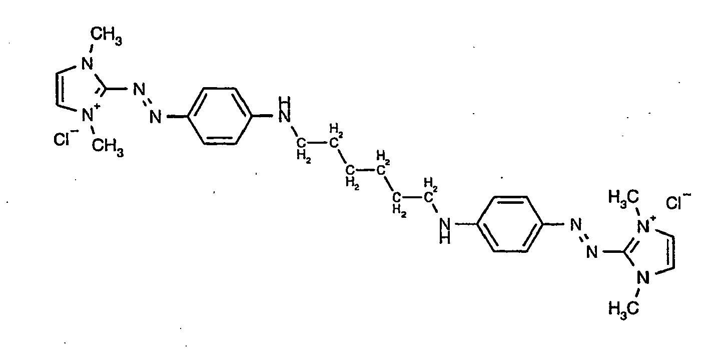 and 1.5 g of potassium acetate are suspended in 20 g of isopropanol and stirred for 5 hours at 60°C. Clarifying filtration is then carried out while hot. The salt residue is 1.12 g. The acetate of the dye crystallises from the mother liquor. Filtration and washing with isopropanol yield 3.43 g (dry) of dark crystal powder whose solubility in water has increased one hundred fold from < 0.03 % to 3 %.

### Example 44:

If in Example 43 the potassium acetate is replaced by 1.3 g of potassium formate and heating is carried out for 4 hours at 80°C, there are obtained, with an otherwise identical procedure and working up, 3.54 g of the formate of the dye, whose solubility in water is about 1.2 %. The salt residue from the clarifying filtration is 1.0 g.

### Example 45:

1.29 g of potassium acetate are added to 3 g of the compound of formula 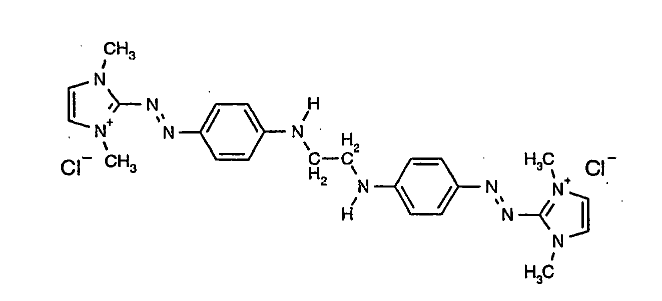 in 15 g of methanol, and stirring is carried out for 4 hours at a temperature of 55°C. A highly fluid suspension is obtained. Upon cooling, no dye precipitates and the clarifying filtration can therefore be carried out at room temperature; clarified residue 1.1 g. The mother liquor is concentrated to dryness by evaporation under gentle conditions at 50°C and 70 mbar, yielding 3.1 g of the acetate of the dye which, in contrast to the starting product, is excellently soluble in water.

### Example 46:

By means of the procedure described in Example 36 it is also possible to convert the chloride of the optical brightener of the following formula into the acetate:

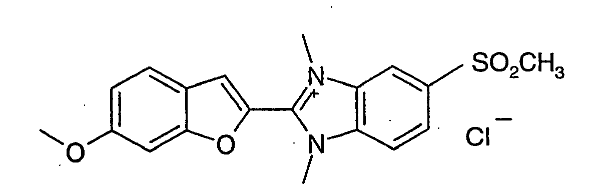

### Example 47:

By means of the procedure described in Example 36 it is also possible to convert the chloride of the UV absorber of the following formula into the acetate:

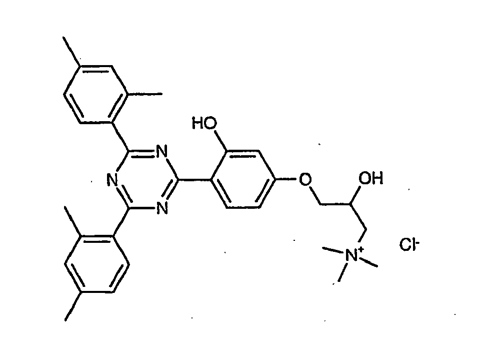

### Example 48:

By means of the procedure described in Example 36 it is also possible to convert the chloride of the cationic dye of the following formula into the acetate:

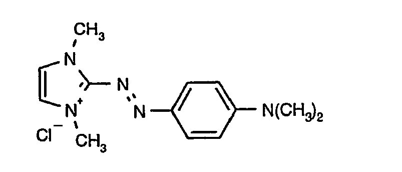

### Example 49:

By means of the procedure described in Example 36 it is also possible to convert the chloride of the cationic dye of the following formula into the acetate:

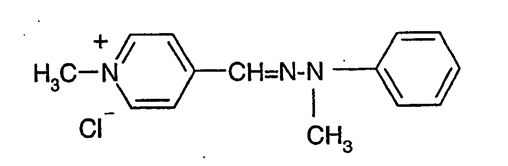

### Example 50:

By means of the procedure described in Example 36 it is also possible to convert the chloride of the cationic dye of the following formula into the acetate:

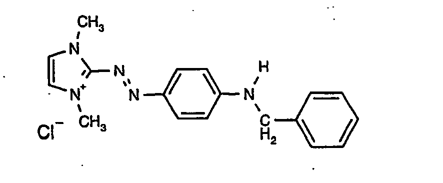

## Claims

1. A process for converting sparingly soluble salts of cationic dyes and inorganic acids into more readily soluble salts of organic acids, which process comprises
a) preparing a sparingly water-soluble salt of the cationic dyes with the anion of an inorganic acid,
b) adding thereto, in a monohydric aliphatic alcohol, an alkali metal salt of an organic acid,
c) filtering off the resulting sparingly soluble alkali metal salt of the inorganic acid, and
d) optionally converting the resulting solution into a solid form,

2. A process according to claim 1, wherein a monoazo, disazo or hydrazon dye is used.

3. A process according to any one of claims 1 and 2, wherein there is used a dye of formula 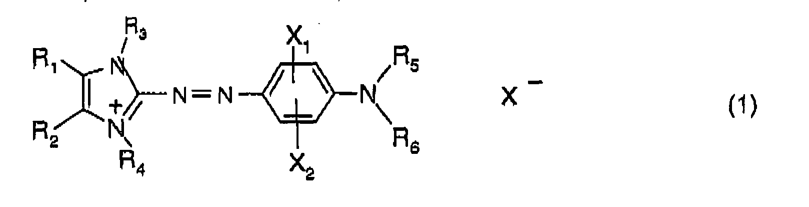 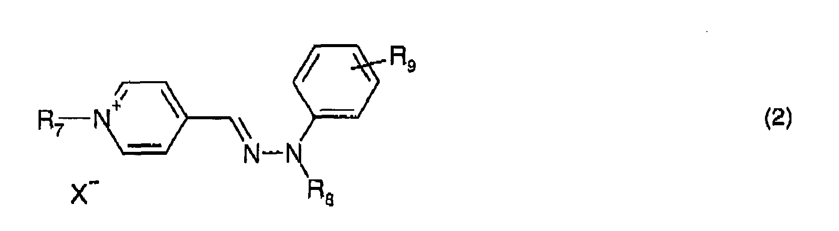 or 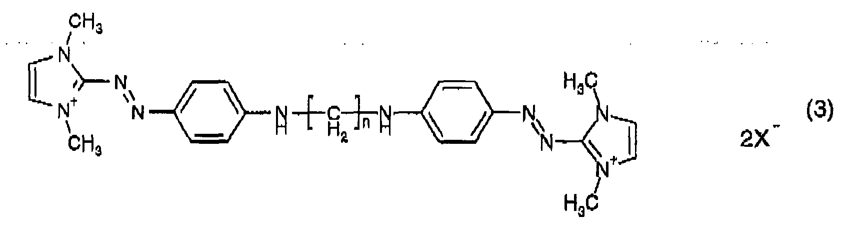 wherein
R₁ and R₂ are each independently of the other hydrogen, C₁-C₄alkyl, halogen or nitro,
R₃ and R₄ are each independently of the other unsubstituted C₁-C₄alkyl or C₁-C₄alkyl substituted by OH, C₁-C₄alkoxy, halogen, CN or by phenyl,
X₁ and X₂ are each independently of the other hydrogen, C₁-C₄alkyl, C₁-C₄alkoxy or halogen, R₅ is hydrogen or C₁-C₄alkyl,
R₆ is hydrogen, phenyl, C₁-C₁₂alkyl or C₅-C₈cycloalkyl, each of which may be unsubstituted or substituted by OH, G₁-C₄alkoxy, halogen, CN, or R₈ is C₁-C₄alkyl substituted by phenyl or by C₅-C₈cycloalkyl,
or wherein
R₅ and R₆, together with the nitrogen atom linking them, form a piperazine ring which is substituted by C₁-C₆alkyl or by phenyl on the nitrogen atom not bonded to the phenyl ring or which is quaternised at that nitrogen atom by means of two such groups, the C₁-C₆alkyl and phenyl radicals mentioned as substituents on the nitrogen atom of the piperazine ring being unsubstituted or substituted by OH, C₁-C₄alkoxy, halogen, CN or by phenyl, and
wherein
R₇ and R₈ are each independently of the other a C₁-C₈alkyl radical or an unsubstituted or substituted benzyl radical, and
R₉ is hydrogen, C₁-C₈alkyl, C₁-C₈alkoxy, cyanide or halide, and
n is a whole number in the range from 2 to 12, and, wherein X⁻ is an anion.

4. A process according to any one of claims 1 to 3, wherein there is used a dye of formula 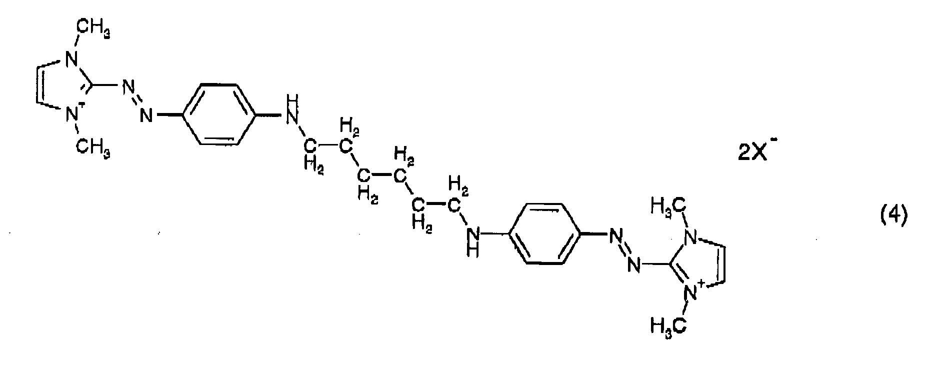 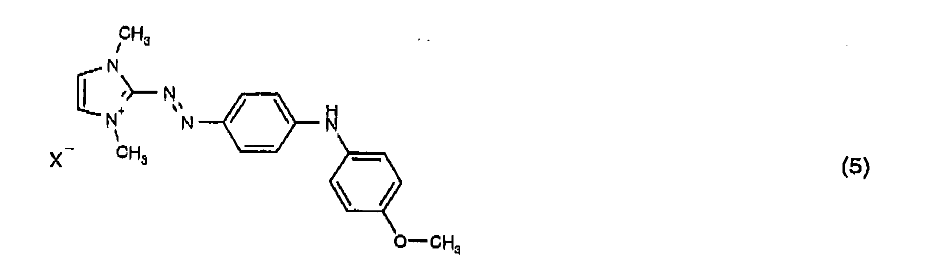 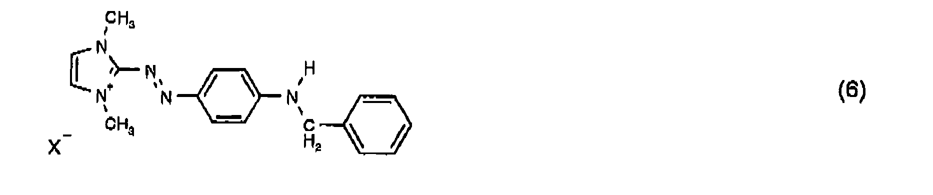 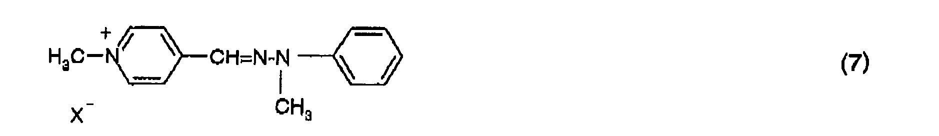 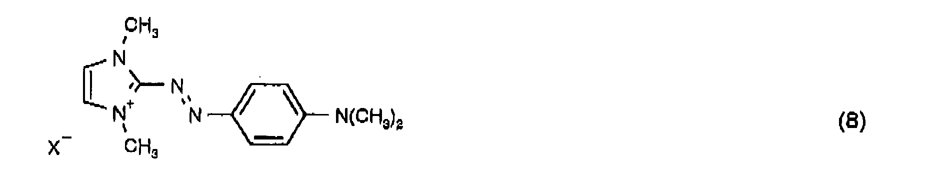 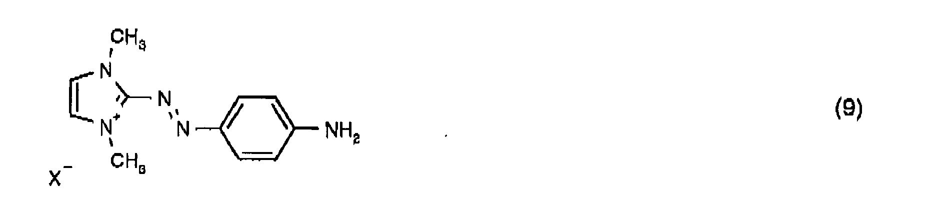 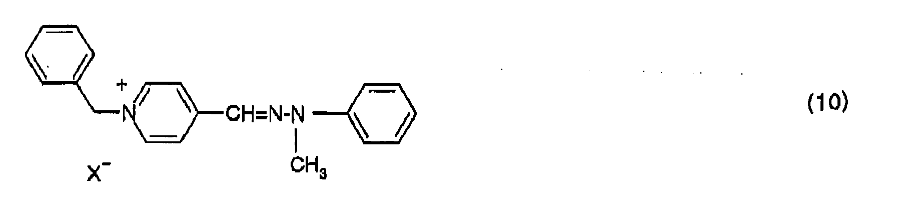 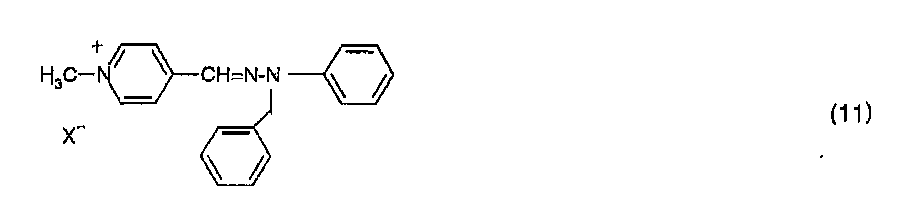 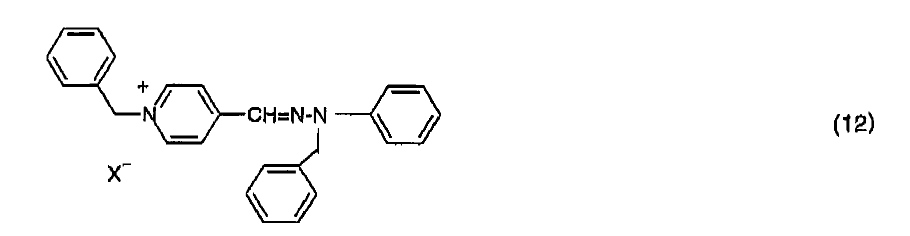 and 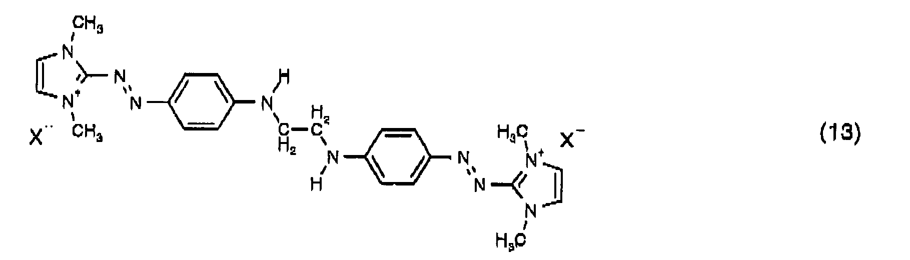 wherein X⁻ is an anion.

5. A process according to any one of claims 1 to 4, wherein the cationic dye is used in the form of an iodide, bromide, chloride, sulfate, hydrogen sulfate, methyl sulfate, phosphate, borfluorate or perchlorate,

6. A process according to any one of claims 1 to 5, wherein there is used as the alkali metal salt of an organic acid a formate, acetate, propionate, butyrate, monochloroacetate, trifluoroacetate, tartrate, oxalate, stearate, maleate, acrylate, succinate, citrate, lactate, methanesulfonate or ethanesulfonate.

7. A process according to claim 6, wherein a formate, acetate, stearate, propionate, citrate, lactate or trifluoroacetate is used.

8. A process according to any one of claims 1 to 7, wherein a lithium salt, sodium salt or, especially, a potassium salt is used.

9. A process according to any one of claims 1 to 8, wherein methanol, ethanol, n- or iso-propanol, n-, iso- or tert-butanol is used as the alcohol.

10. A process according to claim 9, wherein methanol, ethanol or isopropanol is used.

## Patentansprüche

1. Verfahren zum Umwandeln schlecht löslicher Salze von kationischen Farbstoffen und anorganischen Säuren in leichter lösliche Salze von organischen Säuren, wobei das Verfahren umfasst:
a) Herstellen eines in Wasser schlecht löslichen Salzes von den kationischen Farbstoffen mit dem Anion von einer anorganischen Säure,
b) Zusetzen dazu in einem einwertigen aliphatischen Alkohol eines Alkalimetallsalzes von einer organischen Säure,
c) Abfiltrieren des erhaltenen schlecht löslichen Alkalimetallsalzes von der anorganischen Säure und
d) gegebenenfalls Umwandeln der erhaltenen Lösung in eine feste Form.

2. Verfahren nach Anspruch 1, wobei ein Monoazo-, Diazo- oder Hydrazon-Farbstoff verwendet wird.

3. Verfahren nach einem der Ansprüche 1 und 2, wobei ein Farbstoff der Formel 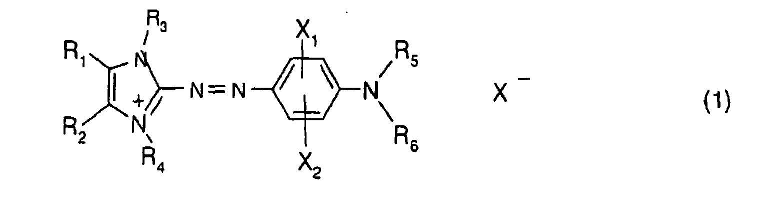 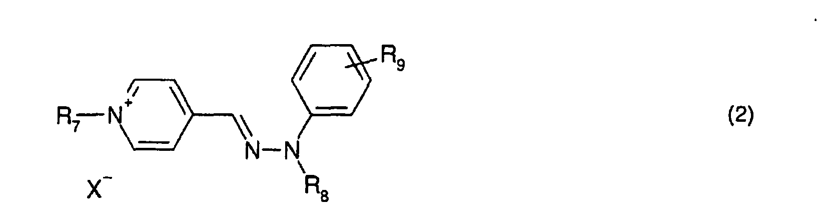 oder 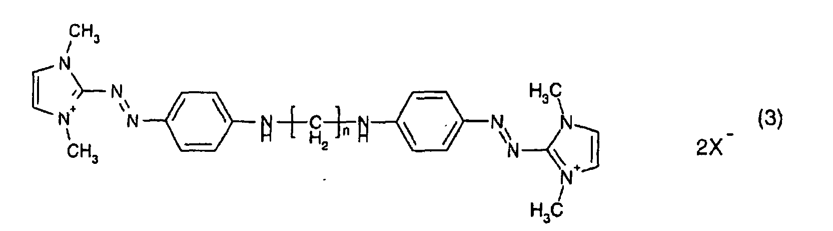 eingesetzt wird, worin R₁ und R₂ jeweils unabhängig voneinander Wasserstoff, C₁-C₄-Alkyl, Halogen oder Nitro darstellen,
R₃ und R₄ jeweils unabhängig voneinander unsubstituiertes C₁-C₄-Alkyl oder C₁-C₄-Alkyl, substituiert mit OH, C₁-C₄-Alkoxy, Halogen, CN oder mit Phenyl, darstellen,
X₁ und X₂ jeweils unabhängig voneinander Wasserstoff, C₁-C₄-Alkyl, C₁-C₄-Alkoxy oder Halogen darstellen,
R₅ Wasserstoff oder C₁-C₄-Alkyl darstellt,
R₆ Wasserstoff, Phenyl, C₁-C₁₂-Alkyl oder C₅-C₈-Cycloalkyl darstellt, wobei jeder davon unsubstituiert oder mit OH, C₁-C₄-Alkoxy, Halogen, CN substituiert sein kann, oder R₆ C₁-C₄-Alkyl, substituiert mit Phenyl oder mit C₅-C₈-Cycloalkyl, darstellt,
oder worin
R₅ und R₆ zusammen mit dem sie verbindenden Stickstoffatom einen Piperazinring bilden, der mit C₁-C₈-Alkyl oder mit Phenyl an dem nicht an den Phenylring gebundenen Stickstoffatom substituiert ist oder der an dem Stickstoffatom mithilfe von zwei solchen Gruppen quaternisiert ist, wobei die C₁-C₈-Alkyl- und Phenylreste, die als Substituenten an dem Stickstoffatom des Piperazinrings erwähnt wurden, unsubstituiert oder mit OH, C₁-C₄-Alkoxy, Halogen, CN oder mit Phenyl substituiert sind, und
worin
R₇ und R₈ jeweils unabhängig voneinander einen C₁-C₈-Alkylrest oder einen unsubstituierten oder substituierten Benzylrest darstellen, und
R₉ Wasserstoff, C₁-C₈-Alkyl , C₁-C₈-Alkoxy, Cyanid oder Halogenid darstellt, und
n eine ganze Zahl in dem Bereich von 2 bis 12 ist, und
worin X⁻ ein Anion darstellt.

4. Verfahren nach einem der Ansprüche 1 bis 3, wobei ein Farbstoff der Formel 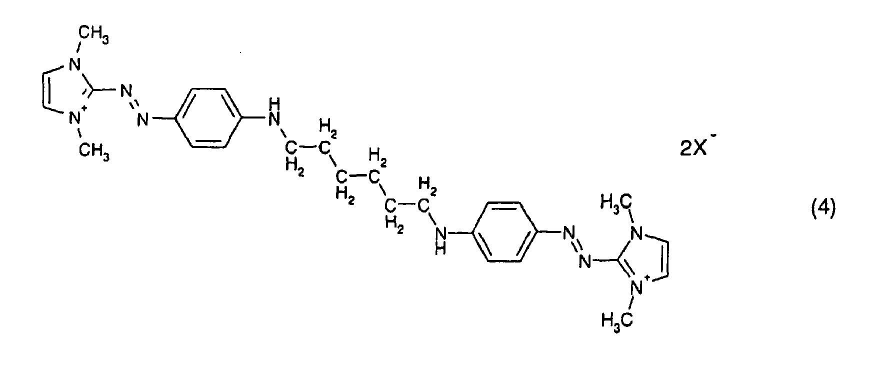 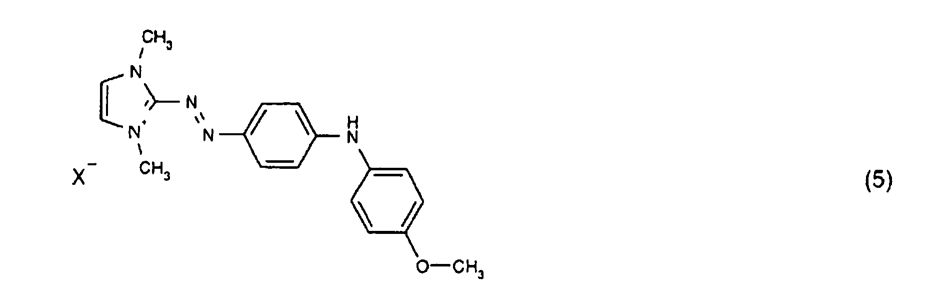 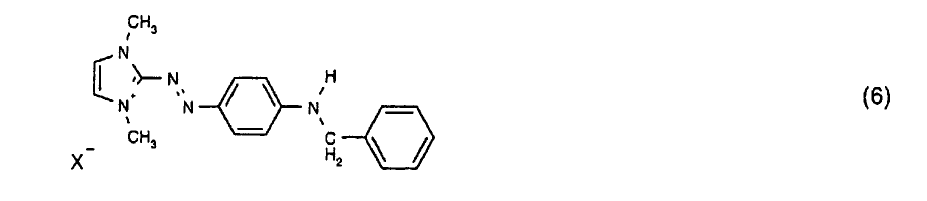 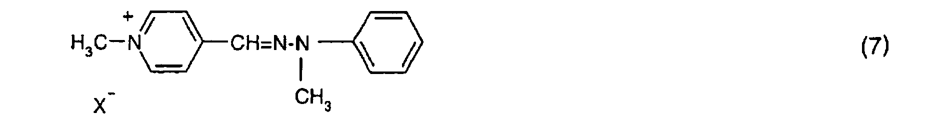 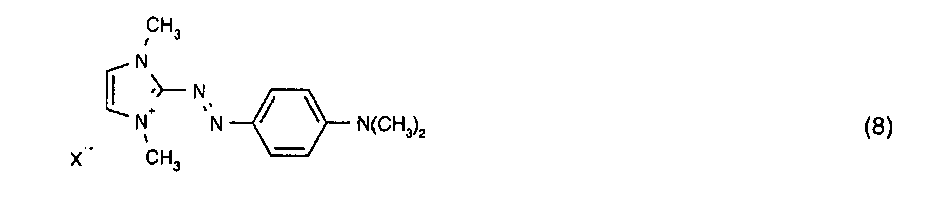 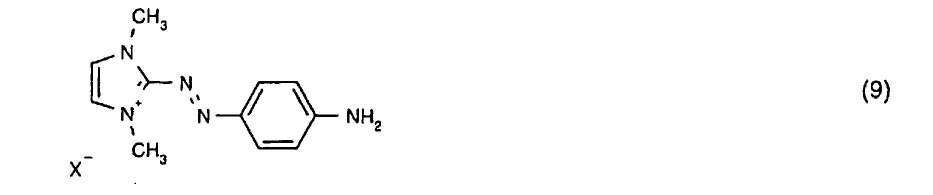 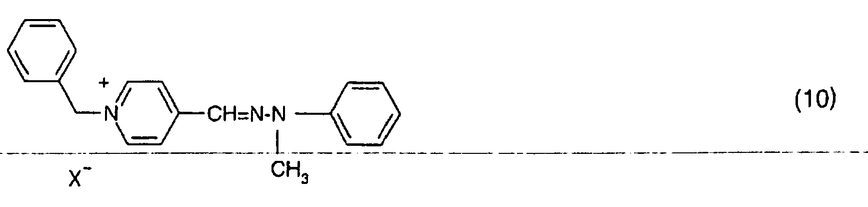 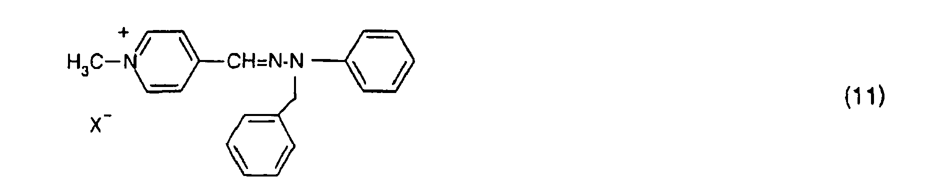 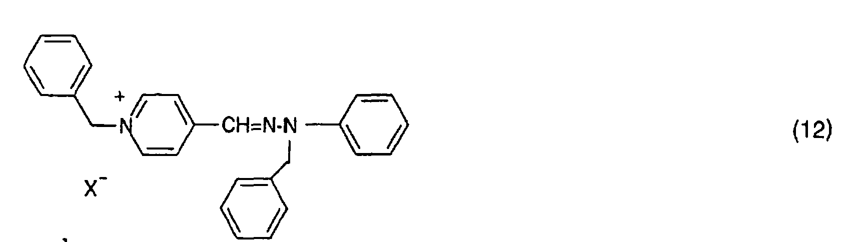 und 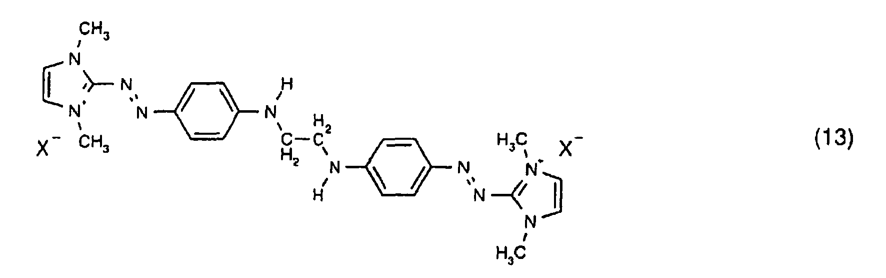 worin X⁻ ein Anion darstellt, verwendet wird.

5. Verfahren nach einem der Ansprüche 1 bis 4, wobei der kationische Farbstoff in Form eines Jodids, Bromids, Chlorids, Sulfats, Hydrogensulfats, Methylsulfats, Phosphats, Borfluorats oder Perchlorats verwendet wird.

6. Verfahren nach einem der Ansprüche 1 bis 5, wobei als das Alkalimetallsalz von einer organischen Säure ein Formiat, Acetat, Propionat, Butyrat, Monochloracetat, Trifluoracetat, Tartrat, Oxalat, Stearat, Maleat, Acrylat, Succinat, Citrat, Lactat, Methansulfonat oder Ethansulfonat verwendet wird.

7. Verfahren nach Anspruch 6, wobei ein Formiat, Acetat, Stearat, Propionat, Citrat, Lactat oder Trifluoracetat verwendet wird.

8. Verfahren nach einem der Ansprüche 1 bis 7, wobei ein Lithiumsalz, Natriumsalz oder insbesondere ein Kaliumsalz verwendet wird.

9. Verfahren nach einem der Ansprüche 1 bis 8, wobei Methanol, Ethanol, n- oder Isopropanol, n-, Iso- oder tert-Butanol als der Alkohol verwendet wird.

10. Verfahren nach Anspruch 9, wobei Methanol, Ethanol oder Isopropanol verwendet wird.

## Revendications

1. Procédé de conversion de sels modérément solubles de colorants cationiques et d'acides inorganiques en sels plus facilement solubles d'acides organiques, procédé qui comprend les étapes consistant à :
a) préparer un sel modérément hydrosoluble des colorants cationiques avec l'anion d'un acide inorganique,
b) y ajouter, dans un monoalcool aliphatique, un sel de métal alcalin d'un acide organique,
c) éliminer par filtration le sel de métal alcalin modérément soluble ainsi obtenu de l'acide inorganique, et
d) convertir éventuellement la solution résultante sous forme solide.

2. Procédé selon la revendication 1, dans lequel un colorant monoazoïque, diazoïque ou hydrazonique est utilisé.

3. Procédé selon l'une quelconque des revendications 1 et 2, dans lequel il est utilisé un colorant de formule : 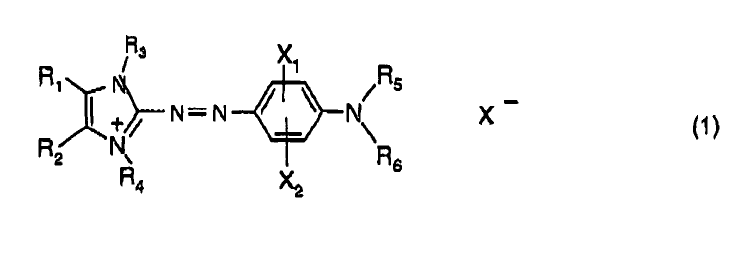 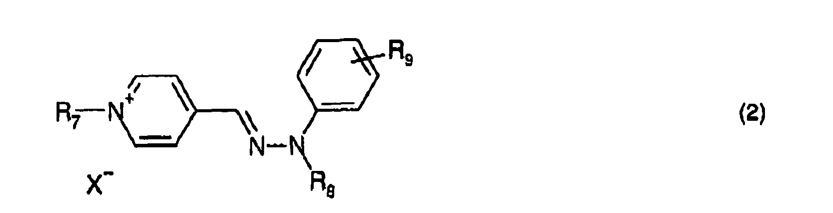 ou 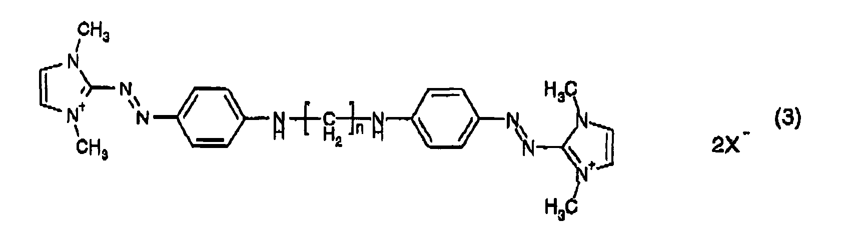 dans laquelle
R₁ et R₂ représentent chacun, indépendamment l'un de l'autre, un atome d'hydrogène, un groupe alkyle en C₁ à C₄, un atome d'halogène ou un groupe nitro,
R₃ et R₄ représentent chacun, indépendamment l'un de l'autre, un groupe alkyle en C₁ à C₄ non substitué ou alkyle en C₁ à C₄ substitué par OH, un groupe alcoxy en C₁ à C₄, un atome d'halogène, -CN ou un groupe phényle,
X₁ et X₂ représentent chacun, indépendamment l'un de l'autre, un atome d'hydrogène, un groupe alkyle en C₁ à C₄, alcoxy en C₁ à C₄ ou un atome d'halogène,
R₅ représente un atome d'hydrogène ou un groupe alkyle en C₁ à C₄,
R₆ représente un atome d'hydrogène, un groupe phényle, alkyle en C₁ à C₁₂ ou cycloalkyle en C₅ à C₈, dont chacun peut être non substitué ou substitué par OH, un groupe alcoxy en C₁ à C₄, un atome d'halogène, -CN, ou R₆ représente un groupe alkyle en C₁ à C₄ substitué par un groupe phényle ou cycloalkyle en C₅ à C₈,
ou dans laquelle
R₅ et R₆, conjointement avec l'atome d'azote auquel ils sont liés, forment un cycle pipérazine qui est substitué par un groupe alkyle en C₁ à C₈ ou phényle sur l'atome d'azote non lié au cycle phényle ou qui est quaternisé à cet atome d'azote au moyen de deux de ces groupes, les radicaux alkyle en C₁ à C₈ et phényle mentionnés en tant que substituants sur l'atome d'azote du cycle pipérazine étant non substitués ou substitués par OH, un groupe alcoxy en C₁ à C₄, un atome d'halogène, le nitrate de cellulose ou un groupe phényle, et dans lequel
R₇ et R₈ représentent chacun, indépendamment l'un de l'autre, un radical alkyle en C₁ à C₈ ou un radical benzyle non substitué ou substitué, et
R₉ représente un atome d'hydrogène, un groupe alkyle en C₁ à C₈, alcoxy en C₁ à C₈, un cyanure ou un halogénure, et
n est un nombre entier dans la gamme allant de 2 à 12,
et, dans laquelle X⁻ représente un anion.

4. Procédé selon l'une quelconque des revendications 1 à 3, dans lequel il est utilisé un colorant de formule: 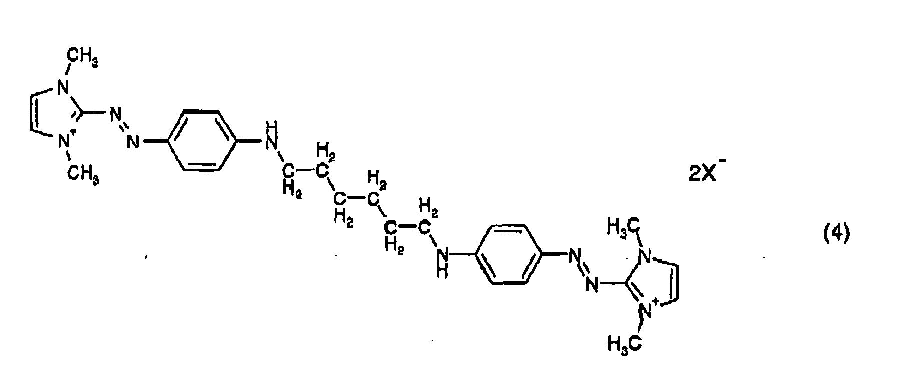 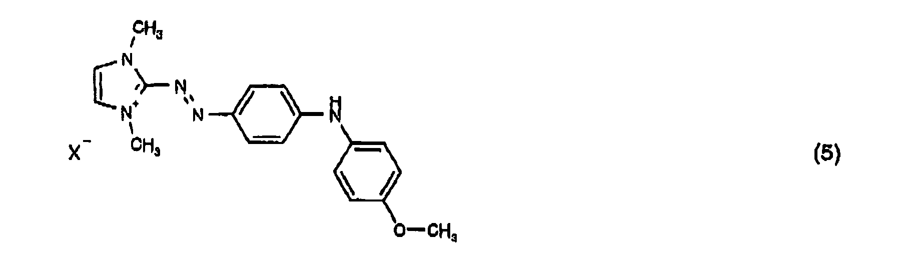 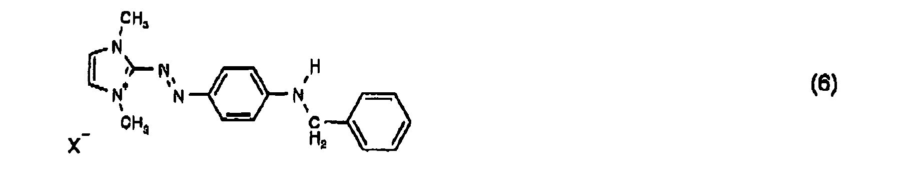 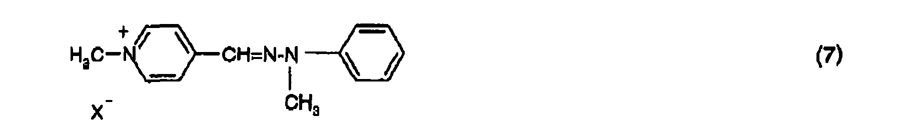 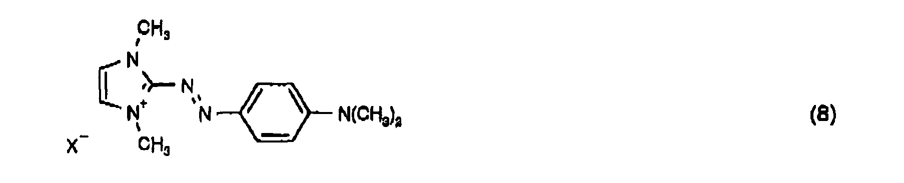 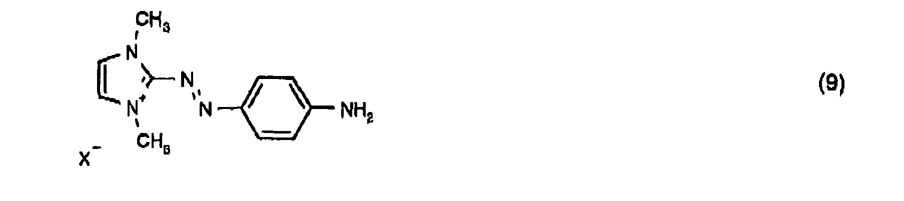 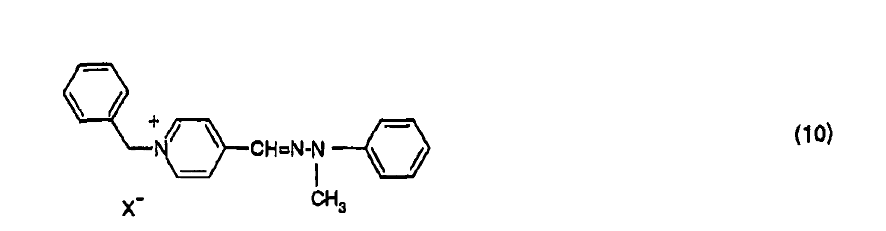 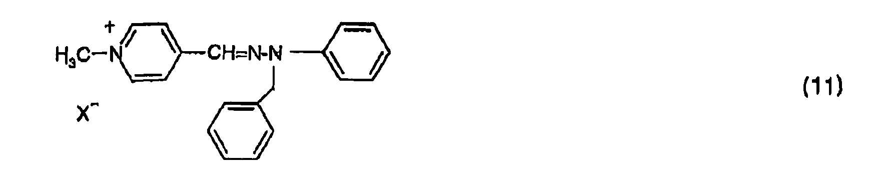 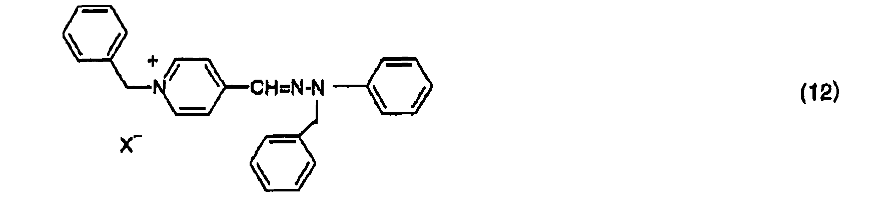 et 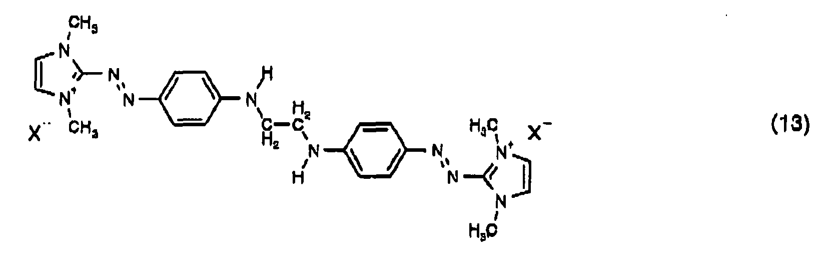 dans lequel X⁻ représente un anion.

5. Procédé selon l'une quelconque des revendications 1 à 4, dans lequel le colorant cationique est utilisé sous forme d'un iodure, bromure, chlorure, sulfate, hydrogénosulfate, méthylsulfate, phosphate, borofluorate ou perchlorate.

6. Procédé selon l'une quelconque des revendications 1 à 5, dans lequel il est utilisé, en tant que sel de métal alcalin d'un acide organique, un formiate, un acétate, un propionate, un butyrate, un monochloroacétate, un trifluoroacétate, un tartrate, un oxalate, un stéarate, un maléate, un acrylate, un succinate, un citrate, un lactate, un méthanesulfonate ou un éthanesulfonate.

7. Procédé selon la revendication 6, dans lequel un formiate, un acétate, un stéarate, un propionate, un citrate, un lactate ou un trifluoroacétate est utilisé.

8. Procédé selon l'une quelconque des revendications 1 à 7, dans lequel un sel de lithium, un sel de sodium ou, en particulier, un sel de potassium est utilisé.

9. Procédé selon l'une quelconque des revendications 1 à 8, dans lequel le méthanol, l'éthanol, le n- ou iso-propanol, le n-, iso- ou tert-butanol est utilisé en tant qu'alcool.

10. Procédé selon la revendication 9, dans lequel le méthanol, l'éthanol ou l'isopropanol est utilisé.
